# EUROPEAN PATENT APPLICATION

(11) **EP 3 878 863 A1**
(43) Date of publication of application: **15.09.2021**
(21) Application number: 19855250.7
(22) Date of filing: 26.08.2019
(51) Int. Cl.: C07K 16/18, C12N 15/13, A61K 39/395, A61P 35/00

(54) **ANTI-CLAUDIN18.2 ANTIBODY AND USE THEREOF**

(30) Priority: 27.08.2018 CN 201810984724
(71) Applicant: Nanjing Sanhome Pharmaceutical Co., Ltd., Nanjing Jiangsu 210038 (CN)
(72) Inventor: WANG, Yong, Nanjing, Jiangsu 210038 (CN); ZHAO, Liwen, Nanjing, Jiangsu 210038 (CN); LU, Zhenzhen, Nanjing, Jiangsu 210038 (CN); SUN, Shichao, Nanjing, Jiangsu 210038 (CN); PANG, Yuhong, Nanjing, Jiangsu 210038 (CN); WEI, Jian, Nanjing, Jiangsu 210038 (CN); ZHOU, Qiuhua, Nanjing, Jiangsu 210038 (CN); HAN, Luwei, Nanjing, Jiangsu 210038 (CN); SONG, Qifeng, Nanjing, Jiangsu 210038 (CN)
(74) Representative: Cabinet Becker et Associés
(86) International application number: PCT/CN2019/102502
(87) International publication number: WO 2020/043044

(57) **Abstract**

Provided are an anti-Claudin18.2 antibody or an antigen binding fragment thereof, a pharmaceutical composition comprising an anti-Claudin18.2 antibody or an antigen binding fragment thereof, and uses thereof. The antibody and Claudin18.2 have significant binding activity and affinity, have efficient in-vitro ADCC cytotoxic activity against a tumor cell, and can be applied in the preparation of an antineoplastic drug.

## Description

### FIELD OF THE INVENTION

The present application relates to the technical field of antibody drugs, in particular to an anti-Claudin18.2 antibody or an antigen binding fragment thereof, a pharmaceutical composition comprising an anti-Claudin18.2 antibody or an antigen binding fragment thereof, and uses thereof.

### BACKGROUND OF THE INVENTION

Gastric cancer is a type of cancer with the fifth highest incidence rate in the world, and its fatality rate is the third highest. Currently, chemotherapy is the standard first-line treatment for advanced or recurrent gastric cancer. More than 90% cancer patients will receive ordinary chemotherapy and targeted chemotherapy, however, chemotherapy generally cannot cure cancer, it only can prolong the survival time of patients and improve their quality of life, and it is easy to develop drug resistance. Biological targeted therapy usually targets tumor-specific antigens or tumor-related antigens, and relies on the binding or blocking of antibodies to the target to trigger the immune response in the body, selectively killing the tumor without affecting the surrounding normal cells and tissues. Targeted therapy has strong specificity, few side effects and precise efficacy.

However, due to the heterogeneity of malignant tumors, different cancers show different molecular biology characteristics. Especially for gastric cancer, of which the degree of heterogeneity is relatively high, the biological targeted therapeutic drugs developed in the past for gastric cancer when used alone are basically ineffective or effective for a very small portion of patients. The reason is that those targets targeted by these monoclonal antibody drugs are only expressed in specific subtypes of gastric cancer patients. A typical example is Trastuzumab, which has a good efficacy when combined with chemotherapy, but only can apply to HER2-positive individuals, which only account for 15% of all gastric cancer patients.

Claudins are the most important skeleton proteins that determine the tight junction structure between cells, which are involved in adherent junction and play an important role in the metastasis and invasion of tumor cells. Claudin proteins are widely distributed in mammalian epithelial and endothelial cells, mainly on the side of epithelial cells and on the plasma membrane of basal cells.

Different Claudin proteins have their own specific expression in different tissues, wherein the Claudin18 (CLDN18) gene is located at 3q22.3, has a molecular weight of 24 kDa, contains 261 amino acid residues, is a member of the Claudin superfamily and its protein structure contains 2 extracellular loops and 4 transmembrane domains. The two subtypes of human CLDN18 protein are CLDN18.1 (NM_016369.3) and CLDN18.2 (NM_001002026.2), respectively, and in the primary structure sequence of both proteins, there are only differences in the amino acid residues in certain positions of the structure of the N-terminal signal peptide to the extracellular loop 1 (Loop1), and in particular in the extracellular loop 1, CLDN18.1 and CLDN18.2 differ only by 8 amino acids. In addition, the sequence homology among different species of the two subtype proteins of CLDN18 is also very high. Among them, the extracellular loop 1 of CLDN18.2 has the exactly same sequence in different species such as human, mouse, and macaque, while the CLDN18.2 protein homology between human and mouse reaches 84%, indicating that the CLDN18.2 protein sequence is extremely conservative (O. Tureci., et al., Gene 481: 83-92, 2011).

The two subtype proteins of CLDN18 are expressed and exert their physiological functions in different tissues, respectively. CLDN18.1 is constitutively expressed in normal lung tissue cells; whereas CLDN18.2 protein is expressed in normal gastric epithelial cell membranes, and is not expressed or expressed in very low levels in various tissues except the stomach. CLDN 18.2 was detected positive by Salin., et al in a variety of cancer tissues including gastric cancer, pancreatic cancer, and esophageal cancer using RT-PCR and immunohistochemical methods, with the overall positive rate reaching 62.7% (U. Salin., et al., Clin Cancer Res 14(23): 7624-34, 2008). Other studies have shown that CLDN18.2 protein expressed by normal gastric parietal cells has homogenous glycosylation modification and compact structures between the cells. However, the structures between gastric cancer cells are loose, and the cells are easy to invade and metastasize, and the CLDN18.2 protein expressed thereon is incompletely glycosylated, which may lead to target exposure (WO2004/047863). Therefore, CLDN18.2 may be an ideal tumor-associated antigen target.

Currently, Astellas Pharma Inc. has developed a monoclonal antibody drug Zolbetuximab (also known as IMAB362) targeting human Claudin18.2, which has significant clinical efficacy. Phase II clinical trials have shown that (S. Batran. et al., Journal of Clinical Oncology 34 (no.18_suppl), 2016), in the treatment of advanced or recurrent gastric cancer using the combination of monoclonal antibody drugs and chemotherapeutic drugs, the median survival time of patients was 13.2 months, significantly longer than the 8.4 months using chemotherapy alone. It is preliminarily confirmed that specific antibodies targeting CLDN18.2 can significantly improve the progression-free survival time and overall survival time of patients with advanced gastric cancer, adenocarcinoma of gastroesophageal junction.

The preparation of a human-mouse chimeric monoclonal antibody described in WO2004/047863 is the first therapeutic antibody developed against the human Claudin18.2 target (hereinafter referred to as anti-CLDN18.2 antibody) worldwide. The antibody described in that patent application specifically kills CLDN18.2 positive tumor cells mainly through the antibody-dependent cell-mediated cytotoxicity (ADCC) pathway. However, the specific recognition and killing between the antibody and tumor cells described in that patent application mainly depends on the abundance of the CLDN18.2 target expressed on the tumor cells, and relatively obvious binding and cytotoxic activity are only shown when the expression of CLDN18.2 on the cell surface is high. This indicates that it is necessary to prepare a high-affinity monoclonal antibody to improve the ability to selectively kill tumor cells.

Based on the prior art above, the present invention now provides an efficient CLDN18.2 antibody or an antigen binding fragment thereof, which has excellent anti-tumor activity.

### SUMMARY OF THE INVENTION

One aspect of the present invention provides an anti-CLDN18.2 antibody or an antigen binding fragment thereof, which comprises a heavy chain variable region and/or a light chain variable region, wherein the heavy chain variable region comprises complementarity determining region 1 of the heavy chain variable region (HCDR1), complementarity determining region 2 of the heavy chain variable region (HCDR2), and/or complementarity determining region 3 of the heavy chain variable region (HCDR3), and the light chain variable region comprises complementarity determining region 1 of the light chain variable region (LCDR1), complementarity determining region 2 of the light chain variable region (LCDR2) and/or complementarity determining region 3 of the light chain variable region (LCDR3).

In some embodiments, the present invention provides an anti-CLDN18.2 antibody or an antigen binding fragment thereof, which comprises a heavy chain variable region and a light chain variable region, wherein:
(1) the heavy chain variable region comprises HCDR1, HCDR2 and HCDR3:
   (a1) the amino acid sequence of the HCDR1 is selected from SEQ ID NO 101, SEQ ID NO 102, SEQ ID NO 103 and amino acid sequences having at least 85% sequence identity with them;
   (a2) the amino acid sequence of the HCDR2 is selected from SEQ ID NO 104, SEQ ID NO 105 and amino acid sequences having at least 85% sequence identity with them;
   (a3) the amino acid sequence of the HCDR3 is selected from SEQ ID NO 106, SEQ ID NO 107 and amino acid sequences having at least 85% sequence identity with them; and
(2) the light chain variable region comprises LCDR1, LCDR2 and LCDR3, wherein:
   (a4) the amino acid sequence of the LCDR1 is SEQ ID NO 108;
   (a5) the amino acid sequence of LCDR2 is selected from SEQ ID NO 109, SEQ ID NO 110, SEQ ID NO 111 and amino acid sequences having at least 85% sequence identity with them;
   (a6) the amino acid sequence of the LCDR3 is selected from SEQ ID NO 112, SEQ ID NO 113, SEQ ID NO 114, SEQ ID NO 115, SEQ ID NO 116 and amino acid sequences having at least 85% sequence identity with them.

In one specific embodiment, the present invention provides an anti-CLDN18.2 antibody or an antigen binding fragment thereof, which has a heavy chain variable region with the HCDR1, HCDR2, and HCDR3 being SEQ ID NO 101, 105 and 106, respectively, or CDRs having at least 85% sequence identity with amino acid sequences shown in SEQ ID NO 101, 105 and 106, and a light chain variable region with the LCDR1, LCDR2, and LCDR3 being SEQ ID NO 108, 111 and 115, respectively, or CDRs having at least 85% sequence identity with amino acid sequences shown in SEQ ID NO 108, 111 and 115.

In some specific embodiments, the anti-CLDN18.2 antibody or the antigen binding fragment thereof according to the present invention is a monoclonal antibody or an antigen binding fragment thereof.

In some specific embodiments, the anti-CLDN18.2 antibody or the antigen binding fragment thereof according to the present invention is a murine-derived antibody or an antigen binding fragment thereof, a chimeric antibody or an antigen binding fragment thereof, or a humanized antibody or an antigen binding fragment thereof.

In some specific embodiments, the present invention provides an anti-CLDN18.2 antibody or an antigen binding fragment thereof, which comprises a heavy chain variable region and a light chain variable region, wherein:
(1) the amino acid sequence of the heavy chain variable region is selected from:
   (b1) an amino acid sequence shown in SEQ ID NO 50, SEQ ID NO 72, SEQ ID NO 74, SEQ ID NO 77, SEQ ID NO 79;
   (b2) an amino acid sequence obtained by substituting, deleting or adding one or more amino acids of the amino acid sequences shown in (b1), and with the same or similar function as the amino acid sequences shown in (b1); and
   (b3) an amino acid sequence having at least 80% sequence identity with the amino acid sequences shown in (b1); and
(2) the amino acid sequence of the light chain variable region is selected from:
   (b4) an amino acid sequence shown in SEQ ID NO 51, SEQ ID NO 70, SEQ ID NO 71, SEQ ID NO 73, SEQ ID NO 75, SEQ ID NO 76, SEQ ID NO 78, SEQ ID NO 80, SEQ ID NO 81;
   (b5) an amino acid sequence obtained by substituting, deleting or adding one or more amino acids of the amino acid sequences shown in (b4), and with the same or similar function as the amino acid sequences shown in (b4); and
   (b6) an amino acid sequence having at least 80% sequence identity with the amino acid sequences shown in (b4).

In some specific embodiments, the present invention provides an anti-CLDN18.2 antibody or an antigen binding fragment thereof, wherein the amino acid sequence of the heavy chain variable region is SEQ ID NO:50, the amino acid sequence obtained by substituting, deleting or adding one or more amino acids of SEQ ID NO:50 and with the same function as SEQ ID NO:50 or the amino acid sequence having at least 85% sequence identity with SEQ ID NO:50, and the amino acid sequence of the light chain variable region is SEQ ID NO:51, the amino acid sequence obtained by substituting, deleting or adding one or more amino acids of SEQ ID NO:51 and with the same function as SEQ ID NO:51 or the amino acid sequence having at least 85% sequence identity with SEQ ID NO:51.

In some specific embodiments, the present invention provides an anti-CLDN18.2 antibody or an antigen binding fragment thereof, wherein the amino acid sequence of the heavy chain variable region is SEQ ID NO:50, the amino acid sequence obtained by substituting, deleting or adding one or more amino acids of SEQ ID NO:50 and with the same function as SEQ ID NO:50 or the amino acid sequence having at least 85% sequence identity with SEQ ID NO:50, and the amino acid sequence of the light chain variable region is SEQ ID NO:70, the amino acid sequence obtained by substituting, deleting or adding one or more amino acids of SEQ ID NO:70 and with the same function as SEQ ID NO:70 or the amino acid sequence having at least 85% sequence identity with SEQ ID NO:70.

In some specific embodiments, the present invention provides an anti-CLDN18.2 antibody or an antigen binding fragment thereof, wherein the amino acid sequence of the heavy chain variable region is SEQ ID NO:50, the amino acid sequence obtained by substituting, deleting or adding one or more amino acids of SEQ ID NO:50 and with the same function as SEQ ID NO:50 or the amino acid sequence having at least 85% sequence identity with SEQ ID NO:50, and the amino acid sequence of the light chain variable region is SEQ ID NO:71, the amino acid sequence obtained by substituting, deleting or adding one or more amino acids of SEQ ID NO:71 and with the same function as SEQ ID NO:71 or the amino acid sequence having at least 85% sequence identity with SEQ ID NO:71.

In some specific embodiments, the present invention provides an anti-CLDN18.2 antibody or an antigen binding fragment thereof, wherein the amino acid sequence of the heavy chain variable region is SEQ ID NO:72, the amino acid sequence obtained by substituting, deleting or adding one or more amino acids of SEQ ID NO: 72 and with the same function as SEQ ID NO:72 or the amino acid sequence having at least 85% sequence identity with SEQ ID NO:72, and the amino acid sequence of the light chain variable region is SEQ ID NO:73, the amino acid sequence obtained by substituting, deleting or adding one or more amino acids of SEQ ID NO:73 and with the same function as SEQ ID NO:73 or the amino acid sequence having at least 85% sequence identity with SEQ ID NO:73.

In some specific embodiments, the present invention provides an anti-CLDN18.2 antibody or an antigen binding fragment thereof, wherein the amino acid sequence of the heavy chain variable region is SEQ ID NO:74, the amino acid sequence obtained by substituting, deleting or adding one or more amino acids of SEQ ID NO: 74 and with the same function as SEQ ID NO:74 or the amino acid sequence having at least 85% sequence identity with SEQ ID NO:74, and the amino acid sequence of the light chain variable region is SEQ ID NO:75, the amino acid sequence obtained by substituting, deleting or adding one or more amino acids of SEQ ID NO:75 and with the same function as SEQ ID NO:75 or the amino acid sequence having at least 85% sequence identity with SEQ ID NO:75.

In some specific embodiments, the present invention provides an anti-CLDN18.2 antibody or an antigen binding fragment thereof, wherein the amino acid sequence of the heavy chain variable region is SEQ ID NO:50, the amino acid sequence obtained by substituting, deleting or adding one or more amino acids of SEQ ID NO:50 and with the same function as SEQ ID NO:50 or the amino acid sequence having at least 85% sequence identity with SEQ ID NO:50, and the amino acid sequence of the light chain variable region is SEQ ID NO:76, the amino acid sequence obtained by substituting, deleting or adding one or more amino acids of SEQ ID NO:76 and with the same function as SEQ ID NO:76 or the amino acid sequence having at least 85% sequence identity with SEQ ID NO:76.

In some specific embodiments, the present invention provides an anti-CLDN18.2 antibody or an antigen binding fragment thereof, wherein the amino acid sequence of the heavy chain variable region is SEQ ID NO:77, the amino acid sequence obtained by substituting, deleting or adding one or more amino acids of SEQ ID NO: 77 and with the same function as SEQ ID NO:77 or the amino acid sequence having at least 85% sequence identity with SEQ ID NO:77, and the amino acid sequence of the light chain variable region is SEQ ID NO:78, the amino acid sequence obtained by substituting, deleting or adding one or more amino acids of SEQ ID NO:78 and with the same function as SEQ ID NO:78 or the amino acid sequence having at least 85% sequence identity with SEQ ID NO:78.

In some specific embodiments, the present invention provides an anti-CLDN18.2 antibody or an antigen binding fragment thereof, wherein the amino acid sequence of the heavy chain variable region is SEQ ID NO:74, the amino acid sequence obtained by substituting, deleting or adding one or more amino acids of SEQ ID NO: 74 and with the same function as SEQ ID NO:74 or the amino acid sequence having at least 85% sequence identity with SEQ ID NO:74, and the amino acid sequence of the light chain variable region is SEQ ID NO:70, the amino acid sequence obtained by substituting, deleting or adding one or more amino acids of SEQ ID NO:70 and with the same function as SEQ ID NO:70 or the amino acid sequence having at least 85% sequence identity with SEQ ID NO:70.

In some specific embodiments, the present invention provides an anti-CLDN18.2 antibody or an antigen binding fragment thereof, wherein the amino acid sequence of the heavy chain variable region is SEQ ID NO:74, the amino acid sequence obtained by substituting, deleting or adding one or more amino acids of SEQ ID NO: 74 and with the same function as SEQ ID NO:74 or the amino acid sequence having at least 85% sequence identity with SEQ ID NO:74, and the amino acid sequence of the light chain variable region is SEQ ID NO:71, the amino acid sequence obtained by substituting, deleting or adding one or more amino acids of SEQ ID NO:71 and with the same function as SEQ ID NO:71 or the amino acid sequence having at least 85% sequence identity with SEQ ID NO:71.

In some specific embodiments, the present invention provides an anti-CLDN18.2 antibody or an antigen binding fragment thereof, wherein the amino acid sequence of the heavy chain variable region is SEQ ID NO:74, the amino acid sequence obtained by substituting, deleting or adding one or more amino acids of SEQ ID NO: 74 and with the same function as SEQ ID NO:74 or the amino acid sequence having at least 85% sequence identity with SEQ ID NO:74, and the amino acid sequence of the light chain variable region is SEQ ID NO:76, the amino acid sequence obtained by substituting, deleting or adding one or more amino acids of SEQ ID NO:76 and with the same function as SEQ ID NO:76 or the amino acid sequence having at least 85% sequence identity with SEQ ID NO:76.

In some specific embodiments, the present invention provides an anti-CLDN18.2 antibody or an antigen binding fragment thereof, wherein the amino acid sequence of the heavy chain variable region is SEQ ID NO:72, the amino acid sequence obtained by substituting, deleting or adding one or more amino acids of SEQ ID NO: 72 and with the same function as SEQ ID NO:72 or the amino acid sequence having at least 85% sequence identity with SEQ ID NO:72, and the amino acid sequence of the light chain variable region is SEQ ID NO:76, the amino acid sequence obtained by substituting, deleting or adding one or more amino acids of SEQ ID NO:76 and with the same function as SEQ ID NO:76 or the amino acid sequence having at least 85% sequence identity with SEQ ID NO:76.

In some specific embodiments, the present invention provides an anti-CLDN18.2 antibody or an antigen binding fragment thereof, wherein the amino acid sequence of the heavy chain variable region is SEQ ID NO:77, the amino acid sequence obtained by substituting, deleting or adding one or more amino acids of SEQ ID NO: 77 and with the same function as SEQ ID NO:77 or the amino acid sequence having at least 85% sequence identity with SEQ ID NO:77, and the amino acid sequence of the light chain variable region is SEQ ID NO:70, the amino acid sequence obtained by substituting, deleting or adding one or more amino acids of SEQ ID NO:70 and with the same function as SEQ ID NO:70 or the amino acid sequence having at least 85% sequence identity with SEQ ID NO:70.

In some specific embodiments, the present invention provides an anti-CLDN18.2 antibody or an antigen binding fragment thereof, wherein the amino acid sequence of the heavy chain variable region is SEQ ID NO:77, the amino acid sequence obtained by substituting, deleting or adding one or more amino acids of SEQ ID NO: 77 and with the same function as SEQ ID NO:77 or the amino acid sequence having at least 85% sequence identity with SEQ ID NO:77, and the amino acid sequence of the light chain variable region is SEQ ID NO:76, the amino acid sequence obtained by substituting, deleting or adding one or more amino acids of SEQ ID NO:76 and with the same function as SEQ ID NO:76 or the amino acid sequence having at least 85% sequence identity with SEQ ID NO:76.

In some specific embodiments, the present invention provides an anti-CLDN18.2 antibody or an antigen binding fragment thereof, wherein the amino acid sequence of the heavy chain variable region is SEQ ID NO:79, the amino acid sequence obtained by substituting, deleting or adding one or more amino acids of SEQ ID NO: 79 and with the same function as SEQ ID NO:79 or the amino acid sequence having at least 85% sequence identity with SEQ ID NO:79, and the amino acid sequence of the light chain variable region is SEQ ID NO:70, the amino acid sequence obtained by substituting, deleting or adding one or more amino acids of SEQ ID NO:70 and with the same function as SEQ ID NO:70 or the amino acid sequence having at least 85% sequence identity with SEQ ID NO:70.

In some specific embodiments, the present invention provides an anti-CLDN18.2 antibody or an antigen binding fragment thereof, wherein the amino acid sequence of the heavy chain variable region is SEQ ID NO:79, the amino acid sequence obtained by substituting, deleting or adding one or more amino acids of SEQ ID NO: 79 and with the same function as SEQ ID NO:79 or the amino acid sequence having at least 85% sequence identity with SEQ ID NO:79, and the amino acid sequence of the light chain variable region is SEQ ID NO:76, the amino acid sequence obtained by substituting, deleting or adding one or more amino acids of SEQ ID NO:76 and with the same function as SEQ ID NO:76 or the amino acid sequence having at least 85% sequence identity with SEQ ID NO:76.

In some specific embodiments, the present invention provides an anti-CLDN18.2 antibody or an antigen binding fragment thereof, wherein the amino acid sequence of the heavy chain variable region is SEQ ID NO:50, the amino acid sequence obtained by substituting, deleting or adding one or more amino acids of SEQ ID NO:50 and with the same function as SEQ ID NO:50 or the amino acid sequence having at least 85% sequence identity with SEQ ID NO:50, and the amino acid sequence of the light chain variable region is SEQ ID NO:80, the amino acid sequence obtained by substituting, deleting or adding one or more amino acids of SEQ ID NO:80 and with the same function as SEQ ID NO:80 or the amino acid sequence having at least 85% sequence identity with SEQ ID NO:80.

In some specific embodiments, the present invention provides an anti-CLDN18.2 antibody or an antigen binding fragment thereof, wherein the amino acid sequence of the heavy chain variable region is SEQ ID NO:50, the amino acid sequence obtained by substituting, deleting or adding one or more amino acids of SEQ ID NO:50 and with the same function as SEQ ID NO:50 or the amino acid sequence having at least 85% sequence identity with SEQ ID NO:50, and the amino acid sequence of the light chain variable region is SEQ ID NO:81, the amino acid sequence obtained by substituting, deleting or adding one or more amino acids of SEQ ID NO:81 and with the same function as SEQ ID NO:81 or the amino acid sequence having at least 85% sequence identity with SEQ ID NO:81.

In some specific embodiments, the present invention provides an anti-CLDN18.2 antibody or an antigen binding fragment thereof, wherein the amino acid sequence of the heavy chain variable region is SEQ ID NO:79, the amino acid sequence obtained by substituting, deleting or adding one or more amino acids of SEQ ID NO:79 and with the same function as SEQ ID NO:79 or the amino acid sequence having at least 85% sequence identity with SEQ ID NO:79, and the amino acid sequence of the light chain variable region is SEQ ID NO:80, the amino acid sequence obtained by substituting, deleting or adding one or more amino acids of SEQ ID NO:80 and with the same function as SEQ ID NO:80 or the amino acid sequence having at least 85% sequence identity with SEQ ID NO:80.

In some specific embodiments, the present invention provides an anti-CLDN18.2 antibody or an antigen binding fragment thereof, wherein the amino acid sequence of the heavy chain variable region is SEQ ID NO:79, the amino acid sequence obtained by substituting, deleting or adding one or more amino acids of SEQ ID NO:79 and with the same function as SEQ ID NO:79 or the amino acid sequence having at least 85% sequence identity with SEQ ID NO:79, and the amino acid sequence of the light chain variable region is SEQ ID NO:81, the amino acid sequence obtained by substituting, deleting or adding one or more amino acids of SEQ ID and with the same function as SEQ ID or the amino acid sequence having at least 85% sequence identity with SEQ ID NO:81.

In some specific embodiments, the present invention provides an anti-CLDN18.2 antibody or an antigen binding fragment thereof, wherein the antibody further contains a heavy chain constant region of human-derived IgG1 or variants thereof, and a light chain constant region of human-derived kappa chain or variants thereof.

In some specific embodiments, the present invention provides an anti-CLDN18.2 antibody or an antigen binding fragment thereof, wherein amino acid replacements in the heavy chain variable region with amino acid sequence shown in SEQ ID NO 79 can be made as follows:
(c1) K3Q, L19R, R42G, P45L, A49S, E73D, T78S, E82Q, S84N, S88A, M93V, A118S, the amino acid sequence after replacement is shown in SEQ ID NO 82;
(c2) K3Q, V5L, L19R, R42G, P45L, A49S, E73D, A75S, E82Q, S8N, S88A, M93V, A118S, the amino acid sequence after replacement is shown in SEQ ID NO 88.

And amino acid replacements in the light chain variable region with amino acid sequence shown in SEQ ID NO 81 can be made as follows:
(c3) D1E, S9P, S10T, T12S, V13L, T14S, A15P, K18R, K21L, P49A, K51L, G63S, V64I, D66A, T69S, V84L, A86P, A106Q, L112I, the amino acid sequence after replacement is shown in SEQ ID NO 84;
(c4) S9D, T12A, T14S, A15L, K18R, V19A, M21I, S22N, T69S, V84L, L89V, A106Q, L112I, the amino acid sequence after replacement is shown in SEQ ID NO 86;
(c5) A9L, T12P, A15L, E17Q, K18P, V19A, T20S, M21I, K45R, P49S, K51R, T69S, T80K, S83R, Q85E, L89V, A90G, A106Q, L112I, the amino acid sequence after replacement is shown in SEQ ID NO 90.

In some specific embodiments, the present invention provides an anti-CLDN18.2 antibody or an antigen binding fragment thereof, wherein:
(1) the amino acid sequence of the heavy chain variable region is selected from:
   (c1) an amino acid sequence shown in SEQ ID NO 82, SEQ ID NO 88;
   (c2) an amino acid sequence obtained by substituting, deleting or adding one or more amino acids of the amino acid sequences shown in (c1), and with the same or similar function as the amino acid sequence shown in (c1); and
   (c3) an amino acid sequence having at least 80% sequence identity with the amino acid sequence shown in (c1); and
(2) the amino acid sequence of the light chain variable region is selected from:
   (c4) an amino acid sequence shown in SEQ ID NO 84, SEQ ID NO 86, SEQ ID NO 90;
   (c5) an amino acid sequence obtained by substituting, deleting or adding one or more amino acids of the amino acid sequences shown in (c4), and with the same or similar function as the amino acid sequence shown in (c4); and
   (c6) an amino acid sequence having at least 80% sequence identity with the amino acid sequence shown in (c4).

In some specific embodiments, the present invention provides an anti-CLDN18.2 antibody or an antigen binding fragment thereof, wherein the amino acid sequence of the heavy chain variable region is SEQ ID NO 82, the amino acid sequence obtained by substituting, deleting or adding one or more amino acids of SEQ ID NO 82 and with the same function as SEQ ID NO 82 or the amino acid sequence having at least 85% sequence identity with SEQ ID NO 82, and the amino acid sequence of the light chain variable region is SEQ ID NO 84, the amino acid sequence obtained by substituting, deleting or adding one or more amino acids of SEQ ID NO 84 and with the same function as SEQ ID NO 84 or the amino acid sequence having at least 85% sequence identity with SEQ ID NO 84.

In some specific embodiments, the present invention provides an anti-CLDN18.2 antibody or an antigen binding fragment thereof, wherein the amino acid sequence of the heavy chain variable region is SEQ ID NO 82, the amino acid sequence obtained by substituting, deleting or adding one or more amino acids of SEQ ID NO 82 and with the same function as SEQ ID NO 82 or the amino acid sequence having at least 85% sequence identity with SEQ ID NO 82, and the amino acid sequence of the light chain variable region is SEQ ID NO 86, the amino acid sequence obtained by substituting, deleting or adding one or more amino acids of SEQ ID NO 86 and with the same function as SEQ ID NO 86 or the amino acid sequence having at least 85% sequence identity with SEQ ID NO 86.

In some specific embodiments, the present invention provides an anti-CLDN18.2 antibody or an antigen binding fragment thereof, wherein the amino acid sequence of the heavy chain variable region is SEQ ID NO 88, the amino acid sequence obtained by substituting, deleting or adding one or more amino acids of SEQ ID NO 88 and with the same function as SEQ ID NO 88 or the amino acid sequence having at least 85% sequence identity with SEQ ID NO 88, and the amino acid sequence of the light chain variable region is SEQ ID NO 84, the amino acid sequence obtained by substituting, deleting or adding one or more amino acids of SEQ ID NO 84 and with the same function as SEQ ID NO 84 or the amino acid sequence having at least 85% sequence identity with SEQ ID NO 84.

In some specific embodiments, the present invention provides an anti-CLDN18.2 antibody or an antigen binding fragment thereof, wherein the amino acid sequence of the heavy chain variable region is SEQ ID NO 88, the amino acid sequence obtained by substituting, deleting or adding one or more amino acids of SEQ ID NO 88 and with the same function as SEQ ID NO 88 or the amino acid sequence having at least 85% sequence identity with SEQ ID NO 88, and the amino acid sequence of the light chain variable region is SEQ ID NO 86, the amino acid sequence obtained by substituting, deleting or adding one or more amino acids of SEQ ID NO 86 and with the same function as SEQ ID NO 86 or the amino acid sequence having at least 85% sequence identity with SEQ ID NO 86.

In some specific embodiments, the present invention provides an anti-CLDN18.2 antibody or an antigen binding fragment thereof, wherein the amino acid sequence of the heavy chain variable region is SEQ ID NO 82, the amino acid sequence obtained by substituting, deleting or adding one or more amino acids of SEQ ID NO 82 and with the same function as SEQ ID NO 82 or the amino acid sequence having at least 85% sequence identity with SEQ ID NO 82, and the amino acid sequence of the light chain variable region is SEQ ID NO 90, the amino acid sequence obtained by substituting, deleting or adding one or more amino acids of SEQ ID NO 90 and with the same function as SEQ ID NO 90 or the amino acid sequence having at least 85% sequence identity with SEQ ID NO 90.

In some specific embodiments, the present invention provides an anti-CLDN18.2 antibody or an antigen binding fragment thereof, wherein the amino acid sequence of the heavy chain variable region is SEQ ID NO 88, the amino acid sequence obtained by substituting, deleting or adding one or more amino acids of SEQ ID NO 88 and with the same function as SEQ ID NO 88 or the amino acid sequence having at least 85% sequence identity with SEQ ID NO 88, and the amino acid sequence of the light chain variable region is SEQ ID NO 90, the amino acid sequence obtained by substituting, deleting or adding one or more amino acids of SEQ ID NO 90 and with the same function as SEQ ID NO 90 or the amino acid sequence having at least 85% sequence identity with SEQ ID NO 90.

In some specific embodiments, the present invention provides an anti-CLDN18.2 antibody or an antigen binding fragment thereof, wherein the amino acid sequence of the heavy chain is SEQ ID NO 92, the amino acid sequence obtained by substituting, deleting or adding one or more amino acids of SEQ ID NO 92 and with the same function as SEQ ID NO 92 or the amino acid sequence having at least 85% sequence identity with SEQ ID NO 92, and the amino acid sequence of the light chain is SEQ ID NO 94, the amino acid sequence obtained by substituting, deleting or adding one or more amino acids of SEQ ID NO 94 and with the same function as SEQ ID NO 94 or the amino acid sequence having at least 85% sequence identity with SEQ ID NO 94.

In some specific embodiments, the present invention provides an anti-CLDN18.2 antibody or an antigen binding fragment thereof, wherein the amino acid sequence of the heavy chain is SEQ ID NO 96, the amino acid sequence obtained by substituting, deleting or adding one or more amino acids of SEQ ID NO 96 and with the same function as SEQ ID NO 96 or the amino acid sequence having at least 85% sequence identity with SEQ ID NO 96, and the amino acid sequence of the light chain is SEQ ID NO 98, the amino acid sequence obtained by substituting, deleting or adding one or more amino acids of SEQ ID NO 98 and with the same function as SEQ ID NO 98 or the amino acid sequence having at least 85% sequence identity with SEQ ID NO 98.

Another aspect of the present invention provides an isolated nucleotide, which encodes the anti-CLDN18.2 antibody or the antigen binding fragment thereof of the present invention.

In some specific embodiments, the present invention provides nucleotide sequences encoding the anti-CLDN18.2 antibody or the antigen binding fragment thereof of the present invention, wherein,
(1) the nucleotide sequence encoding the amino acid sequence of the heavy chain is shown in SEQ ID NO 93 or SEQ ID NO 97;
(2) the nucleotide sequence encoding the amino acid sequence of the light chain is shown in SEQ ID NO 95 or SEQ ID NO 99.

In some specific embodiments, the present invention provides nucleotide sequences encoding the anti-CLDN18.2 antibody or the antigen binding fragment thereof of the present invention, wherein,
(1) the nucleotide sequence encoding the amino acid sequence SEQ ID NO 92 of the heavy chain is shown in SEQ ID NO 93; and
(2) the nucleotide sequence encoding the amino acid sequence SEQ ID NO 94 of the light chain is shown in SEQ ID NO 95.

In some specific embodiments, the present invention provides nucleotide sequences encoding the anti-CLDN18.2 antibody or the antigen binding fragment thereof of the present invention, wherein,
(1) the nucleotide sequence encoding the amino acid sequence SEQ ID NO 96 of the heavy chain is shown in SEQ ID NO 97; and
(2) the nucleotide sequence encoding the amino acid sequence SEQ ID NO 98 of the light chain is shown in SEQ ID NO 99.

Another aspect of the present invention provides an expression vector, which expresses the anti-CLDN18.2 antibody or the antigen binding fragment thereof of the present invention. The expression vector according to the present invention comprises the isolated nucleotide molecule of the present invention.

Another aspect of the present invention provides a host cell transfected with the expression vector as described above.

In some embodiments, the host cell according to the present invention is selected from a prokaryotic cell and a eukaryotic cell. In some embodiments, the host cell is bacteria, preferably *Escherichia coli.* In another preferred embodiment, the host cell is a mammalian cell.

Another aspect of the present invention provides a method for preparing the anti-CLDN18.2 antibody or the antigen binding fragment thereof of the present invention, which comprises steps of expressing the antibody in the host cell and isolating the antibody from the host cell.

Another aspect of the present invention provides a pharmaceutical composition, which comprises the anti-CLDN18.2 humanized antibody or the antigen binding fragment thereof of the present invention and a pharmaceutically acceptable carrier. In some embodiments, the present invention provides a pharmaceutical composition, which comprises the anti-CLDN18.2 humanized antibody or the antigen binding fragment thereof of the present invention, as well as other active components, such as other antibodies, targeting drugs and the like. In some embodiments, the pharmaceutically acceptable carrier is selected from antioxidants, polypeptides, proteins, hydrophilic polymers, amino acids, sugars, chelating agents, sugar alcohols, ions, and surfactants. In one specific embodiment, the pharmaceutically acceptable carrier is a buffered aqueous solution. In another specific embodiment, the pharmaceutically acceptable carrier is in the form of liposomes.

The anti-CLDN18.2 humanized antibody or the antigen binding fragment thereof of the present invention can be mixed with a pharmaceutically acceptable carrier, diluent or excipient to prepare a pharmaceutical preparation suitable for oral or parenteral administration. Methods of administration include, but are not limited to oral, intradermal, intramuscular, intraperitoneal, intravenous, intracerebral, intraocular, intratracheal, subcutaneous, and intranasal routes. The preparation can be administered by any route, for example, by infusion or bolus injection, by the route of absorption through the epithelium or skin mucosa (for example, oral mucosa or rectum, etc.). Administration can be systemic or local. The preparation can be prepared by methods known in the art, and comprises carriers, diluents or excipients conventionally used in the field of pharmaceutical preparations.

Another aspect of the present invention provides a method for therapy targeting CLDN18.2, including administering the anti-CLDN18.2 antibody or the antigen binding fragment thereof of the present invention or the pharmaceutical composition of the present invention to an individual in need thereof.

Another aspect of the present invention provides the use of the anti-CLDN18.2 antibody or the antigen binding fragment thereof of the present invention or the pharmaceutical composition of the present invention in the preparation of a drug targeting CLDN18.2. In some embodiments, the drug targeting CLDN18.2 is used to treat gastric cancer, esophageal cancer, pancreatic cancer, lung cancer, ovarian cancer, colon cancer, liver cancer, head and neck cancer, and gallbladder cancer. In some embodiments, the present invention provides the use of the aforementioned anti-CLDN18.2 antibody or the antigen binding fragment thereof or the pharmaceutical composition of the present invention in the preparation of an anti-tumor drug, preferably, the tumor is selected from gastric cancer, esophageal cancer, pancreatic cancer, lung cancer, ovarian cancer, colon cancer, liver cancer, head and neck cancer and gallbladder cancer.

The anti-CLDN18.2 antibody or the antigen binding fragment thereof provided by the present invention has a significant anti-tumor effect, high affinity, strong in-vitro ADCC cytotoxicity against tumor cells, and has significant technical advantages.

### DEFINITION

Unless otherwise defined, the scientific and technical terms used herein have a meaning that is commonly understood by those skilled in the art. The nomenclatures and techniques used in the cell and tissue culture, molecular biology, and protein and oligo- or poly- nucleotide chemistry and hybridization described herein are well known and commonly used in the art. For recombinant DNA, oligonucleotide synthesis, and tissue culture and transfection (such as electroporation, lipofection), standard techniques are used. The enzymatic reaction and purification techniques are carried out according to the manufacturer's manual or methods commonly used in the art or described herein. The aforementioned techniques and methods are generally used as described in multiple comprehensive and relatively specific documents well known in the art and cited and discussed in the specification. See, for example, Sambrook et al., Molecular Cloning: A Laboratory Manual (2nd edition, Cold Spring Harbor Laboratory Press, Cold Spring Harbor, New York (1989)). The nomenclatures and laboratory methods and techniques used in analytical chemistry, synthetic organic chemistry, and medical and pharmaceutical chemistry described herein are well known and commonly used in the art.

In the present invention, the term "at least 80% sequence identity" refers to at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity. In the present invention, the term "at least 85% sequence identity" means at least 85%, 86%, 87%, 88%, 89%, 90%, 91%, 92%, 93%, 94%, 95%, 96%, 97%, 98%, 99%, or 100% sequence identity. In some preferred embodiments, the sequence identity described in the present invention may be at least 90%, 91%, 92%, 93%, 94%, 95%, 96%, 97%, 98%, 99%, or 100%. Sequence comparison and determination of percent identity between two sequences can be performed by the BLASTN/BLASTP algorithm on the National Center For Biotechnology Institute website.

In an antibody molecule, the three hypervariable regions of the light chain and the three hypervariable regions of the heavy chain are arranged relative to each other in the three-dimensional space to form an antigen binding surface. The antigen binding surface is complementary to the three-dimensional surface of the bound antigen, and the three hypervariable regions of each heavy chain and light chain are referred to as "complementarity determining regions" or "CDRs". The assignment of amino acids to each domain is defined according to Kabat "Sequences of Proteins of Immunological Interest" (National Institutes of Health, Bethesda, Maryland (1987 and 1991)) or Chothia and Lesk, J. Mol. Biol. 196:901-917 (1987), Chothia et al., Nature 342:878-883 (1989).

The "antigen binding fragment" described in the present invention refers to Fab fragments, Fab' fragments, and F(ab')2 fragments which have antigen binding activity, and Fv fragments and scFv fragments which bind to the human Claudin18.2. The Fv fragment contains the heavy chain variable region and the light chain variable region of an antibody, but no constant region, and is the smallest antibody fragment with all the antigen binding sites. Generally, the Fv antibody also comprises a polypeptide linker between the VH and VL domains, and can form the structure required for the antigen binding. Different linkers can also be used to connect the two antibody variable regions to form a polypeptide chain, referred to as single-chain antibody or single-chain Fv (scFv).

The antibody described in the present invention refers to an immunoglobulin molecule or an immunologically active part thereof, that is, a molecule comprising an antigen binding site that specifically binds to (immunologically reacts with) an antigen. "Specific binding" refers to that an antibody reacts with one or more epitopes of an antigen but does not react with other polypeptides or binds to other polypeptides with a very low affinity (Kd>10-6). Antibodies include, but are not limited to, polyclonal antibodies, monoclonal antibodies, chimeric antibodies, dAbs (domain antibodies), single chain antibodies, Fab, Fab' and F(ab')2 fragments, Fv, scFv and Fab expression libraries. Monoclonal antibodies (mAbs) are antibodies obtained from a single clonal cell strain, and the cell strain is not limited to eukaryotic, prokaryotic or phage clonal cell strain. Monoclonal antibodies or antigen binding fragments thereof can be recombinantly obtained, for example, by using hybridoma technology, recombination technology, phage display technology, and synthesis technology such as CDR grafting or other existing technologies.

The "murine-derived antibody" described in the present invention is a monoclonal antibody against the human CLDN18.2 prepared according to the knowledge and skills in the art. During the preparation, test subjects are injected with CLDN18.2 antigen, and then hybridomas expressing antibodies with desired sequences or functional characteristics are isolated.

The "chimeric antibody" described in the present invention is an antibody formed by fusing the variable region of a murine-derived antibody with the constant region of a human antibody, which can reduce immune responses induced by the murine-derived antibody. To construct the chimeric antibodies, it is necessary to construct hybridomas secreting murine-derived specific monoclonal antibodies first, and then clone the variable region gene from the mouse hybridoma cells, and then clone the constant region gene of the human antibody as needed, connect the mouse variable region gene to the human constant region gene to form the chimeric gene which is then inserted into an expression vector, and finally express the chimeric antibody molecule in a eukaryotic industrial system or a prokaryotic industrial system.

The "humanized antibody" described in the present invention is also referred to as CDR grafted antibody, which is an antibody produced by grafting the mouse CDR sequence into the human antibody variable region framework (FR). Such variable region framework sequences can be obtained from public DNA databases or published references, for example, from the ImMunoGeneTics (IMGT) website http://imgt.cines.fr or from the Journal of Immunoglobulin, 2001ISBN012441351.

### BRIEF DESCRIPTION OF THE DRAWINGS

Figure 1 is the binding of the chimeric antibodies to CLDN18.2 cells, wherein the horizontal axis is the antibody concentration in ng/ml; the vertical axis is the OD value at 450 nm.
Figure 2 is the binding of the chimeric antibodies to CLDN18.1 cells, wherein the horizontal axis is the antibody concentration in ng/ml; the vertical axis is the OD value at 450 nm.
Figure 3 is the binding of the phage Fabs to the CLDN18.2 protein, wherein the horizontal axis is the phage titer; the vertical axis is the OD value at 450 nm.
Figure 4 is the binding of the humanized antibodies to CHO-K1-CLDN18.2 cells, wherein the horizontal axis is the antibody concentration in mg/ml; the vertical axis is the average fluorescence intensity MFI of binding to the cells.
Figure 5 is the binding of the humanized antibodies to CHO-K1-CLDN18.1 cells, wherein the horizontal axis is the antibody concentration in mg/ml; the vertical axis is the average fluorescence intensity MFI of binding to the cells.
Figure 6 is the ADCC activity of the humanized antibodies.

### DETAILED DESCRIPTION OF THE INVENTION

The following representative examples are used to better illustrate the present invention, rather than to limit the protection scope of the present invention. The experimental methods without indicated conditions in the following examples are generally carried out according to conventional conditions, such as the antibody technology experimental manual, molecular cloning manual or the like of Cold Spring Harbor, or according to the conditions recommended by the raw material or product manufacturers. Materials and reagents used in the examples are all commercially available unless otherwise specified.

### Example 1 Preparation of the positive control antibody

### 1. Preparation of the positive control antibody IMAB362-similar-hG1:

According to the 175D10 clone (IMAB362) antibody sequence provided by the patent application WO2007/059997 (i.e., SEQ ID NO 11 and SEQ ID NO 12 in the present disclosure), Nanjing GenScript Biotechnology Co., Ltd. was entrusted to perform codon optimization and synthesize it in vector pcDNA3.1(+). The constructed plasmids include pcDNA3.1-hG1 and pcDNA3.1-hK. expiCHO cells were co-transfected with the plasmids at a ratio of 1:1 for transient expression, cultured for 4 days at 110 rpm, 37°C, 8% CO₂, transferred to a 32°C shaker to further culture for 8-10 days. After centrifugation at 4500 rpm for 25 minutes, the protein supernatant obtained was purified by ProteinA affinity chromatography as follows: the AKTA chromatography system and columns were cleaned with 0.1 M NaOH solution for 30 minutes, rinsed clean with ultrapure water; equilibrated with 10 column volumes of 20 mM sodium phosphate buffer of pH 7.4; the flow rate was set to 2 ml/min, samples were loaded; the columns were cleaned with 10 column volumes of the equilibration buffer; eluted with 3-5 column volumes of 50 mM acetate buffer of pH 3.4, the eluted protein was collected; concentrated via ultrafiltration to displace the antibody in 20 mM phosphate buffer of pH 7.0 for storage.

Results of the SDS-PAGE gel electrophoresis show that: the molecular weight of the positive antibody IMAB362-similar-hG1 is about 155 KD, the purity by SDS-PAGE is greater than 90%.

### 2. Preparation of murine-derived positive control antibody IMAB362-similar-mG2a:

That is, a combined antibody sequence of the positive antibody variable region sequence SEQ ID NO 13 and mouse IgG2a constant region sequence SEQ ID NO 14. It was synthesized in vector pcDNA3.1(+) after codon optimization. The constructed plasmids include pcDNA3.1-mG2a and pcDNA3.1-mK. expiCHO cells were co-transfected with the plasmids at a ratio of 1:1 for transient expression of the positive antibody proteins. The protein supernatant obtained was purified by ProteinA affinity chromatography as follows: the AKTA chromatography system and columns were cleaned with 0.1 M NaOH solution for 30 minutes, rinsed clean with ultrapure water; equilibrated with 10 column volumes of 20 mM sodium phosphate buffer of pH 7.4; the flow rate was set to 2 ml/min, samples were loaded; the columns were cleaned with 10 column volumes of the equilibration buffer; eluted with 3-5 column volumes of 50 mM acetate buffer of pH 3.4, the eluted protein was collected; concentrated via ultrafiltration to displace the antibody in 20 mM phosphate buffer of pH 7.0 for storage.

Results of the SDS-PAGE gel electrophoresis show that: non-denaturing electrophoresis of the positive antibody IMAB362-similar-mG2a shows its protein molecular weight is about 155 KD, the purity by SDS-PAGE is greater than 90%.

### Example 2 Synthesis of the CLDN18.2 antigen gene and construction of the expression vector

The sequence design of the fusion protein expressing the human CLDN18.2 antigen is shown in Table 1 below:

**Table 1 CLDN18.2 sequence design**

| **No.** | **Sequence name** | **Sequence meaning** | **Amino acid sequence** | **Nucleotide sequence** |
|---|---|---|---|---|
| 1 | 18A2-loopl | Encoding the sequence of extracellular loop 1 of CLDN18.2 | SEQ ID NO 2 | SEQ ID NO 1 |
| 2 | 18A2-TM-Loopl | Encoding the sequence of extracellular loop 1 comprising the transmembrane domain of CLDN18.2 | SEQ ID NO 4 | SEQ ID NO 3 |
| 3 | CpG | Unmethylated cytosine guanine dinucleotide palindrome | | SEQ ID NO 5 |
| 4 | CLDN18.2-FL | Encoding the full-length protein sequence of the human CLDN18.2 | SEQ ID NO 8 | SEQ ID NO 6 |
| | | | | SEQ ID NO 7 |
| 5 | CLDN18.1-FL | Encoding the full-length protein sequence of the human CLDN18.1 | SEQ ID NO 10 | SEQ ID NO 9 |

Eukaryotic codon optimization was carried out for the fusion protein sequence of the human CLDN18.2 antigen designed above, and restriction sites such as NotI and tPA signal peptide sequences were added to the 5' end of the gene, while restriction sites such as XhoI and Myc-His fusion protein tag were added to the 3' end of the gene. The above SEQ ID NO 1, 3, 5, 6 sequences were synthesized and constructed into the pcDNA3.1(+) vector to obtain DNA plasmids expressing the human CLDN18.2 antigen. The plasmids include: pcDNA3.1-loop1, pcDNA3.1-TML, pcDNA3.1-CpG-loop1 and pcDNA3.1-CLDN18.2-FL.

The above SEQ ID NO 7, 9 sequences were optimized and then synthesized by Bao Biological Engineering (Dalian) Co., Ltd. (hereinafter referred to as Bao Biological Company), and constructed into the lentiviral vector pLVX-NS-ZsGreen (Bao Biological Company). The constructed lentiviral plasmids pLVX-CLDN18.2-ZsGreen and pLVX-CLDN18.1-ZsGreen were used to further construct a cell line stably expressing HEK293-CLDN18.2 fusion green fluorescent protein, and to construct the stable cell line expressing HEK293-CLDN18.1 fusion green fluorescent protein.

The amplified plasmids were used for restriction enzyme digestion identification, and the identification results show that the digested band of interest is consistent with the expected size, indicating that the construction is correct.

### Example 3 Expression of the CLDN18.2 antigen protein

### 1. Transient transfection with plasmid DNA in vitro

HEK293T cells (Institute of Biochemistry and Cell Biology, Chinese Academy of Sciences, hereinafter referred to as Shanghai Institute of Cell Biology) were transiently transfected with the plasmid amplified in Example 2 *in vitro,* and analyzed at the protein level. The detailed experimental steps are as follows: one day before transfection, HEK293T cells were plated in six-well plates at a seeding density of 3x10⁵ cells/cm² and cultured (culture medium composition: DMEM culture medium with 10% fetal bovine serum + 1% penicillin-streptomycin) overnight to make the cells fully attached to the plate on the day of transfection, and to make the cells reach 70%-90% confluency (the size of the six-well plate is 9 cm² per well, full confluency means about 2-2.5x10⁶ cells/well); the DNA plasmids were mixed with PEI cationic transfection reagent (sigma) or liposomes (Lipo3000, Thermo) to prepare the DNA plasmids transfection complex, added to cells and let stand at room temperature for 5-20 minutes, incubated at 37° C, 5% CO₂ for 4-5 hours; complete culture medium containing serum was added to the six-well plate and cultured for 48-72 hours to transiently express the exogenous gene in eukaryotic cells *in vitro.*

### 2. Western Blot immunoblotting hybridization

HEK293T cells post transient transfection were collected, and the target protein was hybridized with rabbit anti-human Claudin18 antibody (Thermo, Cat: 700178) at room temperature for 2 hours; washed 3 times with PBST, hybridized with alkaline phosphatase-conjugated goat anti-rabbit IgG (Thermo, Cat: 31346) at room temperature for 1 hour; Western Blot immunoblotted to identify the expression level of the target protein: with Lipo liposome transfection system, the plasmids can successfully express the target protein CLDN18.2-loop1 and CLDN18.2-TM-loop1, with molecular weights of 11 KD and 14 KD, respectively, pcDNA3.1-CLDN18.2-FL plasmid successfully expressed the target protein CLDN18.2 with a molecular weight of 29 KD.

### Example 4 Preparation of the mouse hybridomas

### 1. Animal immunization:

The plasmid DNAs were extracted and prepared according to the manual of EndoFree Plasmid Maxi Kit from QIAGEN, including pcDNA3.1-loop1 (hereinafter referred to as Loop1), pcDNA3.1-TM-Loop1 (hereinafter referred to as TML), pcDNA3.1-CpG-loop1 (hereinafter referred to as Loop1-C). The plasmids were grouped according to the following Table 2 for mouse immunization, wherein the SJL mice were purchased from Beijing Vital River Laboratory Animal Technology Co., Ltd., and the BALB/c mice and the C57 mice were purchased from Huafukang Biological Products Center, Xuanwu District, Nanjing. For immunization, refer to the method described in Zhiwei Chen et al. (Z. Chen., et al., J Clin Invest. 123(6): 2629-2642 (2013)), with appropriate modifications, detailed as follows: DNA plasmids were injected into the left and right hind limb muscles of the mouse at two points respectively through the In Vivo Gene Importer (Shanghai Taresa Biotechnology Co., Ltd.), and the same amount of DNA plasmids was injected at each point, with an injection volume of 50µl. Immediately after the injection, the electrode of the importer was used to input electrical pulse stimulation on the injection sites of the mouse, the voltage was set to 36V, and 6 pulses in total. Electrical pulse stimulation can promote the uptake of DNA plasmids by mouse muscle cells and increase the level of mouse immune responses to DNA, especially the level of humoral immune responses. DNA immunization was injected once every 2 weeks for a total of three immunizations. Seven days after the second and the third immunizations, blood was collected from the fundus venous plexus of the mouse for flow cytometry analysis, and when a clear CLDN18 positive signal was detected in serum antibodies, HEK293-CLDN18.2 cells were used as antigens for the mouse, prepared as cell suspension of 5x10⁷ cells/ml, injected with 100 µl (5x10⁶ cells) into the intraperitoneal cavity of the mouse to boost immunity.

**Table 2 Mouse immunization groups**

| Group | Type of mice | Number of mice | DNA prime immunization | | | Cell boost immunization |
|---|---|---|---|---|---|---|
| | | | Week 0 | Week 2 | Week 4 | |
| 1 | BALB/c, female, SPF, 6-8 weeks | 3 | Loopl, 50µg per animal | | | 3-4 days before the fusion of splenocytes, 5x10⁶ HEK293-CLDN18.2 cells per animal were injected |
| 2 | | 3 | Loopl-C, 50µg per animal | | | |
| 3 | | 3 | TML, 50µg per animal | | | |
| 4 | SJL, female, SPF, 4-6 weeks | 3 | Loopl, 100µg per animal | | | |
| 5 | | 4 | Loopl-C, 100µg per animal | | | |
| 6 | | 3 | TML, 100µg per animal | | | |
| 7 | C57, female, SPF, 6-8 weeks | 3 | Loopl, 25µg per animal | | | intraperitoneally |
| 8 | | 3 | Loopl-C, 25µg per animal | | | |
| 9 | | 3 | TML, 25µg per animal | | | |

### 2. FACS detection of the immune serum:

One week after the second and the third DNA immunizations, blood was collected from the fundus venous plexus of the mouse to prepare the immune serum, respectively. The serum was diluted 1:50 in volume using the blocking solution (2% BSA in PBS). Human gastric cancer cells NUGC4 (Nanjing Co-bioer Biosciences Co., Ltd.) were resuscitated and cultured in advance, in 1640 culture medium containing 10% fetal bovine serum, 37°C, 5% CO₂. When the cells were in good condition and the cell growth confluence was 70-80%, gastric cancer cell digestion solution (CHI Scientific, Inc.) was used to digest the cells at 37°C for 15 minutes. Complete 1640 culture medium was used to terminate the digestion, centrifuged at 1000 rpm for 5 minutes, the supernatant was discarded. Appropriate amount of the blocking solution was added to resuspend the cells, the cell density was adjusted to 1×10⁶ cells/ml. 100 µl of NUGC4 cells were mixed with 100 µl of diluted mouse serum, incubated at 4°C for 1 hour; washed twice with Cell Stain Buffer (Biolegend), centrifuged at 1000 rpm for 5 minutes at each time, and the supernatant was discarded. 100µl of PE-conjugated goat anti-mouse antibodies (Biolegend) were added, incubated at 4°C for 1 hour; washed twice with Cell Stain Buffer (Biolegend), centrifuged at 1000 rpm for 5 minutes at each time, and the supernatant was discarded. 300µl of Cell Stain Buffer was added to resuspend the cells, detected with the flow cytometry analyzer.

After the second DNA immunization, an obvioius deviation of the positive signal was detected in mouse number #0103, indicating that this mouse produced specific antibody responses against CLDN18. After the third DNA immunization, positive signals were detected in all mice, wherein the CLDN18 positive rates in mice with numbers #0101, #0102, #0103, #0203, #0301, and #0303 were greater than 50%, indicating these mice produced obvious antibody immune responses, and there were relatively high levels of anti-CLDN18 antibodies in their serum after the third DNA immunization.

### 3. Preparation of the mouse hybridoma cells

Preparation of mouse myeloma cell suspension: mouse myeloma cells SP2/0 were resuscitated two weeks in advance, cultured and passaged in 1640 culture medium containing 10% fetal bovine serum at 37°C, 5% CO₂. On the day of fusion, the status of SP2/0 cells was observed. The cell confluence of SP2/0 cells was 70%-80% by microscopic examination, therefore it can be determined that the cells were in the logarithmic growth phase. SP2/0 cells were collected by centrifugation at 300 g for 5 minutes, washed once with 20 ml of PBS, 1000 rpm, 5 min, the supernatant was discarded; cells were resuspended with an appropriate amount of 1640 culture medium, and the concentration of SP2/0 cells was adjusted to 1×10⁷ cells/ml for later use.

Preparation of mouse feeder cell suspension: the day before fusion, 20 healthy Balb/c mice aged 10-12 weeks were sacrificed through cervical dislocation, the mouse abdominal skin was cut open using sterile surgical scissors, a small cut was made across the abdomen first, and then the chest war cut longitudinally to expose the mouse abdomen. Collection of peritoneal macrophages: the mouse abdomen was disinfected with alcohol cotton ball first, and then the mouse was injected intraperitoneally with 5 ml of 1640 basal culture medium using a syringe, the mouse abdomen was massaged gently for 5 minutes, then the fluid in the abdominal cavity was aspirated with the syringe. Repeated once, 3 ml of 1640 basal culture medium was injected and then aspirated (or the peritoneum was cut open, then the remaining macrophages were aspirated using pasteur pipettes). The collected macrophages were centrifuged at 1500 rpm for 10 minutes, the supernatant was discarded, the cells were resuspended using 10 ml (1640 culture medium containing 10% fetal bovine serum) for later use.

Preparation of mouse spleen lymphocyte suspension: 3-4 days after cell boost immunization, splenocytes of the immunized mice were taken to fuse with SP2/0. The mice were sacrificed through cervical dislocation, the peritoneum was cut open sterilely, the spleen was taken out and the surrounding connective tissue was peeled off. Mouse splenocytes were grinded and then filtered with a 70µm sieve into a 50 ml centrifuge tube pre-filled with 15 ml of 1640 culture medium to prepare the single cell suspension; 1500 rpm, 5 min, washed with 1640 culture medium, the supernatant was discarded; 4 ml of red blood cell lysis solution (Sigma) was added to lyse red blood cells for about 2 min; an equal volume of 1640 culture medium was added, centrifuged at 1500 rpm for 5 min, the upper layer of red blood cells was discarded; resuspended using 20 ml of PBS, washed twice, centrifuged at 1500 rpm for 5 min. After the second wash, the cells were resuspended with 15 ml of PBS, and if there were residual agglomerated connective tissue at this time, all the cells can be filtered through sieves and transferred to a new 50 ml centrifuge tube; the spleen lymphocytes were counted, resuspended with an appropriate amount of 1640 culture medium, the splenocytes were adjusted to 1×10⁷ cells/ml.

1×10⁸ spleen cells were mixed with SP2/0 cells in the ratio of 1:1, centrifuged at 1500 rpm for 5 minutes, the supernatant was discarded. Cells were resuspended and mixed well with BTX electrofusion buffer, and electrofusion operations were performed according to the manual of the BTX electrofusion instrument. The fused cells were allowed to stand at room temperature for 30 minutes, and 1640 selective culture medium containing 1xHAT and 10% fetal bovine serum was added, the feeder cell suspension prepared in advance was also added, plated to a total of 200 96-well plates, incubated at 37°C, 5% CO₂ for 5-7 days. Then, the fused hybridoma cell clones were cultured with refreshed culture medium containing 1×HT.

### Example 5 Preparation of the positive cell line

### a. Construction of CHO-K1-CLDN18.2 stable cell line

CHO-K1 cells (Shanghai Institute of Cell Biology) were resuscitated one week in advance, and cultured in DME/F12 complete culture medium containing 10% fetal bovine serum at 37°C, 5% CO₂. The plasmid used to transfect the cells was pcDNA3.1-CLDN18.2-FL (see SEQ ID NO 6 for the synthetic sequence). On the day of transfection, the growth density of CHO-K1 cells reached 80%-90%. According to the manual of the Biorad Gene Pulser Xcell electroporation instrument, the plasmids were transfected into CHO-K1 cells, and all the transfected cells were transferred to petri dishes with pre-heated complete culture medium, let stand at 37°C, 5% CO₂ overnight to culture. Next day, G418 (Sigma) was added at a final concentration of 1 mg/ml for resistance screening for 3-4 days, the CLDN18.2 expression level of cells was detected after passaging for at least 5 generations using complete culture medium containing G418 resistance to stabilize the clone. The results show that the transfected cells stably express the human Claudin18.2 after passaging for 5 generations compared with CHO-K1 empty cells, by using the positive antibody IMAB362-similar-hG1 (final concentration of 10 µg/ml) to be incubated with the cells and detected with flow cytometry.

### b. Construction CHO-K1-CLDN18.1 stable cell line

Using the pLVX-CLDN18.1-ZsGreen plasmid as a template, primer sequences SEQ ID NO 15 and SEQ ID NO 16 were designed, the PCR system was set up according to manual of PrimeStar (Takara), the PCR program was set to denature at 98°C for 30 seconds, anneal at 60°C for 30 seconds, extend at 72°C for 1 minute and amplify for 30 cycles to obtain the CLDN18.1-FL target gene fragment (see SEQ ID NO 17). The target gene and pcDNA3.1(+) vector were digested with the two enzymes BamHI and EcoRI, ligated with T4 ligase (Takara) at 16°C overnight. The ligation product was transformed into E.coli, and the plasmid pcDNA3.1-CLDN18.1 was extracted with miniprep kit. The plasmid was electrotransfected into CHO-K1 cells according to the steps above, and the cells were cultured and passaged in culture medium with G418 resistance, Western Blot results show that the transfected cells can be stained positive with anti-CLDN18 antibody (Thermo), while FACS analyses show that the transfected cells exhibit negative fluorescent signals after incubating with the CLDN18.2 positive antibody IMAB362-similar-hG1 at a concentration of 10 µg/ml. It indicates that the transfected CLDN18.1 cells are successfully constructed.

### Example 6 Antibody screen of the mother clones of hybridomas

The first round of primary screening was carried out for the mother clones of hybridoma cells obtained after the fusion, and the screening methods include cellular ELISA and FACS to detect supernatant of the hybridoma cell culture, respectively, from which the mother clones of the CLDN18 positive hybridomas were screened. The CLDN18 positive clones in the primary screen were amplified, then the second round of re-screening was carried out, from which mother clones of hybridomas showing CLDN18.2 positive and CLDN18.1 negative in FACS were screened. After two rounds of cellular ELISA and FACS screening of hybridoma antibodies, a total of 9 mother clones secreting CLDN18.2-specific antibodies were screened out of hundreds of mother clones, which can positively bind with HEK293-CLDN18.2 cells, while the binding to HEK293 -CLDN18.1 cells was negative.

### Example 7 Culture and screening of subclones

### 1. Culture of subclones

Monoclonal clones were prepared from the hybridoma cells obtained from screenings in Example 6. Hybridoma cells were resuspended in 24-well plates to prepare the cell suspension. Cells were counted for each clone, the final cell concentration was adjusted to 1×10⁴ cells/ml using 1640 complete culture medium; 60µl of cell suspension was added to 140µl of 1640 complete culture medium to achieve a total of 200µl, respectively, mixed well; the semi-solid culture medium D (StemCell) stored at -20°C was taken out one day in advance, thawed at 4°C, and placed at 37°C for 1 hour before use. 2.5 ml of semi-solid culture medium was taken and mixed thoroughly with 200µl of cell suspension, let stand at room temperature for 15 minutes; the well mixed cell suspension in semi-solid culture medium was transferred to 6-well cell culture plates (Corning), the inoculation quantity being 600 cells per well, an appropriate amount of sterile water was added around the plates, placed at 37°C, 5% CO₂ for 5-7 days. When the cells in the plates grew into dense spherical colonies of monoclonal cells, then they were aspirated using 10µl pipette tips and transferred to 200µl of 1640 culture medium containing 1×HT, 10%CloneEasy and 10% fetal bovine serum, cell suspension was thoroughly mixed and incubated at 37°C, 5% CO₂ for 2-3 days.

### 2. Screening of subclones

To identify the CLDN18.2 specificity of antibodies in the cell culture supernatant of the subclones, the screening method used was cellular ELISA to detect the positive binding of supernatant antibodies to CLDN18.2 and CLDN18.1 stably transfected cells. The detailed protocol was as follows:
HEK293-CLDN18.1 and HEK293-CLDN18.2 cells were cultured separately with DMEM complete culture medium containing 10% fetal bovine serum at 37°C, 5% CO₂. When the cells grew to 80%-90% confluency, they were digested with 0.5mM EDTA-PBS non-trypsin digestion solution at room temperature, and centrifuged at 1000 rpm for 5 minutes, the supernatant was discarded. Resuspended in DMEM complete culture medium, and the cell concentration was adjusted to 6×10⁵ cells/ml. Cells were plated on 96-well cell plates (Corning) pre-coated with polylysine, 100 µl per well, incubated at 37°C, 5% CO₂ for 24 hours, making cells evenly and completely plated on the bottom of the well. The culture supernatant was aspirated, and the cells were fixed with 4% paraformaldehyde at room temperature for 20 minutes, washed 3 times with 200µl PBS, 2 minutes at each time. The well plates were blocked with 200µl of 3%BSA-PBS at 37°C for 1 hour. 100µl of subclone culture supernatant was taken and added into the well plate, incubated at 37°C for 1 hour, washed 5 times with PBS. Incubation with HRP-anti mouse IgG+IgM (Jackson) diluted 1:10000 in blocking solution was performed at 37°C for 1 hour followed by washing 5 times with PBS. 100µl of TMB one-component color developing solution was added, protected from light at room temperature for 5-10 minutes, terminated with 2M sulfuric acid, and OD450 was read on the microplate reader. The results are shown in Table 3, subclones of which the hybridoma culture supernatant show strong positive binding to CLDN18.2 (that is, the OD value is 50% higher than the OD value of the positive control) include: S3F10C5, S5H3A6, S5H3B6, S5H3D6, S6F10E7, S6H9B8, S6H9C8, C40B10D9, C42B12D10 and C58B11E11. Among these subclones, S5H3A6, S5H3B6, S5H3D6, C40B10D9, C42B12D10 and C58B11E11 show negative binding to CLDN18.1 cells.

**Table 3 Results of subclone screening using cellular ELISA**

| No. | Subclone No. | OD450 (CLDN18.2) | OD450 (CLDN18.1) |
|---|---|---|---|
| 1 | mG2a (positive control) | 1.286 | 0.168 |
| 2 | B4B10G3 | 0.343 | 0.200 |
| 3 | S3F10B5 | 0.615 | 0.198 |
| 4 | S3F10C5 | 0.948 | 0.278 |
| 5 | S3F10E5 | 0.504 | 0.213 |
| 6 | S5H3A6 | 1.928 | 0.192 |
| 7 | S5H3B6 | 1.379 | 0.152 |
| 8 | S5H3D6 | 1.636 | 0.166 |
| 9 | S6F10E7 | 0.818 | 0.350 |
| 10 | S6F10H7 | 0.452 | 0.205 |
| 11 | S6H9B8 | 2.353 | 1.495 |
| 12 | S6H9C8 | 2.165 | 1.303 |
| 13 | C40B10D9 | 0.664 | 0.114 |
| 14 | C42B12D10 | 0.760 | 0.172 |
| 15 | C58B11E11 | 0.671 | 0.197 |

Clonal expansion was carried out for subclones B4B10G3, S5H3B6, S6H9B8, C40B10D9, C42B12D10 and C58B11E11. Cells in the 96-well plate were resuspended with 200µL pipettes, all the cell suspension was transferred into the 24-well cell plate (Corning), 1 ml of 1640 culture medium containing 2% CloneEasy, 10% fetal bovine serum was added, placed in a 37°C, 5% CO₂ incubator to incubate for 3-4 days.

### Example 8 Identification of antibody subtypes

The subclone cell culture supernatant to be detected was added to identify the subtypes of the mouse antibodies according to the manual of the mouse antibody subtype identification kit SBA Clonotyping System (SouthernBiotech). The identification results are as follows:
Subclone B4B10G3, the subtype of the heavy chain constant region of the antibody is the murine IgG2b; the subtype of the light chain constant region of the antibody is the murine Kappa chain;
Subclone S5H3B6, the subtype of the heavy chain constant region of the antibody is the murine IgM; the subtype of the light chain constant region of the antibody is the murine Kappa chain;
Subclone S6H9B8, the subtype of the heavy chain constant region of the antibody is the murine IgG3; the subtype of the light chain constant region of the antibody is the murine Kappa chain;
Subclone C58B11E11, the subtype of the heavy chain constant region of the antibody is the murine IgG2c; the subtype of the light chain constant region of the antibody is the murine Kappa chain;
Subclone C40B10D9, the subtype of the heavy chain constant region of the antibody is the murine IgM; the subtype of the light chain constant region of the antibody is the murine Kappa chain;
Subclone C42B12D10, the subtype of the heavy chain constant region of the antibody is the murine IgM; the subtype of the light chain constant region of the antibody is the murine Kappa chain;

### Example 9 Sequencing of the variable region of the antibody

Monoclonal hybridoma cells in the 24-well plate were collected, adjusted to 1×10⁶ cells/ml, 1 ml in total, centrifuged at 1000 rpm for 5 minutes, the supernatant was discarded. Miniprep of total RNA of cells was carried out according the manual of RNA extraction kit (Cat:#74134) from QIAGEN, NanoDrop nucleic acid quantitative analyzer was used to determine the RNA concentration. Next, 2 µg of total RNA was reverse transcribed into cDNA according to the Takera Reverse Transcription Kit (Cat:#6110A), stored at -80°C for later use. Using cDNA as template, the PCR systems were prepared and the PCR instrument programs were set up for sequencing the antibody light and heavy chains respectively according to manual of PremixTaq enzyme from Takara. The primers used in the PCR system were synthesized by Suzhou GENEWIZ Inc., and detailed primer sequences are as follows:
heavy chain sequencing primer VH-Fs including:
   VH-F1 (comprising primer sequences SEQ ID NO 18 and SEQ ID NO 19), VH-F2 (comprising primer sequences SEQ ID NO 20, SEQ ID NO 21, SEQ ID NO 22, and primer sequences SEQ ID NO 23, SEQ ID NO 24, SEQ ID NO 25), VH-F3 (primer sequence SEQ ID NO 26)
heavy chain sequencing primer VH-Rs including:
   VH-R1 (primer sequence SEQ ID NO 27), VH-R2 (primer sequence SEQ ID NO 28)
light chain sequencing primer VL-Fs including:
   VL-F1 (primer sequence SEQ ID NO 29), VL-F2 (comprising primer sequences SEQ ID NO 30 and SEQ ID NO 31), VL-F3 (comprising primer sequences SEQ ID NO 32, SEQ ID NO 33, SEQ ID NO 34)
light chain sequencing primer VL-Rs including:
   VL-R (primer sequence SEQ ID NO 35)

The PCR products obtained by PCR amplification were entrusted to Suzhou GENEWIZ Inc. for sequencing, and the sequencing results are shown in Table 4.

**Table 4 Table of nucleotide sequences of subclones**

| Subclone | Nucleotide sequence | |
|---|---|---|
| | Heavy chain variable region | Light chain variable region |
| B4B10G3 | SEQ ID NO 36 | SEQ ID NO 37 |
| S5H3B6 | SEQ ID NO 38 | SEQ ID NO 39 |
| S6H9B8 | SEQ ID NO 40 | SEQ ID NO 41 |
| C58B11E11 | SEQ ID NO 42 | SEQ ID NO 43 |
| C40B10D9 | SEQ ID NO 44 | SEQ ID NO 45 |
| C42B12D10 | SEQ ID NO 46 | SEQ ID NO 47 |

### Example 10 Preparation of the chimeric antibodies

According to the variable region sequences of the murine-derived antibodies obtained in Example 9, the constant region of the human IgG1 and the variable region of the mouse antibodies were combined to construct the human-murine chimeric antibodies through gene synthesis to further study the antibody affinity and antibody properties.

### a. Synthesis of the human-murine chimeric antibody genes

Suzhou GENEWIZ Biotechnology Co., Ltd. was entrusted to synthesize optimized chimeric antibody genes and construct them into the eukaryotic expression vector pcDNA3.1(+), and the chimeric antibodies were named as 1CHI, 3CHI, 7CHI, 17CHI, 18CHI, 19CHI, respectively. The antibody variable region sequence corresponding to each chimeric antibody is shown in Table 5 below:

**Table 5 Sequences of the chimeric antibodies**

| Monoclonal antibody | Chimeric antibody | Amino acid sequence | | Nucleotide sequence | |
|---|---|---|---|---|---|
| | | Heavy chain variable region | Light chain variable region | Heavy chain variable region | Light chain variable region |
| B4B10G3 | 1CHI | SEQ ID NO 48 | SEQ ID NO 49 | SEQ ID NO 36 | SEQ ID NO 37 |
| S5H3B6 | 3CHI | SEQ ID NO 50 | SEQ ID NO 51 | SEQ ID NO 52 | SEQ ID NO 53 |
| S6H9B8 | 7CHI | SEQ ID NO 54 | SEQ ID NO 55 | SEQ ID NO 56 | SEQ ID NO 57 |
| C58B11E11 | 17CHI | SEQ ID NO 58 | SEQ ID NO 59 | SEQ ID NO 60 | SEQ ID NO 61 |
| C40B10D9 | 18CHI | SEQ ID NO 62 | SEQ ID NO 63 | SEQ ID NO 64 | SEQ ID NO 65 |
| C42B12D10 | 19CHI | SEQ ID NO 66 | SEQ ID NO 67 | SEQ ID NO 68 | SEQ ID NO 69 |

### b. Expression and protein purification of the chimeric antibodies

Plasmids containing the light and heavy chain genes of the above chimeric antibodies were transiently transfected into HEK293E cells (Shanghai Institute of Cell Biology) in the ratio of 1:1. The cells are shaking cultured for 7 days with shaking speed of 110 rpm, 37°C, 8% CO₂; centrifuged at 4500 rpm for 20 minutes; and the supernatant protein was harvested. Purification by ProteinA affinity chromatography is performed as follows: the AKTA chromatography system and columns were cleaned with 0.1M NaOH solution for 30 minutes, rinsed clean with ultrapure water; equilibrated with 10 column volumes of 20mM sodium phosphate buffer of pH 7.4; the flow rate was set to 2 ml/min, samples were loaded; the columns were cleaned with 10 column volumes of the equilibration buffer; eluted with 3-5 column volumes of 50mM acetate buffer of pH 3.4, the eluted protein was collected; concentrated via ultrafiltration to displace the antibodies in 50 mM acetate buffer of pH 5.55 for storage.

Among them, the SDS-PAGE gel electrophoresis of the chimeric antibodies 3CHI, 18CHI and 19CHI shows that the purity of the chimeric antibodies 3CHI, 18CHI and 19CHI by SDS-PAGE are all greater than 90%.

### Example 11 Determination of the specific binding of the chimeric antibodies to CLDN18.2

HEK293-CLDN18.1 and HEK293-CLDN18.2 cells were resuscitated two weeks in advance, cultured with DMEM complete culture medium containing 10% fetal bovine serum at 37°C, 5% CO₂. When the cells grew to 80%-90% confluency, they were digested with 0.5mM EDTA-PBS (Sigma) at room temperature, centrifuged at 1000 rpm for 5 minutes, the supernatant was discarded. Cells were resuspended in DMEM complete culture medium, the cell concentration was adjusted to 6×10⁵ cells/ml. Cells were plated on 96-well cell plates (Corning) pre-coated with polylysine at 2.5 µg/well, 100 µl per well, incubated at 37°C, 5% CO₂ for 24 hours, making cells evenly and completely plated on the bottom of the well. The supernatant was aspirated, and the cells were fixed with 4% paraformaldehyde at room temperature for 20 minutes, washed 3 times with 200 µl PBS, 2 minutes at each time. The well plates were blocked with 200 µl of 3%BSA-PBS at 37°C for 1 hour. Each chimeric antibody was diluted with 3%BSA-PBS to a concentration of 6 µg/ml, and diluted in a 6-fold gradient to a total of 7 concentrations. At the same time, IMAB362-similar-hG1 diluted in the same concentration gradient as the positive control, and human IgG1, kappa isotype control antibody (hereinafter referred to as Isotype, provided by CrownBio (Taicang) Co., Ltd.) as the negative control were added to HEK293 -CLDN18.2 and HEK293-CLDN18.1 cell plates, respectively. The cells were incubated at 37°C for 1 hour, washed 5 times with PBS. Incubation with HRP-anti human IgG (Jackson) diluted 1:10000 in blocking solution was performed at 37°C for 1 hour followed by washing 5 times with PBS. 100µl of TMB one-component color developing solution was added, protected from light at room temperature for 5-10 minutes, terminated with 50µl of 2M sulfuric acid, OD450 was read on the microplate reader. Data processing: the original data was put into GraphPad 5.0 software for plotting and calculation. The results are shown in Table 6.

**Table 6 Determination of the specific binding of the chimeric antibodies to CLDN18.2**

| **Antibody No.** | **EC50 (nM)** |
|---|---|
| Positive control (IMAB362-similar-hG1) | 0.09 |
| Negative control (Isotype) | N.A |
| 1CHI | 0.55 |
| 3CHI | 0.08 |
| 17CHI | 2.44 |
| 18CHI | 1.75 |
| 19CHI | 29.90 |

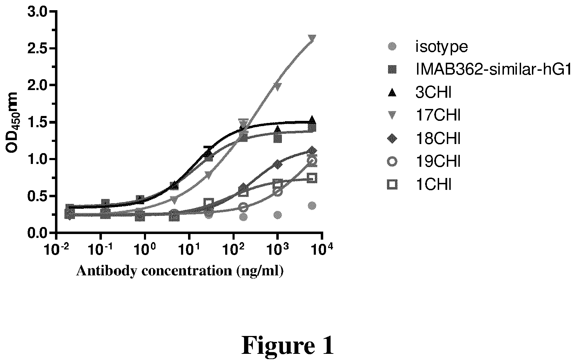

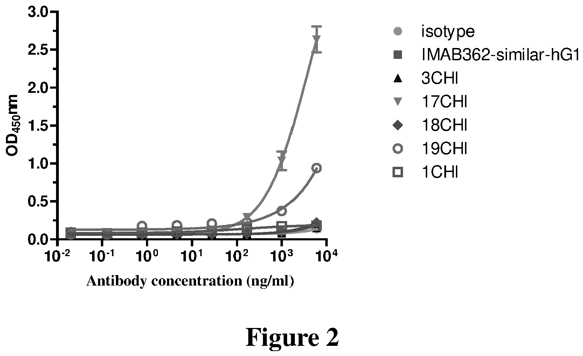

The binding of each chimeric antibody to CLDN18.2 cells and CLDN18.1 cells is shown in Figure 1 and Figure 2, wherein the Isotype is a negative control and IMAB362-similar-hG1 is a positive control. The results show that the binding curve of each chimeric antibody is concentration gradient dependent. Among them, 17CHI exhibits strong positive binding to both CLDN18.2 and CLDN18.1, whereas 19CHI exhibits weak positive binding to CLDN18.2 and CLDN18.1, while 1CHI, 3CHI and 18CHI antibodies can specifically bind to CLDN18.2 without binding to CLDN18.1. At the same time, among the anti-CLDN18.2 specific antibodies, the EC50 value of 3CHI antibody is relatively low, and similar to the EC50 value of the positive control antibody IMAB362-similar-hG1, showing relatively strong activity of specifically binding to CLDN18.2.

### Example 12 Affinity maturation of the chimeric antibody

The chimeric antibody 3CHI with the closest affinity to the positive control antibody in Example 11 was selected for affinity maturation and screening *in vitro.* The phage Fab antibody library was mainly used for screening to obtain antibodies that can maintain the CLDN18.2 specificity while have increased affinity.

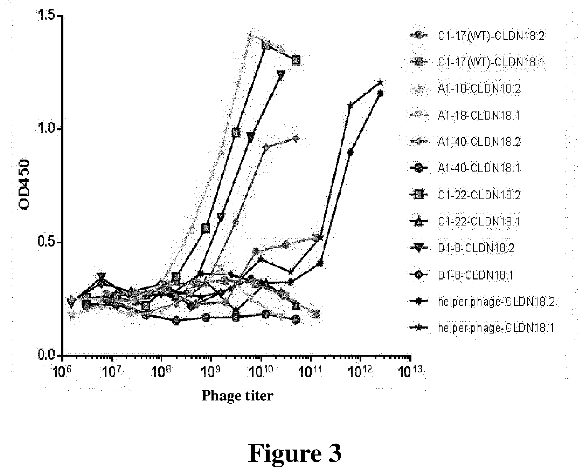

The results of the phage screening are shown in Figure 3, phage antibodies C1-22, A1-18, or the like all exhibit higher CLDN18.2 binding activity than the original 3CHI phage antibody (WT). CDR sequences with amino acid site mutations were obtained after sequencing the phages, and these mutation sites were recombined to construct chimeric antibodies with the constant region of human IgG1. After renumbering and naming the chimeric antibodies derived from phages, whether the 30 chimeric antibodies above specifically bind to CLDN18.2 was identified by cellular ELISA. The results are shown in Table 7, there are 18 specific antibodies with negative binding to CLDN18.1 and positive binding to CLDN18.2 in the table, and their amino acid sequences are shown in Table 11.

**Table 7 Screening results of the phage-derived chimeric antibodies**

| Identification content | Antibody name |
|---|---|
| CLDN18.1 positive and CLDN18.2 positive | 310CHI, 311CHI, 314CHI, 316CHI, 317CHI, 318CHI, 320CHI, 323CHI, 325CHI, 326CHI, 327CHI, 329CHI |
| CLDN18.1 negative and CLDN18.2 positive | 301CHI, 302CHI, 303CHI, 304CHI, 305CHI, 306CHI, 307CHI, 308CHI, 309CHI, 312CHI, 313CHI, 315CHI, 319CHI, 321CHI, 322CHI, 324CHI, 328CHI , 330CHI |

**Table 8 List of amino acid sequences of the specific chimeric antibodies**

| **No.** | **Antibody name** | Heavy chain variable region | Light chain variable region |
|---|---|---|---|
| 1 | 301CHI | SEQ ID NO 50 | SEQ ID NO 70 |
| 2 | 302CHI | SEQ ID NO 50 | SEQ ID NO 71 |
| 3 | 303CHI | SEQ ID NO 72 | SEQ ID NO 73 |
| 4 | 304CHI | SEQ ID NO 74 | SEQ ID NO 75 |
| 5 | 305CHI | SEQ ID NO 50 | SEQ ID NO 76 |
| 6 | 306CHI | SEQ ID NO 77 | SEQ ID NO 78 |
| 7 | 307CHI | SEQ ID NO 74 | SEQ ID NO 70 |
| 8 | 308CHI | SEQ ID NO 74 | SEQ ID NO 71 |
| 9 | 309CHI | SEQ ID NO 74 | SEQ ID NO 76 |
| 10 | 312CHI | SEQ ID NO 72 | SEQ ID NO 76 |
| 11 | 313CHI | SEQ ID NO 77 | SEQ ID NO 70 |
| 12 | 315CHI | SEQ ID NO 77 | SEQ ID NO 76 |
| 13 | 319CHI | SEQ ID NO 79 | SEQ ID NO 70 |
| 14 | 321CHI | SEQ ID NO 79 | SEQ ID NO 76 |
| 15 | 322CHI | SEQ ID NO 50 | SEQ ID NO 80 |
| 16 | 324CHI | SEQ ID NO 50 | SEQ ID NO 81 |
| 17 | 328CHI | SEQ ID NO 79 | SEQ ID NO 80 |
| 18 | 330CHI | SEQ ID NO 79 | SEQ ID NO 81 |

Human gastric cancer cells NUGC4 and stably transfected cells CHO-K1-CLDN18.1 (prepared in Example 5) were used respectively, to verify the specificity and affinity of the anti-CLDN18.2 antibodies above at the cellular level by FACS technology. The results are shown in Table 9. After antibody affinity maturation and screening *in vitro,* each antibody can bind to the CLDN18.2 target on NUGC4 cells, but not to CHO-K1-CLDN18.1 cells, as compared with the original 3CHI antibody. According to the level of antibody affinity indicated by the MFI (Mean Fluorescence Intensity) of FACS, the 330CHI antibody shows a significant increase in its affinity for targeting NUGC4 cells, as compared with the positive control antibody IMAB362-similar-hG1.

**Table 9 FACS detection results of the affinity-matured antibodies**

| Antibody No. | MFI (CLDN18.2) | MFI (CLDN18.1) |
|---|---|---|
| Isotype | 93 | 219 |
| IMAB362-similar-hG1 | 2179 | 59 |
| 3CHI | 2509 | 76 |
| 17CHI | N.A | 14455 |
| 301CHI | 44420 | 114 |
| 303CHI | 38241 | 85 |
| 305CHI | 38913 | 68 |
| 309CHI | 42495 | 78 |
| 315CHI | 42301 | 65 |
| 321CHI | 46327 | 299 |
| 322CHI | 44777 | 57 |
| 324CHI | 36030 | 248 |
| 330CHI | 63666 | 49 |

The affinity of the anti-CLDN18.2 antibodies above were verified at the protein level by the Fortebio molecular interaction instrument: first, the antibody solution and the antigen dilution were prepared by diluting the chimeric antibody with SD buffer (PBS solution containing 0.02% Tween20 and 0.1% BSA) to a final concentration of 5 µg/ml; and by diluting the antigen protein Claudin18.2-His with SD buffer in a 4-fold concentration gradient to make the concentrations 10 µg/ml, 2.5 µg/ml, 0.625 µg/ml, 0 µg/ml, respectively. Next, antibodies were immobilized by the AHC sensor, and then combined with different concentrations of antigens, and determination of the affinity was performed according to the manual of Octet RED96.

The results show that the equilibrium dissociation constant (KD) of the binding of the positive control antibody IMAB362-similar-hG1 to CLDN18.2 protein at 37°C was 7.14 nM, and the KD values of 330CHI and 305CHI antibodies were 0.84 nM and 0.87 nM, respectively, indicating a significantly increase by an order of magnitude compared with the positive control (see Table 10).

**Table 10 Affinity test results of the affinity-matured antibodies**

| No. | Antibody name | Type of sensor | KD (M) | Kon (M⁻¹·s⁻¹) | Kdis (s⁻¹) |
|---|---|---|---|---|---|
| 1 | IMAB362-si | AHC | 7.14×10⁻⁹ | 5.59×10⁴ | 3.99×10⁻⁴ |
| | milar-hG1 | | | | |
| 2 | 330CHI | | 8.40×10⁻¹⁰ | 1.39×10⁵ | 1.17×10⁻⁴ |
| 3 | 305CHI | | 8.65×10⁻¹⁰ | 5.13×10⁴ | 4.44×10⁻⁵ |

### Example 13 Determination of the in-vitro ADCC activities of the chimeric antibodies

The day before the determination experiment, CHO-K1-CLDN18.2 cells (prepared in Example 5) were digested to be resuspended in complete culture medium, the cells were counted, and the density was adjusted to 1×10⁵ cells/ml, inoculated on 96-well plates in 100 µl/well, incubated overnight in a 37°C, 5% CO₂ incubator; on the day of the test, the phenol red-free 1640 culture medium (Gibco) was incubated to 37°C, and the antibody was diluted in a 5-fold gradient; 96-well plates plated with CHO-K1-CLDN18.2 were taken out, culture medium was discarded, 25 µl of phenol red-free 1640 was added to each well, the diluted antibody was added to the sample wells, making the final antibody concentrations being 10 µg/ml, 2 µg/ml, 0.4 µg/ml, 0.08 µg/ml, 0.016 µg/ml and 0.0032 µg/ml, respectively; equal amounts of IMAB362-similar-hG1 and human IgG1 Isotype control antibody (CrownBio) were added to the control wells, incubated in a 37°C, 5% CO₂ incubator for 1h;

Jurkat cells stably expressing the human CD16A protein and NFAT reporter gene protein (hereinafter referred to as Jurkat-CD16A/NF) were used as the effector cells, transferred to centrifuge tubes, centrifuged at 1000 rpm for 5 min at room temperature, the supernatant was discarded; resuspended and washed with 5 ml of phenol red-free 1640 culture medium once, centrifuged at 1000 rpm for 5 min, the supernatant was discarded; the cells were resuspended in phenol red-free 1640, and the cell density was adjusted to 8×10⁶ cells/ml;

2×10⁵ Jurkat-CD16A/NF cells were added to the well plate according to an effector-to-target ratio of 20:1 (that is, the ratio of the number of the effector cells to the target cells is 20:1), centrifuged at 200 g for 2 minutes to make the effector cells fully contacted with the target cells; incubated in a 37°C, 5% CO₂ incubator for 5-6 hours; 30 minutes before the detection, cell plates were recovered to room temperature, detection reagents were added according the manual of the Bio-Turbo Firefly Luciferase Assay Kit (Jiangsu Riogene Biotechnology Co., Ltd.), the supernatant to be detected was transferred to white flat-bottom 96-well plates, and fluorescent signals of the well plates were detected with Thermo Floskan Ascent FL fluorescence microplate reader. The original data was put into Graphpad 5.0 software for calculation and analysis, the results are shown in Table 11.

**Table 11 Determination results of the in-vitro ADCC activities of the chimeric antibodies**

| Group | EC50 (nM) | Top |
|---|---|---|
| Isotype | 13.48 | 6190 |
| IMAB362-similar-hG1 | 0.72 | 215966 |
| 3CHI | 0.54 | 211611 |
| 305CHI | 0.28 | 215996 |
| 330CHI | 0.22 | 214800 |

Compared with the Isotype negative control, the fluorescent signal of each chimeric antibody was dose-dependent with the concentration gradient. In the experimental group, the EC50 of the chimeric antibody 330CHI is 0.22 nM and the EC50 of the chimeric antibody 305CHI is 0.28 nM, indicating the ADCC EC50 activity of the chimeric antibodies was more than 3 times higher than that of the positive control antibody IMAB362-similar-hG1 (0.72 nM). As can be seen, the chimeric antibodies have improved cell binding activity and affinity against CLDN18.2, as well as effectively improved in-vitro ADCC activity effects of the antibodies.

### Example 14 Humanization of the antibody

The sequence of antibody 330CHI modified by affinity maturation *in vitro* in Example 12 was optimized by humanization. Sequence optimization by humanization and identification of antibody physical and chemical properties were entrusted to Hangzhou Healsun Biopharm Co., Ltd. In the sequence optimization by humanization, the source of the murine sequence of the variable region of the 3CHI antibody and 330CHI antibody was confirmed through the immune gene database (IMGT), and through homologous comparison, the FR region of the heavy chain variable region sequence of the 3CHI antibody and 330CHI antibody was derived from the mouse antibody IGHV5-15*01; while the FR sequence of the light chain variable region of the antibody was derived from the mouse antibody IGKV8-19*01. The CDR region of the mouse antibody and the FR region of the human antibody were recombined to form the humanized antibody, wherein the similarities between the used light and heavy chain templates of human-derived antibodies and the corresponding variable region sequences of mouse antibodies were 65% or more, see Table 12 for the source of the human-derived antibodies. The names and sequences of the final humanized antibodies are shown in Table 13.

**Table 12 Design information of the humanized sequences**

| Humanization design of the heavy chain | | | |
|---|---|---|---|
| Source of the human-derived sequence | Similarity | Whether or not the CDR length is the same as the mouse antibody | Recombined humanized sequence |
| IGHV3-48*01 | 77.55% | Yes | SEQ ID NO 82 |
| IGHV3-23*01 | 75.51 % | Yes | SEQ ID NO 88 |

| Humanization design of the light chain | | | |
|---|---|---|---|
| Source of the human-derived sequence | Similarity | Whether or not the CDR length is the same as the mouse antibody | Recombined humanized sequence |
| IGKV4-1*01 | 81.91% | Yes | SEQ ID NO 84 |
| IGKV3-7*01 | 65.96% | No | SEQ ID NO 86 |
| IGKV2-30*01 | 64.89% | No | SEQ ID NO 90 |

**Table 13 Sequence listing of the humanized antibodies**

| **No.** | **Antibody name** | **Amino acid sequence Heavy chain variable region** | **Nucleotide sequence Light chain variable region** | **Heavy chain variable region** | **Light chain variable region** |
|---|---|---|---|---|---|
| 1 | Hu330-1 | SEQ ID NO 82 | SEQ ID NO 84 | SEQ ID NO 83 | SEQ ID NO 85 |
| 2 | Hu330-2 | SEQ ID NO 82 | SEQ ID NO 86 | SEQ ID NO 83 | SEQ ID NO 87 |
| 3 | Hu330-3 | SEQ ID NO 88 | SEQ ID NO 84 | SEQ ID NO 89 | SEQ ID NO 85 |
| 4 | Hu330-4 | SEQ ID NO 88 | SEQ ID NO 86 | SEQ ID NO 89 | SEQ ID NO 87 |
| 5 | Hu330-5 | SEQ ID NO 82 | SEQ ID NO 90 | SEQ ID NO 83 | SEQ ID NO 91 |
| 6 | Hu330-6 | SEQ ID NO 88 | SEQ ID NO 90 | SEQ ID NO 89 | SEQ ID NO 91 |

Plasmid vectors comprising genes of the humanized antibody light or heavy chain were co-transfected into HEK293E cells at a ratio of 1:1, cultured with shaking at 110 rpm, 37°C, 8% CO₂ for 7 days. The cells were centrifuged at 4500 rpm for 20 minutes, the supernatant protein was harvested. Purification by ProteinA affinity chromatography was performed as follows: the AKTA chromatography system and columns were cleaned with 0.1 M NaOH solution for 30 minutes, rinsed clean with ultrapure water; equilibrated with 10 column volumes of 20 mM sodium phosphate buffer of pH 7.4; the flow rate was set to 2 ml/min, samples were loaded; the columns were cleaned with 10 column volumes of the equilibration buffer; eluted with 3-5 column volumes of 50 mM acetate buffer of pH 3.4, the eluted protein was collected; concentrated via ultrafiltration to displace the antibody in 20 mM phosphate buffer of pH 6.5 for storage. The purity of the purified antibody was determined by size exclusion high performance liquid chromatography (SEC-HPLC), and the monomer protein purity of humanized antibodies Hu330-1, Hu330-2 and Hu330-4 all reach more than 95%, among them, for Hu330-1 as an example, the molecular weight of the antibody heavy chain is 48.72 KD and the molecular weight of the light chain is 24.21 KD.

### Example 15 FACS detection of the specificity and affinity of the binding of the humanized antibodies to human CLDN18.2 at the cellular level

CHO-K1-CLDN18.2 cells and CHO-K1-CLDN18.1 cells (prepared in Example 5) were digested with gastric cancer cell digestion solution at 37°C for 15 minutes, respectively; terminated with complete culture medium, centrifuged at 1000 rpm for 5 minutes, the supernatant was discarded; the cells were resuspended with 2% fetal bovine serum-PBS diluent, the cells were adjusted to 1×10⁶ cells/ml, 100 µl of cell suspension was added to each well of the 96-well U-shaped-bottom plate; 2% fetal bovine serum-PBS was used to dilute the humanized antibody and the control antibody to final concentrations of 10 µg/ml, 2 µg/ml, 0.4 µg/ml, 0.08 µg/ml and 0.016 µg/ml, respectively, mixed well with the cells in equal volume, incubated at 4°C for 1 hour; centrifuged at 1000 rpm for 5 minutes, the supernatant was discarded; 200 µl Cell Stain Buffer (Biolegend) was added to each well to wash twice, centrifuged at 1000 rpm for 5 minutes at each time and the supernatant was discarded; 100 µl of PE-anti human Fc antibody (Biolegend) was added, incubated at 4°C for 1 hour; washed with Cell Stain Buffer (Biolegend) twice, centrifuged at 1000 rpm for 5 minutes at each time and the supernatant was discarded. 300 µl Cell Stain Buffer was added to resuspend the cells, and detected by BD FACS Jazz flow cytometry analyzer.

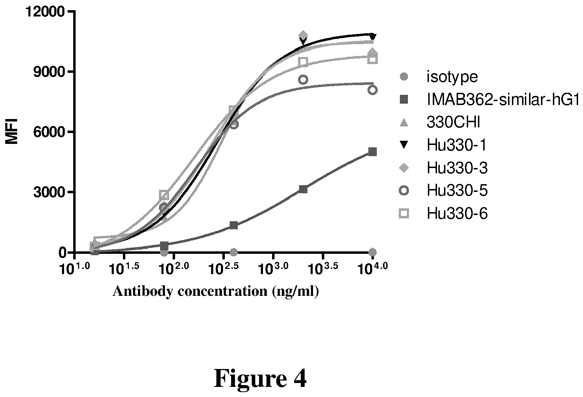

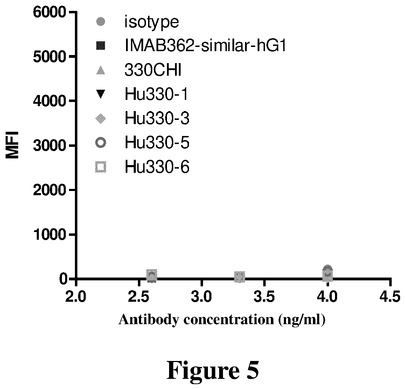

The results are shown in Figure 4 and Figure 5, humanized antibodies Hu330-1, Hu330-3, Hu330-5, and Hu330-6 all showed specific binding to CLDN18.2, while the binding to CLDN18.1 cells was negative. Among them, the binding curve of the humanized antibody Hu330-1 almost completely overlapped with that of the chimeric antibody 330CHI, indicating that after the antibody humanization, its affinity was not significantly different from that of the chimeric antibody, basically maintained at the same level. The values of the relative equilibrium dissociation constant Kd of antibodies calculated according to the Graphpad fitting curve are shown in Table 14. The Kd value of the humanized antibody Hu330-1 is 1.7 nM, which is significantly lower than that of the positive control antibody (28.7 nM), indicating the binding activity of the humanized antibody to cells expressing CLDN18.2 is about 15 times higher than that of the positive control antibody IMAB362-similar-hG1.

**Table 14 Fitting results of the relative equilibrium dissociation constant Kd of the humanized antibody determined by FACS**

| No. | Antibody name | Kd (nM) | Hill slope | Bmax (MFI) |
|---|---|---|---|---|
| 1 | IMAB362-similar-hG1 | 28.7 | 4.816 | 8224 |
| 2 | 330CHI | 1.6 | 7.566 | 10760 |
| 3 | Hu330-1 | 1.7 | 6.781 | 11321 |

### Example 16 Fortebio detection of the specificity and affinity of the binding of the humanized antibody to human CLDN18.2 at the protein level

Another method is to verify the affinity of the humanized antibody above at the protein level. The antibody was immobilized by the AHC sensor, and combined with different concentrations of antigens, and affinity determination was performed according to the manual of Octet RED96. The results are shown in Table 15. The equilibrium dissociation constant (KD) of the binding of the humanized antibody Hu330-1 to CLDN18.2 protein at 37°C is 5.26 nM, indicating that the antibody affinity significantly is increased by about 18 times compared with the positive control.

**Table 15 Relative equilibrium dissociation constant KD value of the humanized antibody determined by Fortebio**

| No. | Antibody name | KD (M) | Kon (M⁻¹s⁻¹) | Kdis (s⁻¹) |
|---|---|---|---|---|
| 1 | IMAB362-similar-hG1 | 9.44×10⁻⁹ | 2.35×10⁵ | 2.22x10⁻³ |
| 2 | 330CHI | 2.49×10-¹⁰ | 1.30×10⁵ | 3.23×10⁻⁵ |
| 3 | Hu330-1 | 5.26×10⁻⁵ | 1.75×10⁵ | 9.22×10⁻⁵ |

### Example 17 Determination of the in-vitro ADCC activities of the humanized antibodies by luciferase labeling

CHO-K1-CLDN18.2 cells (prepared in Example 5) were used as target cells, the cell density was adjusted to 1×10⁵ cells/ml, inoculated 100 µl/well into 96-well plates, incubated overnight in a 37°C, 5% CO₂ incubator; the humanized antibody was diluted in a 5-fold gradient, making the final antibody concentrations 10 µg/ml, 2 µg/ml, 0.4 µg/ml, 0.08 µg/ml, 0.016 µg/ml and 0.0032 µg/ml, respectively; equal amounts of IMAB362-similar-hG1 and 330CHI chimeric antibodies were added to the control wells, respectively, incubated in a 37°C, 5% CO₂ incubator for 1 hour; the effector cells Jurkat-CD16A/NF were added at an effector-to-target ratio of 20:1, 2×10⁵ effector cells per well, centrifuged at 200 g for 2 minutes to make the effector cells fully contacted with the target cells; incubated in a 37°C, 5% CO₂ incubator for 5-6 hours; fluorescent signals of the well plates were detected with Bio-Turbo firefly luciferase assay kit, the test results are shown in Table 16.

**Table 16 Determination of the ADCC activities of the humanized antibodies by luciferase labeling**

| Group | EC50 (nM) | Top |
|---|---|---|
| IMAB362-similar-hG1 | 0.69 | 63575 |
| 330CHI | 0.18 | 64473 |
| Hu330-1 | 0.12 | 68271 |
| Hu330-2 | 0.11 | 65144 |

Compared with the IMAB362-similar-hG1 positive antibody, the EC50s of the humanized antibodies Hu330-1 and Hu330-2 were reduced by more than 6 times, and the ADCC activities were basically the same as that of the chimeric antibody. It can be seen that while the humanized antibodies have high affinity, they also show higher ADCC biological activities than that of the positive antibody.

### Example 18 RTCA determination of the in-vitro ADCC cytotoxic activities of the humanized antibodies against tumor cells

Human peripheral blood was collected, from which PBMC cells were isolated, an equal volume of PBS containing 2% fetal bovine serum was added to dilute the blood sample; 15 ml of Lymphoprep density gradient medium for centrifugation (StemCell) was added to a 50 ml centrifuge tube, then the diluted blood sample was added, the centrifuge brake was turned off, centrifuged at 1200 g at room temperature for 10 minutes. After centrifugation, a buffy coat can be seen, which contained enriched PBMC cells, the buffy coat was aspirated and transferred to a new centrifuge tube, the red blood cells were lysed according to the manual of the red blood cell lysis solution (Sigma) at room temperature, washed twice using PBS containing 2 mM EDTA and 2% fetal bovine serum, centrifuged at 400 g at room temperature for 10 min, the supernatant was discarded; the cells were resuspended in 1640 complete culture medium containing 5% fetal bovine serum, the PBMC concentration was adjusted to 3×10⁶ cells/ml; Next, human gastric cancer cells NUGC4 were used as the target cells, treated with the digestion solution of human gastric cancer tissue-derived cells, the cells were collected by centrifugation at 1000 rpm for 5 minutes, the supernatant was discarded; the cells were adjusted to 1.5×10⁵ cells/ml, 100 µl of cell suspension was added to each well in E-Plate 96-well plates, incubated at 37°C, 5% CO₂ for more than 24 hours, the antibody diluted with 1640 culture medium was added to make the final concentration of antibody 2 µg/ml, incubated at 37°C, 5% CO₂ for 1 hour; The effector cells PBMC were added according to an effector to target ratio of 20:1, 3×10⁵ effector cells were added to each well, centrifuged at 200 g for 2 minutes to make the effector cells fully contacted with the target cells; incubated in a 37°C, 5% CO₂ incubator for 21 hours, RTCA analyzer was used to record the impedance value (Cell Index, CI) of the tumor cells in the 96-well plate in real time, the tumor cell cytotoxic percentage of the humanized antibody was calculated through the formula: cell death%=[(control group CI-experimental group CI)/control group CI]×100%.

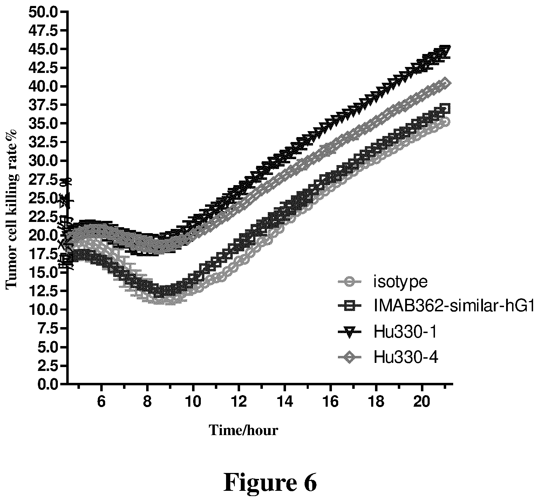

The results are shown in Figure 6. The tumor cell killing rate of each group increases across time during the 8 to 21 hours of incubation for RTCA real-time detection of the effector cells and target cells. At the 21st hour of in-vitro killing, the humanized antibody Hu330-1 has a maximum cytotoxic rate of 45% against NUGC4 cells, which is 10% higher than the ADCC cytotoxic activity of IMAB362-similar-hG1 positive antibody against tumor cells (P<0.05).

In summary, the humanized antibody Hu330-1 can specifically target and bind to CLDN18.2, and its binding activity and affinity are 15-18 times higher than the positive antibody IMAB362-similar-hG1 at both the molecular level and the cellular level, while the antibody-mediated in-vitro ADCC cytotoxic effect against the target cells shows an antibody concentration-dependency, at lower concentrations, Hu330-1 can already exert a certain cytotoxic effect against tumor cells.

### Example 19 Optimization of the constant region of the humanized antibody

The variable region sequence of the Hu330-1 humanized antibody was combined with the IgG1 constant region sequence comprising 5 amino acid site mutations, to construct and express the Fc mutant of the humanized antibody Hu330-1-5M (see Table 14 for the sequence); The positive antibody 5M comprising that Fc mutation (see Table 17 for the sequence) was constructed as a control for the Fc fragment.

**Table 17 Sequence list of the Fc mutant of the humanized antibody**

| **No.** | **Antibody name** | **Amino acid sequence** | | **Nucleotide sequence** | |
|---|---|---|---|---|---|
| | | **Heavy chain** | **Light chain** | **Heavy chain** | **Light chain** |
| 1 | Hu330-1 | SEQ ID NO 92 | SEQ ID NO 94 | SEQ ID NO 93 | SEQ ID NO 95 |
| 2 | Hu330-1-5M | SEQ ID NO 96 | SEQ ID NO 98 | SEQ ID NO 97 | SEQ ID NO 99 |
| 3 | 5M | SEQ ID NO 100 | SEQ ID NO 11 | | |

The effect of the Fc-mutated humanized antibody on the binding to the target CLDN18.2 was identified by FACS. NUGC4 cells were digested using the gastric cancer cell digestion solution at 37°C for 15 minutes; terminated by the complete culture medium, centrifuged at 1000 rpm for 5 minutes, the supernatant was discarded; the cells were resuspended with 2% fetal bovine serum-PBS diluent, the cells were adjusted to 1 × 10⁶ cells/ml, 100 µl of cell suspension was added to each well of the 96-well U-shpaed-bottom plate; the antibody was diluted with 2% fetal bovine serum-PBS to final concentrations of 5 µg/ml, 1 µg/ml, 0.2 µg/ml, 0.04 µg/ml and 0.008 µg/ml, mixed with the cells in equal volume, incubated at 4°C for 1 hour; centrifuged at 1000 rpm for 5 minutes, the supernatant was discarded; 200 µl of Cell Stain Buffer (Biolegend) was added to each well to wash twice, centrifuged at 1000 rpm for 5 minutes at each time and the supernatant was discarded; 100 µl of PE-anti human Fc antibody (Biolegend) was added, incubated at 4°C for 1 hour; washed twice using Cell Stain Buffer (Biolegend), centrifuged at 1000 rpm for 5 minutes at each time and the supernatant was discarded. 300 µl of Cell Stain Buffer was added to resuspend the cells, detected with BD FACS Jazz flow analyzer. The results are shown in Table 18.

**Table 18 FACS detection of the binding activity of the Fc-optimized humanized antibody**

| Group | EC50 (nM) | Top (%) |
|---|---|---|
| IMAB362-similar-hG1 | 9.78 | 58.8 |
| Hu330-1 | 0.80 | 92.1 |
| Hu330-1-5M | 0.76 | 92.4 |

The positive rate of the binding of the Fc-mutated humanized antibody to NUGC4 target cells gradually reaches a platform as the antibody concentration increases, and its binding curve almost completely overlaps with that of the humanized antibody, and the binding activity of the Hu330-1-5M antibody increases by 15 times compared with that of the positive control antibody IMAB362-similar-hG1, which indicates that the amino acid mutation in the Fc fragment of the constant region does not change the specificity and binding activity of the antibody against the CLDN18.2 target.

### Example 20 Detection of the in-vitro ADCC cytotoxic activity of the optimized humanized antibody

### 1. Determination of the in-vitro ADCC activity of the Fc-mutated humanized antibody by luciferase labeling

CHO-K1-CLDN18.2 cells (prepared in Example 5) were used as target cells, and the cell density was adjusted to 1×10⁵ cells/ml, inoculated 100 µl/well into 96-well plates, incubated overnight in a 37°C, 5% CO₂ incubator; the antibody was diluted in a 5-fold gradient, making the final antibody concentrations 10 µg/ml, 2 µg/ml, 0.4 µg/ml, 0.08 µg/ml, 0.016 µg/ml and 0.0032 µg/ml, respectively; incubated at 37°C, 5% CO₂ for 1 hour; the effector cells Jurkat-CD16A/NF were added at an effector-to-target ratio of 20:1, 2 × 10⁵ effector cells per well were added into each well plate, centrifuged at 200 g for 2 minutes to make the effector cells fully contacted with the target cells; incubated in a 37°C, 5% CO₂ incubator for 5-6 hours; fluorescent signals of the well plates were detected with Bio-Turbo firefly luciferase assay kit, the results are shown in Table 19.

**Table 19 Determination of the ADCC activity of the Fc-optimized humanized antibody by luciferase labeling**

| Group | EC50 (nM) | Top |
|---|---|---|
| IMAB362-similar-hG1 | 1.49 | 89555 |
| Hu330-1 | 0.45 | 89817 |
| Hu330-1-5M | 0.17 | 100434 |
| 5M | 0.44 | 98368 |

Compared with IMAB362-similar-hG1, the Fc-mutated positive antibody (5M) and the humanized Hu330-1 antibody both have an ADCC activity increased by 3 times, while the EC50 of Fc-mutated Hu330-1-5M antibody is 0.17 nM, which is higher than the ADCC activity of the positive control antibody IMAB362-similar-hG1 by more than 8 times, indicating obviously optimized in-vitro ADCC enhancement effect.

### 2. Determination of the in-vitro ADCC cytotoxic activity of the Fc-mutated humanized antibody against tumor cells by LDH method

Human peripheral blood was collected, from which PBMC cells were isolated as the effector cells, an equal volume of PBS containing 2% fetal bovine serum was added to dilute the blood sample; 15 ml of Lymphoprep density gradient medium for centrifugation (StemCell) was added to a 50 ml centrifuge tube, then the diluted blood sample was added, the centrifuge brake was turned off, centrifuged at 1200 g at room temperature for 10 minutes. After centrifugation, a buffy coat can be seen, which contained enriched PBMC cells, the buffy coat was aspirated and transferred to a new centrifuge tube. The red blood cells were lysed according to the manual of the red blood cell lysis solution (Sigma) at room temperature, washed twice using PBS containing 2 mM EDTA and 2% fetal bovine serum, centrifuged at 400 g at room temperature for 10 min, the supernatant was discarded; the cells were resuspended using 1640 containing 2% fetal bovine serum prepared using heat-inactivated serum (hereinafter referred to as heat-inactivated culture medium), the PBMC concentration was adjusted to 3×10⁶ cells/ml; human gastric cancer cells NUGC4 were used as the target cells, and treated with the digestion solution of human gastric cancer tissue-derived cells, the cells were collected by centrifugation at 1000 rpm for 5 minutes, the supernatant was discarded; the cells were adjusted to 1.5×10⁵ cells/ml, 100 µl of cell suspension was added to each well of the 96-well plate, incubated at 37°C, 5% CO₂ for 24 hours, the culture medium was aspirated, the antibody diluted with heat-inactivated culture medium was added, making the final concentrations of the antibody 10 µg/ml, 2 µg/ml, 0.4 µg/ml, 0.08 µg/ml, 0.016 µg/ml and 0.0032 µg/ml, incubated at 37°C, 5% CO₂ for 1 hour; the effector cells PBMC were added according to an effector-to-target ratio of 20:1, 3×10⁵ effector cells were added to each well, centrifuged at 200 g for 2 minutes to make the effector cells fully contacted with the target cells; incubated for 22 hours in a 37°C, 5% CO₂ incubator for killing. At the end of the incubation, WST substrate reagent was added to the well plate according to the steps in the manual of the LDH kit (Dojindo Laboratories (Shanghai) Co., Ltd.), color developed under protection from light for 20-30 minutes, the OD450 nm values were determined, and the original data was put into the formula: cell death%=(absorption value in experimental group - spontaneous value of the target cells - value of the effector cells)/(maximum value of the target cells - spontaneous value of the target cells) × 100%, absorption value in experimental group and all control groups = absorption value in that group - background value. The results are shown in Table 20.

**Table 20 Determination of the ADCC activity of the Fc-optimized humanized antibody by LDH method**

| Group | EC50 (nM) | Max (%) |
|---|---|---|
| Isotype | N.A | 52 |
| IMAB362-similar-hG1 | 2.47 | 80.13 |
| Hu330-1-5M | 0.31 | 129.9 |

The Fc-mutated humanized antibody Hu330-1-5M can kill tumor cells NUGC4 *in vitro,* and its ADCC cytotoxic effect is dose-dependent on the antibody concentration, the calculated EC50 (0.3 nM) is higher than that of IMAB362-similar-hG1 positive antibody by 8 times; at the same time, Hu330-1-5M has a maximum cytotoxic rate of 130% against tumor cells, which exhibits significantly enhanced in-vitro ADCC cytotoxic effect compared to the positive antibody which exhibits 80%.

In summary, the Fc-mutated Hu330-1-5M humanized antibody, on one hand, has an affinity increased by 15 times compared with the positive antibody IMAB362-similar-hG1 in the targeted binding ability to CLDN18.2; and on the other hand, the ability of in-vitro ADCC cytotoxicity against tumor cells is further enhanced after Fc optimization, showing a EC50 activity increased by 8 times than that of the positive antibody, while the maximum cytotoxic effect is increased by more than 60%.

In summary, the CLDN18.2 antibody provided by the present invention has significant binding activity and affinity to CLDN18.2, at the same time it has efficient in-vitro ADCC cytotoxic activity against tumor cells, indicating that the antibody has efficient anti-tumor effects, which can be applied in the preparation of an anti-tumor drug and has broad market prospects.

Although the present invention has been described in detail above, those skilled in the art should understand that various modifications and changes can be made to the present invention without departing from the spirit and scope of the present invention. The scope of the claims of the present invention is not limited to the detailed description made above, but should belong to the claims.

## Claims

1. An anti-Claudin18.2 antibody or an antigen binding fragment thereof, which comprises a heavy chain variable region and a light chain variable region, wherein:
(1) the heavy chain variable region comprises HCDR1, HCDR2 and HCDR3, wherein:
(a1) the amino acid sequence of the HCDR1 is selected from SEQ ID NO 101, SEQ ID NO 102, SEQ ID NO 103 and amino acid sequences having at least 85% sequence identity with them;
(a2) the amino acid sequence of the HCDR2 is selected from SEQ ID NO 104, SEQ ID NO 105 and amino acid sequences having at least 85% sequence identity with them;
(a3) the amino acid sequence of the HCDR3 is selected from SEQ ID NO 106, SEQ ID NO 107 and amino acid sequences having at least 85% sequence identity with them; and
(2) the light chain variable region comprises LCDR1, LCDR2 and LCDR3, wherein:
(a4) the amino acid sequence of the LCDR1 is SEQ ID NO 108;
(a5) the amino acid sequence of LCDR2 is selected from SEQ ID NO 109, SEQ ID NO 110, SEQ ID NO 111 and amino acid sequences having at least 85% sequence identity with them;
(a6) the amino acid sequence of the LCDR3 is selected from SEQ ID NO 112, SEQ ID NO 113, SEQ ID NO 114, SEQ ID NO 115, SEQ ID NO 116 and amino acid sequences having at least 85% sequence identity with them.

2. The anti-Claudin18.2 antibody or the antigen binding fragment thereof of claim 1, wherein:
(1) the heavy chain variable region comprises HCDR1 of the amino acid sequence shown in SEQ ID NO 101, HCDR2 of the amino acid sequence shown in SEQ ID NO 105 and HCDR3 of the amino acid sequence shown in SEQ ID NO 106; and
(2) the light chain variable region comprises LCDR1 of the amino acid sequence shown in SEQ ID NO 108, LCDR2 of the amino acid sequence shown in SEQ ID NO 111 and LCDR3 of the amino acid sequence shown in SEQ ID NO 115.

3. The anti-Claudin18.2 antibody or the antigen binding fragment thereof of claim 1 or 2, wherein the antibody is a murine-derived antibody, a chimeric antibody or a humanized antibody.

4. The anti-Claudin18.2 antibody or the antigen binding fragment thereof of any one of claims 1-3, wherein:
(1) the amino acid sequence of the heavy chain variable region is selected from:
(b1) an amino acid sequence shown in SEQ ID NO 50, SEQ ID NO 72, SEQ ID NO 74, SEQ ID NO 77, SEQ ID NO 79, SEQ ID NO 82, SEQ ID NO 88;
(b2) an amino acid sequence obtained by substituting, deleting or adding one or more amino acids of the amino acid sequence shown in (b1), and with the same or similar function as the amino acid sequence shown in (b1); and
(b3) an amino acid sequence having at least 80% sequence identity with the amino acid sequence shown in (b1); and
(2) the amino acid sequence of the light chain variable region is selected from:
(b4) an amino acid sequence shown in SEQ ID NO 51, SEQ ID NO 70, SEQ ID NO 71, SEQ ID NO 73, SEQ ID NO 75, SEQ ID NO 76, SEQ ID NO 78, SEQ ID NO 80, SEQ ID NO 81, SEQ ID NO 84, SEQ ID NO 86, SEQ ID NO 90;
(b5) an amino acid sequence obtained by substituting, deleting or adding one or more amino acids of the amino acid sequence shown in (b4), and with the same or similar function as the amino acid sequence shown in (b4); and
(b6) an amino acid sequence having at least 80% sequence identity with the amino acid sequence shown in (b4).

5. The anti-Claudin18.2 antibody or the antigen binding fragment thereof of claim 4, wherein the amino acid sequence of the heavy chain variable region is SEQ ID NO 79, the amino acid sequence obtained by substituting, deleting or adding one or more amino acids of SEQ ID NO 79 and with the same function as SEQ ID NO 79 or the amino acid sequence having at least 85% sequence identity with SEQ ID NO 79, and the amino acid sequence of the light chain variable region is SEQ ID NO 81, the amino acid sequence obtained by substituting, deleting or adding one or more amino acids of SEQ ID NO 81 and with the same function as SEQ ID NO 81 or the amino acid sequence having at least 85% sequence identity with SEQ ID NO 81.

6. The anti-Claudin18.2 antibody or the antigen binding fragment thereof of claim 4, wherein:
(1) the amino acid sequence of the heavy chain variable region is selected from:
(c1) an amino acid sequence shown in SEQ ID NO 82, SEQ ID NO 88;
(c2) an amino acid sequence obtained by substituting, deleting or adding one or more amino acids of the amino acid sequence shown in (c1), and with the same or similar function as the amino acid sequence shown in (c1); and
(c3) an amino acid sequence having at least 80% sequence identity with the amino acid sequence shown in (c1); and
(2) the amino acid sequence of the light chain variable region is selected from:
(c4) an amino acid sequence shown in SEQ ID NO 84, SEQ ID NO 86, SEQ ID NO 90;
(c5) an amino acid sequence obtained by substituting, deleting or adding one or more amino acids of the amino acid sequence shown in (c4), and with the same or similar function as the amino acid sequence shown in (c4); and
(c6) an amino acid sequence having at least 80% sequence identity with the amino acid sequence shown in (c4).

7. The anti-Claudin18.2 antibody or the antigen binding fragment thereof of claim 6, wherein:
the amino acid sequence of the heavy chain variable region is SEQ ID NO 82, the amino acid sequence obtained by substituting, deleting or adding one or more amino acids of SEQ ID NO 82 and with the same function as SEQ ID NO 82 or the amino acid sequence having at least 85% sequence identity with SEQ ID NO 82, and the amino acid sequence of the light chain variable region is SEQ ID NO 84, the amino acid sequence obtained by substituting, deleting or adding one or more amino acids of SEQ ID NO 84 and with the same function as SEQ ID NO 84 or the amino acid sequence having at least 85% sequence identity with SEQ ID NO 84;
the amino acid sequence of the heavy chain variable region is SEQ ID NO 82, the amino acid sequence obtained by substituting, deleting or adding one or more amino acids of SEQ ID NO 82 and with the same function as SEQ ID NO 82 or the amino acid sequence having at least 85% sequence identity with SEQ ID NO 82, and the amino acid sequence of the light chain variable region is SEQ ID NO 86, the amino acid sequence obtained by substituting, deleting or adding one or more amino acids of SEQ ID NO 86 and with the same function as SEQ ID NO 86 or the amino acid sequence having at least 85% sequence identity with SEQ ID NO 86;
the amino acid sequence of the heavy chain variable region is SEQ ID NO 88, the amino acid sequence obtained by substituting, deleting or adding one or more amino acids of SEQ ID NO 88 and with the same function as SEQ ID NO 88 or the amino acid sequence having at least 85% sequence identity with SEQ ID NO 88, and the amino acid sequence of the light chain variable region is SEQ ID NO 84, the amino acid sequence obtained by substituting, deleting or adding one or more amino acids of SEQ ID NO 84 and with the same function as SEQ ID NO 84 or the amino acid sequence having at least 85% sequence identity with SEQ ID NO 84;
the amino acid sequence of the heavy chain variable region is SEQ ID NO 88, the amino acid sequence obtained by substituting, deleting or adding one or more amino acids of SEQ ID NO 88 and with the same function as SEQ ID NO 88 or the amino acid sequence having at least 85% sequence identity with SEQ ID NO 88, and the amino acid sequence of the light chain variable region is SEQ ID NO 86, the amino acid sequence obtained by substituting, deleting or adding one or more amino acids of SEQ ID NO 86 and with the same function as SEQ ID NO 86 or the amino acid sequence having at least 85% sequence identity with SEQ ID NO 86;
the amino acid sequence of the heavy chain variable region is SEQ ID NO 82, the amino acid sequence obtained by substituting, deleting or adding one or more amino acids of SEQ ID NO 82 and with the same function as SEQ ID NO 82 or the amino acid sequence having at least 85% sequence identity with SEQ ID NO 82, and the amino acid sequence of the light chain variable region is SEQ ID NO 90, the amino acid sequence obtained by substituting, deleting or adding one or more amino acids of SEQ ID NO 90 and with the same function as SEQ ID NO 90 or the amino acid sequence having at least 85% sequence identity with SEQ ID NO 90; or
the amino acid sequence of the heavy chain variable region is SEQ ID NO 88, the amino acid sequence obtained by substituting, deleting or adding one or more amino acids of SEQ ID NO 88 and with the same function as SEQ ID NO 88 or the amino acid sequence having at least 85% sequence identity with SEQ ID NO 88, and the amino acid sequence of the light chain variable region is SEQ ID NO 90, the amino acid sequence obtained by substituting, deleting or adding one or more amino acids of SEQ ID NO 90 and with the same function as SEQ ID NO 90 or the amino acid sequence having at least 85% sequence identity with SEQ ID NO 90.

8. The anti-Claudin18.2 antibody or the antigen binding fragment thereof of claim 7, wherein:
the amino acid sequence of the heavy chain is SEQ ID NO 92, the amino acid sequence obtained by substituting, deleting or adding one or more amino acids of SEQ ID NO 92 and with the same function as SEQ ID NO 92 or the amino acid sequence having at least 85% sequence identity with SEQ ID NO 92, and the amino acid sequence of the light chain is SEQ ID NO 94, the amino acid sequence obtained by substituting, deleting or adding one or more amino acids of SEQ ID NO 94 and with the same function as SEQ ID NO 94 or the amino acid sequence having at least 85% sequence identity with SEQ ID NO 94; or
the amino acid sequence of the heavy chain is SEQ ID NO 96, the amino acid sequence obtained by substituting, deleting or adding one or more amino acids of SEQ ID NO 96 and with the same function as SEQ ID NO 96 or the amino acid sequence having at least 85% sequence identity with SEQ ID NO 96, and the amino acid sequence of the light chain is SEQ ID NO 98, the amino acid sequence obtained by substituting, deleting or adding one or more amino acids of SEQ ID NO 98 and with the same function as SEQ ID NO 98 or the amino acid sequence having at least 85% sequence identity with SEQ ID NO 98.

9. An isolated nucleotide, which encodes the anti-Claudin18.2 antibody or the antigen binding fragment thereof of any one of claims 1-8.

10. The nucleotide of claim 9, wherein:
(1) the nucleotide sequence encoding the amino acid sequence of the heavy chain is shown in SEQ ID NO 93 or SEQ ID NO 97; and
(2) the nucleotide sequence encoding the amino acid sequence of the light chain is shown in SEQ ID NO 95 or SEQ ID NO 99.

11. An expression vector, which comprises the nucleotide of claim 9 or 10.

12. A host cell, which is transfected with the expression vector of claim 11, the host cell is selected from a prokaryotic cell and a eukaryotic cell, preferably a mammalian cell.

13. A method for preparing the anti-Claudin18.2 antibody or the antigen binding fragment thereof of any one of claims 1-8, comprising steps of expressing the antibody in the host cell of claim 12, and isolating the antibody from the host cell.

14. A pharmaceutical composition, which comprises the anti-Claudin18.2 antibody or the antigen binding fragment thereof of any one of claims 1-8 and a pharmaceutically acceptable carrier.

15. Use of the anti-Claudin18.2 antibody or the antigen binding fragment thereof of any one of claims 1-8 in the preparation of a drug targeting Claudin18.2.

16. The use of claim 15, the drug targeting Claudin18.2 is used to treat gastric cancer, esophageal cancer, pancreatic cancer, lung cancer, ovarian cancer, colon cancer, liver cancer, head and neck cancer, and gallbladder cancer.


SEQUENCE LISTING

<110>  NANJING SANHOME PHARMACEUTICAL CO., LTD.

<120>  ANTI-CLAUDIN18.2 ANTIBODY AND USE THEREOF

<160>  116

<170>  SIPOSequenceListing 1.0

<210>  1
<211>  309
<212>  DNA
<213>  Artificial sequence

<400>  1
gctagcatgg acgccatgaa gaggggactg tgctgcgtgc tgctgctgtg cggagccgtg      60
ttcgtgtctc ccagcatgga ccagtggagc acccaggatc tgtacaacaa tcctgtgaca     120
gccgtgttta actatcaggg cctgtggcgg tcctgcgtga gagagagctc cggcttcacc     180
gagtgtaggg gctactttac actgctggga ctgccagcaa tgctgcaggc cgtgcgcgag     240
cagaagctga tctccgagga ggacctgaat tctgccgtgg atcatcacca ccaccaccac     300
tgactcgag                                                             309

<210>  2
<211>  98
<212>  PRT
<213>  Artificial sequence

<400>  2
Met Asp Ala Met Lys Arg Gly Leu Cys Cys Val Leu Leu Leu Cys Gly
1               5                   10                  15
Ala Val Phe Val Ser Pro Ser Met Asp Gln Trp Ser Thr Gln Asp Leu
            20                  25                  30
Tyr Asn Asn Pro Val Thr Ala Val Phe Asn Tyr Gln Gly Leu Trp Arg
        35                  40                  45
Ser Cys Val Arg Glu Ser Ser Gly Phe Thr Glu Cys Arg Gly Tyr Phe
    50                  55                  60
Thr Leu Leu Gly Leu Pro Ala Met Leu Gln Ala Val Arg Glu Gln Lys
65                  70                  75                  80
Leu Ile Ser Glu Glu Asp Leu Asn Ser Ala Val Asp His His His His
                85                  90                  95
His His


<210>  3
<211>  444
<212>  DNA
<213>  Artificial sequence

<400>  3
gctagcatgg acgccatgaa gaggggactg tgctgcgtgc tgctgctgtg cggagccgtg      60
ttcgtgtctc caagcatggc agtgaccgca tgtcagggac tgggctttgt ggtgagcctg     120
atcggaatcg caggaatcat cgcagcaacc tgcatggacc agtggtccac acaggatctg     180
tacaacaatc ccgtgacagc cgtgttcaac tatcagggcc tgtggcggtc ttgcgtgaga     240
gagagctccg gcttcaccga gtgtaggggc tactttacac tgctgggact gcctgcaatg     300
ctgcaggccg tgcgcgccct gatgatcgtg ggaatcgtgc tgggagcaat cggactgctg     360
gtgtccatct ttgagcagaa gctgatctcc gaggaggacc tgaattctgc cgtggatcat     420
caccaccacc accactgact cgag                                            444

<210>  4
<211>  143
<212>  PRT
<213>  Artificial sequence

<400>  4
Met Asp Ala Met Lys Arg Gly Leu Cys Cys Val Leu Leu Leu Cys Gly
1               5                   10                  15
Ala Val Phe Val Ser Pro Ser Met Ala Val Thr Ala Cys Gln Gly Leu
            20                  25                  30
Gly Phe Val Val Ser Leu Ile Gly Ile Ala Gly Ile Ile Ala Ala Thr
        35                  40                  45
Cys Met Asp Gln Trp Ser Thr Gln Asp Leu Tyr Asn Asn Pro Val Thr
    50                  55                  60
Ala Val Phe Asn Tyr Gln Gly Leu Trp Arg Ser Cys Val Arg Glu Ser
65                  70                  75                  80
Ser Gly Phe Thr Glu Cys Arg Gly Tyr Phe Thr Leu Leu Gly Leu Pro
                85                  90                  95
Ala Met Leu Gln Ala Val Arg Ala Leu Met Ile Val Gly Ile Val Leu
            100                 105                 110
Gly Ala Ile Gly Leu Leu Val Ser Ile Phe Glu Gln Lys Leu Ile Ser
        115                 120                 125
Glu Glu Asp Leu Asn Ser Ala Val Asp His His His His His His
    130                 135                 140

<210>  5
<211>  43
<212>  DNA
<213>  Artificial sequence

<400>  5
agatctatcg actctcgagc gttcttcgtt cgttctcacg cgt                         43

<210>  6
<211>  798
<212>  DNA
<213>  Artificial sequence

<400>  6
ggatccatgg cagtgacagc atgccaggga ctgggattcg tggtgtctct gatcggcatc        60
gccggcatca tcgccgccac atgtatggac cagtggagca cccaggatct gtacaacaat       120
ccagtgaccg ccgtgtttaa ctatcagggc ctgtggcggt cctgcgtgag agagagctcc       180
ggcttcacag agtgtagggg ctactttacc ctgctgggac tgccagcaat gctgcaggcc       240
gtgcgcgccc tgatgatcgt gggaatcgtg ctgggagcaa tcggactgct ggtgtctatc       300
ttcgccctga agtgcatcag gatcggcagc atggaggact ccgccaaggc caacatgacc       360
ctgacatccg gcatcatgtt tatcgtgtct ggactgtgcg caatcgcagg cgtgagcgtg       420
ttcgccaaca tgctggtgac aaatttttgg atgtccacag ccaatatgta caccggaatg        480
ggaggaatgg tgcagaccgt gcagacacgg tataccttcg gcgccgccct gtttgtggga       540
tgggtggcag gaggactgac actgatcggc ggcgtgatga tgtgcatcgc atgtagagga       600
ctggcaccag aggagaccaa ttacaaggcc gtgagctatc acgcctctgg acacagcgtg       660
gcatacaagc ctggaggctt caaggcctct acaggctttg gcagcaacac caagaataag       720
aagatctatg acggcggcgc cagaaccgag gatgaggtgc agtcctaccc ctctaagcac       780
gattacgtgt gagaattc                                                     798

<210>  7
<211>  794
<212>  DNA
<213>  Artificial sequence

<400>  7
ctcgagatgg ccgtgactgc ctgtcagggc ttggggttcg tggtttcact gattgggatt        60
gcgggcatca ttgctgccac ctgcatggac cagtggagca cccaagactt gtacaacaac       120
cccgtaacag ctgttttcaa ctaccagggg ctgtggcgct cctgtgtccg agagagctct       180
ggcttcaccg agtgccgggg ctacttcacc ctgctggggc tgccagccat gctgcaggca       240
gtgcgagccc tgatgatcgt aggcatcgtc ctgggtgcca ttggcctcct ggtatccatc       300
tttgccctga aatgcatccg cattggcagc atggaggact ctgccaaagc caacatgaca       360
ctgacctccg ggatcatgtt cattgtctca ggtctttgtg caattgctgg agtgtctgtg       420

tttgccaaca tgctggtgac taacttctgg atgtccacag ctaacatgta caccggcatg 480
ggtgggatgg tgcagactgt tcagaccagg tacacatttg gtgcggctct gttcgtgggc 540
tgggtcgctg gaggcctcac actaattggg ggtgtgatga tgtgcatcgc ctgccggggc 600
ctggcaccag aagaaaccaa ctacaaagcc gtttcttatc atgcctcagg ccacagtgtt 660
gcctacaagc ctggaggctt caaggccagc actggctttg ggtccaacac caaaaacaag 720
aagatatacg atggaggtgc ccgcacagag gacgaggtac aatcttatcc ttccaagcac 780
gactatgtgg atcc 794

<210> 8
<211> 271
<212> PRT
<213> Artificial sequence

<400> 8
Met Ala Val Thr Ala Cys Gln Gly Leu Gly Phe Val Val Ser Leu Ile
1               5                   10                  15
Gly Ile Ala Gly Ile Ile Ala Ala Thr Cys Met Asp Gln Trp Ser Thr
            20                  25                  30
Gln Asp Leu Tyr Asn Asn Pro Val Thr Ala Val Phe Asn Tyr Gln Gly
        35                  40                  45
Leu Trp Arg Ser Cys Val Arg Glu Ser Ser Gly Phe Thr Glu Cys Arg
    50                  55                  60
Gly Tyr Phe Thr Leu Leu Gly Leu Pro Ala Met Leu Gln Ala Val Arg
65                  70                  75                  80
Ala Leu Met Ile Val Gly Ile Val Leu Gly Ala Ile Gly Leu Leu Val
                85                  90                  95
Ser Ile Phe Ala Leu Lys Cys Ile Arg Ile Gly Ser Met Glu Asp Ser
            100                 105                 110
Ala Lys Ala Asn Met Thr Leu Thr Ser Gly Ile Met Phe Ile Val Ser
        115                 120                 125
Gly Leu Cys Ala Ile Ala Gly Val Ser Val Phe Ala Asn Met Leu Val
    130                 135                 140
Thr Asn Phe Trp Met Ser Thr Ala Asn Met Tyr Thr Gly Met Gly Glu
145                 150                 155                 160
Gln Lys Leu Ile Ser Glu Glu Asp Leu Gly Met Val Gln Thr Val Gln
                165                 170                 175
Thr Arg Tyr Thr Phe Gly Ala Ala Leu Phe Val Gly Trp Val Ala Gly
            180                 185                 190
Gly Leu Thr Leu Ile Gly Gly Val Met Met Cys Ile Ala Cys Arg Gly
        195                 200                 205
Leu Ala Pro Glu Glu Thr Asn Tyr Lys Ala Val Ser Tyr His Ala Ser
    210                 215                 220
Gly His Ser Val Ala Tyr Lys Pro Gly Gly Phe Lys Ala Ser Thr Gly
225                 230                 235                 240
Phe Gly Ser Asn Thr Lys Asn Lys Lys Ile Tyr Asp Gly Gly Ala Arg
                245                 250                 255
Thr Glu Asp Glu Val Gln Ser Tyr Pro Ser Lys His Asp Tyr Val
            260                 265                 270

<210> 9
<211> 794
<212> DNA
<213> Artificial sequence

<400> 9
ctcgagatgt ccaccaccac atgccaagtg gtggcgttcc tcctgtccat cctggggctg 60
gccggctgca tcgcggccac cgggatggac atgtggagca cccaggacct gtacgacaac 120
cccgtcacct ccgtgttcca gtacgaaggg ctctggagga gctgcgtgag gcagagttca 180
ggcttcaccg aatgcaggcc ctatttcacc atcctgggac ttccagccat gctgcaggca 240
gtgcgagccc tgatgatcgt aggcatcgtc ctgggtgcca ttggcctcct ggtatccatc 300
tttgccctga aatgcatccg cattggcagc atggaggact ctgccaaagc caacatgaca 360
ctgacctccg ggatcatgtt cattgtctca ggtctttgtg caattgctgg agtgtctgtg 420
tttgccaaca tgctggtgac taacttctgg atgtccacag ctaacatgta caccggcatg 480

ggtgggatgg tgcagactgt tcagaccagg tacacatttg gtgcggctct gttcgtgggc 540
tgggtcgctg gaggcctcac actaattggg ggtgtgatga tgtgcatcgc ctgccggggc 600
ctggcaccag aagaaaccaa ctacaaagcc gtttcttatc atgcctcagg ccacagtgtt 660
gcctacaagc ctggaggctt caaggccagc actggctttg ggtccaacac caaaaacaag 720
aagatatacg atggaggtgc ccgcacagag gacgaggtac aatcttatcc ttccaagcac 780
gactatgtgg atcc 794

<210> 10
<211> 262
<212> PRT
<213> Artificial sequence

<400> 10
Met Ser Thr Thr Thr Cys Gln Val Val Ala Phe Leu Leu Ser Ile Leu
1               5                   10                  15
Gly Leu Ala Gly Cys Ile Ala Ala Thr Gly Met Asp Met Trp Ser Thr
            20                  25                  30
Gln Asp Leu Tyr Asp Asn Pro Val Thr Ser Val Phe Gln Tyr Glu Gly
        35                  40                  45
Leu Trp Arg Ser Cys Val Arg Gln Ser Ser Gly Phe Thr Glu Cys Arg
    50                  55                  60
Pro Tyr Phe Thr Ile Leu Gly Leu Pro Ala Met Leu Gln Ala Val Arg
65                  70                  75                  80
Ala Leu Met Ile Val Gly Ile Val Leu Gly Ala Ile Gly Leu Leu Val
                85                  90                  95
Ser Ile Phe Ala Leu Lys Cys Ile Arg Ile Gly Ser Met Glu Asp Ser
            100                 105                 110
Ala Lys Ala Asn Met Thr Leu Thr Ser Gly Ile Met Phe Ile Val Ser
        115                 120                 125
Gly Leu Cys Ala Ile Ala Gly Val Ser Val Phe Ala Asn Met Leu Val
    130                 135                 140
Thr Asn Phe Trp Met Ser Thr Ala Asn Met Tyr Thr Gly Met Gly Gly
145                 150                 155                 160
Met Val Gln Thr Val Gln Thr Arg Tyr Thr Phe Gly Ala Ala Leu Phe
                165                 170                 175
Val Gly Trp Val Ala Gly Gly Leu Thr Leu Ile Gly Gly Val Met Met
            180                 185                 190
Cys Ile Ala Cys Arg Gly Leu Ala Pro Glu Glu Thr Asn Tyr Lys Ala
        195                 200                 205
Val Ser Tyr His Ala Ser Gly His Ser Val Ala Tyr Lys Pro Gly Gly
    210                 215                 220
Phe Lys Ala Ser Thr Gly Phe Gly Ser Asn Thr Lys Asn Lys Lys Ile
225                 230                 235                 240
Tyr Asp Gly Gly Ala Arg Thr Glu Asp Glu Val Gln Ser Tyr Pro Ser
                245                 250                 255
Lys His Asp Tyr Val Asp
            260

<210> 11
<211> 240
<212> PRT
<213> Artificial sequence

<400> 11
Met Glu Ser Gln Thr Gln Val Leu Met Ser Leu Leu Phe Trp Val Ser
1               5                   10                  15
Gly Thr Cys Gly Asp Ile Val Met Thr Gln Ser Pro Ser Ser Leu Thr
            20                  25                  30
Val Thr Ala Gly Glu Lys Val Thr Met Ser Cys Lys Ser Ser Gln Ser
        35                  40                  45
Leu Leu Asn Ser Gly Asn Gln Lys Asn Tyr Leu Thr Trp Tyr Gln Gln
    50                  55                  60
Lys Pro Gly Gln Pro Pro Lys Leu Leu Ile Tyr Trp Ala Ser Thr Arg

65                  70                  75                  80
Glu Ser Gly Val Pro Asp Arg Phe Thr Gly Ser Gly Ser Gly Thr Asp
                85                  90                  95
Phe Thr Leu Thr Ile Ser Ser Val Gln Ala Glu Asp Leu Ala Val Tyr
            100                 105                 110
Tyr Cys Gln Asn Asp Tyr Ser Tyr Pro Phe Thr Phe Gly Ser Gly Thr
        115                 120                 125
Lys Leu Glu Ile Lys Arg Thr Val Ala Ala Pro Ser Val Phe Ile Phe
    130                 135                 140
Pro Pro Ser Asp Glu Gln Leu Lys Ser Gly Thr Ala Ser Val Val Cys
145                 150                 155                 160
Leu Leu Asn Asn Phe Tyr Pro Arg Glu Ala Lys Val Gln Trp Lys Val
                165                 170                 175
Asp Asn Ala Leu Gln Ser Gly Asn Ser Gln Glu Ser Val Thr Glu Gln
            180                 185                 190
Asp Ser Lys Asp Ser Thr Tyr Ser Leu Ser Ser Thr Leu Thr Leu Ser
        195                 200                 205
Lys Ala Asp Tyr Glu Lys His Lys Val Tyr Ala Cys Glu Val Thr His
    210                 215                 220
Gln Gly Leu Ser Ser Pro Val Thr Lys Ser Phe Asn Arg Gly Glu Cys
225                 230                 235                 240

<210>  12
<211>  467
<212>  PRT
<213>  Artificial sequence

<400>  12
Met Gly Trp Ser Cys Ile Ile Leu Phe Leu Val Ala Thr Ala Thr Gly
1               5                   10                  15
Val His Ser Gln Val Gln Leu Gln Gln Pro Gly Ala Glu Leu Val Arg
            20                  25                  30
Pro Gly Ala Ser Val Lys Leu Ser Cys Lys Ala Ser Gly Tyr Thr Phe
        35                  40                  45
Thr Ser Tyr Trp Ile Asn Trp Val Lys Gln Arg Pro Gly Gln Gly Leu
    50                  55                  60
Glu Trp Ile Gly Asn Ile Tyr Pro Ser Asp Ser Tyr Thr Asn Tyr Asn
65                  70                  75                  80
Gln Lys Phe Lys Asp Lys Ala Thr Leu Thr Val Asp Lys Ser Ser Ser
                85                  90                  95
Thr Ala Tyr Met Gln Leu Ser Ser Pro Thr Ser Glu Asp Ser Ala Val
            100                 105                 110
Tyr Tyr Cys Thr Arg Ser Trp Arg Gly Asn Ser Phe Asp Tyr Trp Gly
        115                 120                 125
Gln Gly Thr Thr Leu Thr Val Ser Ser Ala Ser Thr Lys Gly Pro Ser
    130                 135                 140
Val Phe Pro Leu Ala Pro Ser Ser Lys Ser Thr Ser Gly Gly Thr Ala
145                 150                 155                 160
Ala Leu Gly Cys Leu Val Lys Asp Tyr Phe Pro Glu Pro Val Thr Val
                165                 170                 175
Ser Trp Asn Ser Gly Ala Leu Thr Ser Gly Val His Thr Phe Pro Ala
            180                 185                 190
Val Leu Gln Ser Ser Gly Leu Tyr Ser Leu Ser Ser Val Val Thr Val
        195                 200                 205
Pro Ser Ser Ser Leu Gly Thr Gln Thr Tyr Ile Cys Asn Val Asn His
    210                 215                 220
Lys Pro Ser Asn Thr Lys Val Asp Lys Lys Val Glu Pro Lys Ser Cys
225                 230                 235                 240
Asp Lys Thr His Thr Cys Pro Pro Cys Pro Ala Pro Glu Leu Leu Gly
                245                 250                 255
Gly Pro Ser Val Phe Leu Phe Pro Pro Lys Pro Lys Asp Thr Leu Met
            260                 265                 270
Ile Ser Arg Thr Pro Glu Val Thr Cys Val Val Val Asp Val Ser His

275                 280                 285
Glu Asp Pro Glu Val Lys Phe Asn Trp Tyr Val Asp Gly Val Glu Val
    290                 295                 300
His Asn Ala Lys Thr Lys Pro Arg Glu Glu Gln Tyr Asn Ser Thr Tyr
305                 310                 315                 320
Arg Val Val Ser Val Leu Thr Val Leu His Gln Asp Trp Leu Asn Gly
                325                 330                 335
Lys Glu Tyr Lys Cys Lys Val Ser Asn Lys Ala Leu Pro Ala Pro Ile
            340                 345                 350
Glu Lys Thr Ile Ser Lys Ala Lys Gly Gln Pro Arg Glu Pro Gln Val
        355                 360                 365
Tyr Thr Leu Pro Pro Ser Arg Asp Glu Leu Thr Lys Asn Gln Val Ser
    370                 375                 380
Leu Thr Cys Leu Val Lys Gly Phe Tyr Pro Ser Asp Ile Ala Val Glu
385                 390                 395                 400
Trp Glu Ser Asn Gly Gln Pro Glu Asn Asn Tyr Lys Thr Thr Pro Pro
                405                 410                 415
Val Leu Asp Ser Asp Gly Ser Phe Phe Leu Tyr Ser Lys Leu Thr Val
            420                 425                 430
Asp Lys Ser Arg Trp Gln Gln Gly Asn Val Phe Ser Cys Ser Val Met
        435                 440                 445
His Glu Ala Leu His Asn His Tyr Thr Gln Lys Ser Leu Ser Leu Ser
    450                 455                 460
Pro Gly Lys
465

<210>  13
<211>  239
<212>  PRT
<213>  Artificial sequence

<400>  13
Met Glu Thr Asp Thr Leu Leu Leu Trp Val Leu Leu Leu Trp Val Pro
1               5                   10                  15
Gly Ser Thr Gly Asp Ile Val Met Thr Gln Ser Pro Ser Ser Leu Thr
            20                  25                  30
Val Thr Ala Gly Glu Lys Val Thr Met Ser Cys Lys Ser Ser Gln Ser
        35                  40                  45
Leu Leu Asn Ser Gly Asn Gln Lys Asn Tyr Leu Thr Trp Tyr Gln Gln
    50                  55                  60
Lys Pro Gly Gln Pro Pro Lys Leu Leu Ile Tyr Trp Ala Ser Thr Arg
65                  70                  75                  80
Glu Ser Gly Val Pro Asp Arg Phe Thr Gly Ser Gly Ser Gly Thr Asp
                85                  90                  95
Phe Thr Leu Thr Ile Ser Ser Val Gln Ala Glu Asp Leu Ala Val Tyr
            100                 105                 110
Tyr Cys Gln Asn Asp Tyr Ser Tyr Pro Phe Thr Phe Gly Ser Gly Thr
        115                 120                 125
Lys Leu Glu Ile Lys Ala Asp Ala Ala Pro Thr Val Ser Ile Phe Pro
    130                 135                 140
Pro Ser Ser Glu Gln Leu Thr Ser Gly Gly Ala Ser Val Val Cys Phe
145                 150                 155                 160
Leu Asn Asn Phe Tyr Pro Lys Asp Ile Asn Val Lys Trp Lys Ile Asp
                165                 170                 175
Gly Ser Glu Arg Gln Asn Gly Val Leu Asn Ser Trp Thr Asp Gln Asp
            180                 185                 190
Ser Lys Asp Ser Thr Tyr Ser Met Ser Ser Thr Leu Thr Leu Thr Lys
        195                 200                 205
Asp Glu Tyr Glu Arg His Asn Ser Tyr Thr Cys Glu Ala Thr His Lys
    210                 215                 220
Thr Ser Thr Ser Pro Ile Val Lys Ser Phe Asn Arg Asn Glu Cys
225                 230                 235

<210> 14
<211> 466
<212> PRT
<213> Artificial sequence

<400> 14
Met Gly Trp Ser Trp Ile Phe Leu Phe Leu Leu Ser Gly Thr Ala Gly
1               5                   10                  15
Val Leu Ser Gln Val Gln Leu Gln Gln Pro Gly Ala Glu Leu Val Arg
            20                  25                  30
Pro Gly Ala Ser Val Lys Leu Ser Cys Lys Ala Ser Gly Tyr Thr Phe
        35                  40                  45
Thr Ser Tyr Trp Ile Asn Trp Val Lys Gln Arg Pro Gly Gln Gly Leu
    50                  55                  60
Glu Trp Ile Gly Asn Ile Tyr Pro Ser Asp Ser Tyr Thr Asn Tyr Asn
65                  70                  75                  80
Gln Lys Phe Lys Asp Lys Ala Thr Leu Thr Val Asp Lys Ser Ser Ser
                85                  90                  95
Thr Ala Tyr Met Gln Leu Ser Ser Pro Thr Ser Glu Asp Ser Ala Val
            100                 105                 110
Tyr Tyr Cys Thr Arg Ser Trp Arg Gly Asn Ser Phe Asp Tyr Trp Gly
        115                 120                 125
Gln Gly Thr Thr Leu Thr Val Ser Ser Lys Thr Thr Ala Pro Ser Val
    130                 135                 140
Tyr Pro Leu Ala Pro Val Cys Gly Asp Thr Thr Gly Ser Ser Val Thr
145                 150                 155                 160
Leu Gly Cys Leu Val Lys Gly Tyr Phe Pro Glu Pro Val Thr Leu Thr
                165                 170                 175
Trp Asn Ser Gly Ser Leu Ser Ser Gly Val His Thr Phe Pro Ala Val
            180                 185                 190
Leu Gln Ser Asp Leu Tyr Thr Leu Ser Ser Ser Val Thr Val Thr Ser
        195                 200                 205
Ser Thr Trp Pro Ser Gln Ser Ile Thr Cys Asn Val Ala His Pro Ala
    210                 215                 220
Ser Ser Thr Lys Val Asp Lys Lys Ile Glu Pro Arg Gly Pro Thr Ile
225                 230                 235                 240
Lys Pro Cys Pro Pro Cys Lys Cys Pro Ala Pro Asn Leu Leu Gly Gly
                245                 250                 255
Pro Ser Val Phe Ile Phe Pro Pro Lys Ile Lys Asp Val Leu Met Ile
            260                 265                 270
Ser Leu Ser Pro Ile Val Thr Cys Val Val Val Asp Val Ser Glu Asp
        275                 280                 285
Asp Pro Asp Val Gln Ile Ser Trp Phe Val Asn Asn Val Glu Val His
    290                 295                 300
Thr Ala Gln Thr Gln Thr His Arg Glu Asp Tyr Asn Ser Thr Leu Arg
305                 310                 315                 320
Val Val Ser Ala Leu Pro Ile Gln His Gln Asp Trp Met Ser Gly Lys
                325                 330                 335
Glu Phe Lys Cys Lys Val Asn Asn Lys Asp Leu Pro Ala Pro Ile Glu
            340                 345                 350
Arg Thr Ile Ser Lys Pro Lys Gly Ser Val Arg Ala Pro Gln Val Tyr
        355                 360                 365
Val Leu Pro Pro Pro Glu Glu Glu Met Thr Lys Lys Gln Val Thr Leu
    370                 375                 380
Thr Cys Met Val Thr Asp Phe Met Pro Glu Asp Ile Tyr Val Glu Trp
385                 390                 395                 400
Thr Asn Asn Gly Lys Thr Glu Leu Asn Tyr Lys Asn Thr Glu Pro Val
                405                 410                 415
Leu Asp Ser Asp Gly Ser Tyr Phe Met Tyr Ser Lys Leu Arg Val Glu
            420                 425                 430
Lys Lys Asn Trp Val Glu Arg Asn Ser Tyr Ser Cys Ser Val Val His
        435                 440                 445
Glu Gly Leu His Asn His His Thr Thr Lys Ser Phe Ser Arg Thr Pro

450                 455                 460
Gly Lys
465

<210>  15
<211>  31
<212>  DNA
<213>  Artificial sequence

<400>  15
cgcggatcca tgtccaccac cacatgccaa g                                    31

<210>  16
<211>  33
<212>  DNA
<213>  Artificial sequence

<400>  16
ccggaattct tacacatagt cgtgcttgga agg                                  33

<210>  17
<211>  798
<212>  DNA
<213>  Artificial sequence

<400>  17
ggatccatgt ccaccaccac atgccaagtg gtggcgttcc tcctgtccat cctggggctg      60
gccggctgca tcgcggccac cgggatggac atgtggagca cccaggacct gtacgacaac     120
cccgtcacct ccgtgttcca gtacgaaggg ctctggagga gctgcgtgag gcagagttca     180
ggcttcaccg aatgcaggcc ctatttcacc atcctgggac ttccagccat gctgcaggca     240
gtgcgagccc tgatgatcgt aggcatcgtc ctgggtgcca ttggcctcct ggtatccatc     300
tttgccctga aatgcatccg cattggcagc atggaggact ctgccaaagc caacatgaca     360
ctgacctccg ggatcatgtt cattgtctca ggtctttgtg caattgctgg agtgtctgtg     420
tttgccaaca tgctggtgac taacttctgg atgtccacag ctaacatgta caccggcatg     480
ggtgggatgg tgcagactgt tcagaccagg tacacatttg gtgcggctct gttcgtgggc     540
tgggtcgctg gaggcctcac actaattggg ggtgtgatga tgtgcatcgc ctgccggggc     600
ctggcaccag aagaaaccaa ctacaaagcc gtttcttatc atgcctcagg ccacagtgtt     660
gcctacaagc ctggaggctt caaggccagc actggctttg ggtccaacac caaaaacaag     720
aagatatacg atggaggtgc ccgcacagag gacgaggtac aatcttatcc ttccaagcac     780
gactatgtgt aagaattc                                                   798

<210>  18
<211>  25
<212>  DNA
<213>  Artificial sequence

<400>  18
atgrasttsk ggytmarctk grttt                                           25

<210>  19
<211>  26
<212>  DNA
<213>  Artificial sequence

<400>  19
atgraatgsa sctgggtywt yctctt                                          26

<210>  20
<211>  29
<212>  DNA
<213>  Artificial sequence

<400>  20

atggactcca ggctcaattt agttttcct 29

<210> 21
<211> 26
<212> DNA
<213> Artificial sequence

<400> 21
   atggctgtcy trgbgctgyt cytctg 26

<210> 22
<211> 29
<212> DNA
<213> Artificial sequence

<400> 22
atggvttggs tgtggamctt gcyattcct 29

<210> 23
<211> 26
<212> DNA
<213> Artificial sequence

<400> 23
atgaaatgca gctggrtyat sttctt 26

<210> 24
<211> 26
<212> DNA
<213> Artificial sequence

<400> 24
atggrcagrc ttacwtyytc attcct 26

<210> 25
<211> 26
<212> DNA
<213> Artificial sequence

<400> 25
atgatggtgt taagtcttct gtacct 26

<210> 26
<211> 20
<212> DNA
<213> Artificial sequence

<400> 26
tgtcaggaac tgcaggtgtc 20

<210> 27
<211> 20
<212> DNA
<213> Artificial sequence

<400> 27
ggacagggmt ccakagttcc 20

<210> 28
<211> 21
<212> DNA
<213> Artificial sequence

<400> 28
cctggatgac ttcagtgttg t 21

<210> 29
<211> 24
<212> DNA
<213> Artificial sequence

<400> 29
atgragwcac akwcycaggt cttt 24

<210> 30
<211> 31
<212> DNA
<213> Artificial sequence

<400> 30
atgaggrccc ctgctcagwt tyttggwtct t 31

<210> 31
<211> 31
<212> DNA
<213> Artificial sequence

<400> 31
atgggcwtca agatgragtc acakwyycwg g 31

<210> 32
<211> 29
<212> DNA
<213> Artificial sequence

<400> 32
atgagtgtgc ycactcaggt cctggsgtt 29

<210> 33
<211> 31
<212> DNA
<213> Artificial sequence

<400> 33
atgtggggay cgktttyamm cttttcaatt g 31

<210> 34
<211> 28
<212> DNA
<213> Artificial sequence

<400> 34
atggaagccc cagctcagct tctcttcc 28

<210> 35
<211> 28
<212> DNA
<213> Artificial sequence

<400> 35
gtcgttcact gccatcaatc ttccactt 28

<210> 36
<211> 357
<212> DNA
<213> Artificial sequence

<400>  36
cagatccagt tggtgcagtc tggacctgag ctgaagaagc ctggagagac agtcaagatc      60
tcctgcaagg cttctgggta taccttcaca aactatggaa tgaactgggt gaagcaggct     120
ccaggaaagg gtttaaagtg gatgggctgg ataaacacct acactggaga gccaacatat     180
gctgatgact tcaagggacg gtttgccttc tctttggaaa cctctgccag cactgcctat     240
ttgcagatca acaacctcaa aaatgaggac atggctacat atttctgtgc aagagacgga     300
cagctcgggc tacggtttgc ttactggggc caagggactc tggtcactgt ctctgca        357

<210>  37
<211>  336
<212>  DNA
<213>  Artificial sequence

<400>  37
aacattatga tgacacagtc gccatcatct ctggctgtgt ctgcaggaga aaaggtcact      60
atgagctgta agtccagtca aagtgtttta tacagttcaa atcagaagaa ctacttggcc     120
tggtaccagc agaaaccagg gcagtctcct aaactgctga tctactgggc atccactagg     180
gaatctggtg tccctgatcg cttcacaggc agtggatctg ggacagattt tactcttacc     240
atcagcagtg tacaagctga agacctggca gtttattact gtcatcaata cctctcctcg     300
cggacgttcg gtggaggcac caagctggaa atcaaa                               336

<210>  38
<211>  355
<212>  DNA
<213>  Artificial sequence

<400>  38
gaggtgaagc tggtggagtc tgggggaggc ttagtgcagc ctggagggtc cctgaaactc      60
tcctgtgcag cctctggatt cactttcagt gactacggaa tggcgtgggt tcgacaggct     120
ccaaggaagg ggcctgagtg ggtagcattc attagtaatt tggcatatag tatctactat     180
gcagacactg tgacgggccg attcaccatc tctagagaga atgccaagaa caccctgtac     240
ctggaaatga gcagtctgag gtctgaggac acggccatgt attactgtgc aagacttggg     300
tatggtaact cctttgctta ctggggccaa gggactctgg tcactgtctc tgcag          355

<210>  39
<211>  340
<212>  DNA
<213>  Artificial sequence

<400>  39
gacattgtga tgacacagtc tccatcctcc ctgactgtga cagcaggaga gaaggtcact      60
atgagctgca agtccagtca gagtctgtta aacagtggaa atcaaaagaa ctacttgacc     120
tggtaccagc agaaaccagg gcagcctcct aaactgttga tctactgggc atccactagg     180
gaatctgggg tccctgatcg cttcacaggc agtggatctg gaacagattt cactctcacc     240
atcagcagtg tgcaggctga agacctggca gtttattact gtcagaatga ttatagttat     300
ccgctcacgt tcggtgctgg gaccaagctg gagctgaaac                           340

<210>  40
<211>  345
<212>  DNA
<213>  Artificial sequence

<400>  40
aggttcagct gcagcagtct ggagctgagc tggcgaggcc tggggcttca gtgaagctgt      60
cctgcaaggc ttctggctac accttcacaa gctatggtat aagctgggtg aagcagagaa     120
ctggacaggg ccttgagtgg attggagaga tttatcctag aagtggtaat acttactaca     180
atgagaagtt caagggcaag gccacactga ctgcagacaa atcctccagc acagcgtaca     240
tggagctccg cagcctgaca tctgaggact ctgcggtcta tttctgtgca agccttgcct     300
ggtttgctta ctggggccaa gggactctgg tcactgtctc tgcag                     345

<210>  41
<211>  337

<212>  DNA
<213>  Artificial sequence

<400>  41
gatattgtga tgactcaggc tgcaccctct gtacctgtca ctcctggaga gtcagtatcc      60
atctcctgca ggtctagtaa gagtctcctg catagtaatg gcaacactta cttgtattgg     120
ttcctgcaga ggccaggcca gtctcctcag ctcctgatat atcggatgtc caaccttgcc     180
tcaggagtcc cagacaggtt cagtggcagt gggtcaggaa ctgctttcac actgagaatc     240
agtagagtgg aggctgagga tgtgggtgtt tattactgta tgcaacatct agaatatccg     300
tacacgttcg gaggggggac caagctggaa ataaaac                              337

<210>  42
<211>  355
<212>  DNA
<213>  Artificial sequence

<400>  42
gaggtccagc tgcaacagtc tggacctgag ctggtgaagc ctggggcttc agtgaagatg      60
tcctgcaagg cttctggata cacattcact gactacaaca tgcactgggt gaagcagagc     120
catggaaaga gccttgagtg gattggatat attaacccta acaatggtgg tactagctac     180
aaccagaagt tcaagggcaa ggccacattg actgtaaaca agtcctccag cacagcctac     240
atggagctcc gcagcctgac atcggaggat tctgcagtct attactgtgc aaggatctac     300
tatggtaact cgtttgctta ctggggccaa gggactctgg tcactgtctc tgcag          355

<210>  43
<211>  340
<212>  DNA
<213>  Artificial sequence

<400>  43
gacattgtga tgacacagtc tccatcctcc ctgactgtga cagcaggaga gaaggtcact      60
atgagctgca agtccagtca gagtctgtta aacagtggaa atcaaaagaa ctacttgacc     120
tggtaccagc agaaaccagg gcagcctcct aaactgttga tctactgggc atccactagg     180
gaatctgggg tccctgatcg cttcacaggc agtggatctg gaacagattt cactctcacc     240
atcagcagtg tgcaggctga agacctggca gtttattact gtcagaatga ttatagttat     300
ccattcacgt tcggctcggg gacaaagttg gaaataaaac                           340

<210>  44
<211>  348
<212>  DNA
<213>  Artificial sequence

<400>  44
aggtgcagct gaaggagtca ggacctggcc tggtggcgcc ctcacagagc ctgtccatca      60
catgcactgt ctcagggttc tcattaacca gctatggtgt aagctgggtt cgccagcctc     120
caggaaaggg tctggagtgg ctgggagtaa tatggggtga cgggagcaca aattatcatt     180
cagctctcat atccagactg agcatcagca aggataactc caagagccaa gttttcttaa     240
aactgaacag tctgcaaact gatgacacag ccacgtacta ctgtgccggg ggattacgac     300
tctggtttgc ttactggggc caagggactc tggtcactgt ctctgcag                  348

<210>  45
<211>  340
<212>  DNA
<213>  Artificial sequence

<400>  45
gacattgtga tgacacagtc tccatcctcc ctggctatgt cagtaggaca gaaggtcact      60
atgagctgca agtccagtca gagcctttta aatagtagca atcaaaagaa ctatttggcc     120
tggtaccagc agaaaccagg acagtctcct aaacttctgg tatactttgc atccactagg     180
gaatctgggg tccctgatcg cttcataggc agtggatctg ggacagattt cactcttacc     240
atcagcagtg tgcaggctga agacctggca gattacttct gtcagcaaca ttatagcact     300
ccattcacgt tcggctcggg gacaaagttg gaaataaaac                           340

<210>  46
<211>  361
<212>  DNA
<213>  Artificial sequence

<400>  46
caggttcagc tgcagcagtc tggggctgag ctagtgaagc ctggagcctc agtgaagatg      60
tcctgcaagg cttctggcta caccttcact acctatccta tagagtggat gaagcagaat     120
catggaaaga gcctagagtg gattggaaat tttcatcctt acaatgatga tactaagtac     180
aatgaaaagt tcaagggcaa ggccacattg actgtagaaa aatcctctag cacagtctac     240
ttggagctca gccgattaac atctgatgac tctgctgttt attactgtgc aaggaggggc     300
tacggtagta gctacgctat ggactactgg ggtcaaggaa cctcagtcac cgtctcctca     360
g                                                                     361

<210>  47
<211>  340
<212>  DNA
<213>  Artificial sequence

<400>  47
gacattgtga tgacacagtc tccatcctcc ctgagtgtgt cagcaggaga gaaggtcact      60
atgagctgca agtccagtca gagtctgtta aacagtggaa atcaaaagaa ctacttggcc     120
tggtaccagc agaaaccagg gcagcctcct aaactgttga tctacggggc atccactagg     180
gaatctgggg tccctgatcg cttcacaggc agtggatctg gaaccgattt cactcttacc     240
atcagcagtg tgcaggctga agacctggca gtttattact gtcagaatga tcatagttat     300
ccgtacacgt tcggaggggg gaccaagctg gaaataaaac                           340

<210>  48
<211>  119
<212>  PRT
<213>  Artificial sequence

<400>  48
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Asn Tyr
            20                  25                  30
Gly Met Asn Trp Val Lys Gln Ala Pro Gly Lys Gly Leu Lys Trp Met
        35                  40                  45
Gly Trp Ile Asn Thr Tyr Thr Gly Glu Pro Thr Tyr Ala Asp Asp Phe
    50                  55                  60
Lys Gly Arg Phe Ala Phe Ser Leu Glu Thr Ser Ala Ser Thr Ala Tyr
65                  70                  75                  80
Leu Gln Ile Asn Asn Leu Lys Asn Glu Asp Met Ala Thr Tyr Phe Cys
                85                  90                  95
Ala Arg Asp Gly Gln Leu Gly Leu Arg Phe Ala Tyr Trp Gly Gln Gly
            100                 105                 110
Thr Leu Val Thr Val Ser Ala
        115

<210>  49
<211>  112
<212>  PRT
<213>  Artificial sequence

<400>  49
Asn Ile Met Met Thr Gln Ser Pro Ser Ser Leu Ala Val Ser Ala Gly
1               5                   10                  15
Glu Lys Val Thr Met Ser Cys Lys Ser Ser Gln Ser Val Leu Tyr Ser
            20                  25                  30
Ser Asn Gln Lys Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val

50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys His Gln
                85                  90                  95
Tyr Leu Ser Ser Arg Thr Phe Gly Gly Gly Thr Lys Leu Glu Ile Lys
            100                 105                 110

<210>  50
<211>  118
<212>  PRT
<213>  Artificial sequence

<400>  50
Glu Val Lys Leu Val Glu Ser Gly Gly Gly Leu Val Gln Pro Gly Gly
1               5                   10                  15
Ser Leu Lys Leu Ser Cys Ala Ala Ser Gly Phe Thr Phe Ser Asp Tyr
            20                  25                  30
Gly Met Ala Trp Val Arg Gln Ala Pro Arg Lys Gly Pro Glu Trp Val
        35                  40                  45
Ala Phe Ile Ser Asn Leu Ala Tyr Ser Ile Tyr Tyr Ala Asp Thr Val
    50                  55                  60
Thr Gly Arg Phe Thr Ile Ser Arg Glu Asn Ala Lys Asn Thr Leu Tyr
65                  70                  75                  80
Leu Glu Met Ser Ser Leu Arg Ser Glu Asp Thr Ala Met Tyr Tyr Cys
                85                  90                  95
Ala Arg Leu Gly Tyr Gly Asn Ser Phe Ala Tyr Trp Gly Gln Gly Thr
            100                 105                 110
Leu Val Thr Val Ser Ala
        115

<210>  51
<211>  113
<212>  PRT
<213>  Artificial sequence

<400>  51
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asp Tyr Ser Tyr Pro Leu Thr Phe Gly Ala Gly Thr Lys Leu Glu Leu
            100                 105                 110
Lys


<210>  52
<211>  355
<212>  DNA
<213>  Artificial sequence

<400>  52
gaagtgaaac tggtggagag cggcggcggc ctcgtgcagc ctggaggctc cctgaagctg        60
agctgtgccg ccagcggctt tacctttagc gattacggca tggcctgggt gagacaggcc       120
cctagaaagg gccccgaatg ggtggccttc atctccaacc tggcctacag catctactac       180

gccgacaccg tgaccggaag attcaccatc tccagagaga acgccaagaa caccctgtac 240
ctggagatga gctccctgag gagcgaggac accgccatgt actactgcgc caggctgggc 300
tacggcaaca gcttcgccta ctggggacag ggcaccctgg tgacagtgag cgctg 355

<210> 53
<211> 340
<212> DNA
<213> Artificial sequence

<400> 53
gacatcgtga tgacccagag cccctcctcc ctgacagtga ccgccggcga gaaggtgacc 60
atgagctgca agagctccca gagcctgctg aacagcggca atcagaagaa ctacctgacc 120
tggtaccagc agaagcccgg ccagcctccc aagctgctga tctactgggc cagcaccaga 180
gagagcggcg tgcctgacag attcaccggc agcggctccg gcaccgactt taccctgacc 240
atctccagcg tgcaggccga ggacctggcc gtgtactatt gccagaacga ctacagctac 300
cccctgacct tcggcgccgg aaccaagctg gagctgaaga 340

<210> 54
<211> 115
<212> PRT
<213> Artificial sequence

<400> 54
Gln Val Gln Leu Gln Gln Ser Gly Ala Glu Leu Ala Arg Pro Gly Ala
1               5                   10                  15
Ser Val Lys Leu Ser Cys Lys Ala Ser Gly Tyr Thr Phe Thr Ser Tyr
            20                  25                  30
Gly Ile Ser Trp Val Lys Gln Arg Thr Gly Gln Gly Leu Glu Trp Ile
        35                  40                  45
Gly Glu Ile Tyr Pro Arg Ser Gly Asn Thr Tyr Tyr Asn Glu Lys Phe
    50                  55                  60
Lys Gly Lys Ala Thr Leu Thr Ala Asp Lys Ser Ser Ser Thr Ala Tyr
65                  70                  75                  80
Met Glu Leu Arg Ser Leu Thr Ser Glu Asp Ser Ala Val Tyr Phe Cys
                85                  90                  95
Ala Ser Leu Ala Trp Phe Ala Tyr Trp Gly Gln Gly Thr Leu Val Thr
            100                 105                 110
Val Ser Ala
        115

<210> 55
<211> 112
<212> PRT
<213> Artificial sequence

<400> 55
Asp Ile Val Met Thr Gln Ala Ala Pro Ser Val Pro Val Thr Pro Gly
1               5                   10                  15
Glu Ser Val Ser Ile Ser Cys Arg Ser Ser Lys Ser Leu Leu His Ser
            20                  25                  30
Asn Gly Asn Thr Tyr Leu Tyr Trp Phe Leu Gln Arg Pro Gly Gln Ser
        35                  40                  45
Pro Gln Leu Leu Ile Tyr Arg Met Ser Asn Leu Ala Ser Gly Val Pro
    50                  55                  60
Asp Arg Phe Ser Gly Ser Gly Ser Gly Thr Ala Phe Thr Leu Arg Ile
65                  70                  75                  80
Ser Arg Val Glu Ala Glu Asp Val Gly Val Tyr Tyr Cys Met Gln His
                85                  90                  95
Leu Glu Tyr Pro Tyr Thr Phe Gly Gly Gly Thr Lys Leu Glu Ile Lys
            100                 105                 110

<210> 56
<211> 346

<212>  DNA
<213>  Artificial sequence

<400>  56
caggtgcagc tgcaacaatc cggcgccgag ctggccaggc ctggagcctc cgtgaagctg      60
agctgcaagg cctccggcta caccttcacc tcctacggca tcagctgggt gaaacagagg     120
acaggccagg gcctggagtg gattggcgag atctacccca gatccggaaa cacctactac     180
aacgagaagt tcaagggcaa ggctaccctg accgctgaca agagcagcag cacagcttac     240
atggagctga gatccctgac cagcgaggat agcgccgtct acttctgcgc cagcctcgct     300
tggtttgctt actggggcca gggaaccctg gtgaccgtga gcgctg                    346

<210>  57
<211>  337
<212>  DNA
<213>  Artificial sequence

<400>  57
gacatcgtga tgacccaggc tgccccttcc gtgcctgtga cccctggaga gagcgtgagc      60
atcagctgca ggagcagcaa gtccctgctg cacagcaacg gcaacaccta cctgtactgg     120
tttctgcaga gacctggcca gagcccccag ctgctgatct acaggatgag caatctggcc     180
tccggcgtgc ccgacagatt tagcggctcc ggcagcggca ccgcctttac cctgaggatc     240
agcagggtgg aggccgagga cgtgggcgtg tactactgta tgcagcacct ggagtacccc     300
tataccttcg gcggcggcac caagctggag atcaaga                              337

<210>  58
<211>  118
<212>  PRT
<213>  Artificial sequence

<400>  58
Glu Val Gln Leu Gln Gln Ser Gly Pro Glu Leu Val Lys Pro Gly Ala
1               5                   10                  15
Ser Val Lys Met Ser Cys Lys Ala Ser Gly Tyr Thr Phe Thr Asp Tyr
            20                  25                  30
Asn Met His Trp Val Lys Gln Ser His Gly Lys Ser Leu Glu Trp Ile
        35                  40                  45
Gly Tyr Ile Asn Pro Asn Asn Gly Gly Thr Ser Tyr Asn Gln Lys Phe
    50                  55                  60
Lys Gly Lys Ala Thr Leu Thr Val Asn Lys Ser Ser Ser Thr Ala Tyr
65                  70                  75                  80
Met Glu Leu Arg Ser Leu Thr Ser Glu Asp Ser Ala Val Tyr Tyr Cys
                85                  90                  95
Ala Arg Ile Tyr Tyr Gly Asn Ser Phe Ala Tyr Trp Gly Gln Gly Thr
            100                 105                 110
Leu Val Thr Val Ser Ala
        115

<210>  59
<211>  113
<212>  PRT
<213>  Artificial sequence

<400>  59
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95
Asp Tyr Ser Tyr Pro Phe Thr Phe Gly Ser Gly Thr Lys Leu Glu Ile
            100                 105                 110
Lys

<210>  60
<211>  354
<212>  DNA
<213>  Artificial sequence

<400>  60
gaggtgcagc tgcagcagag cggacccgaa ctggtgaaac ccggcgcctc cgtgaagatg      60
agctgcaagg ccagcggcta caccttcacc gactacaaca tgcactgggt gaagcagagc     120
cacggcaagt ctttagagtg gattggctac atcaacccca acaacggcgg caccagctac     180
aaccagaaat tcaagggcaa ggccaccctc accgtgaaca agagctccag caccgcctac     240
atggagttac gttctttaac ctccgaggac agcgccgtgt actactgcgc tcgtatctac     300
tacggcaaca gcttcgctta ctggggccaa ggtactttag tgaccgtgag cgcc           354

<210>  61
<211>  340
<212>  DNA
<213>  Artificial sequence

<400>  61
gatatcgtga tgacccagtc tccatccagc ctgaccgtga cagccggcga gaaggtgacc      60
atgagctgca agtcttccca gtctctgctg aactccggca atcagaagaa ctacctgaca     120
tggtatcagc agaagccagg ccagccccct aagctgctga tctactgggc tagcaccagg     180
gagtctggag tgccagaccg gttcacaggc agcggctctg gcaccgactt caccctgaca     240
atcagctctg tgcaggccga ggacctggcc gtgtactatt gccagaatga ttactcttat     300
cccttcacat ttggctccgg caccaagctg gagatcaaga                           340

<210>  62
<211>  116
<212>  PRT
<213>  Artificial sequence

<400>  62
Gln Val Gln Leu Lys Glu Ser Gly Pro Gly Leu Val Ala Pro Ser Gln
1               5                   10                  15
Ser Leu Ser Ile Thr Cys Thr Val Ser Gly Phe Ser Leu Thr Ser Tyr
            20                  25                  30
Gly Val Ser Trp Val Arg Gln Pro Pro Gly Lys Gly Leu Glu Trp Leu
        35                  40                  45
Gly Val Ile Trp Gly Asp Gly Ser Thr Asn Tyr His Ser Ala Leu Ile
    50                  55                  60
Ser Arg Leu Ser Ile Ser Lys Asp Asn Ser Lys Ser Gln Val Phe Leu
65                  70                  75                  80
Lys Leu Asn Ser Leu Gln Thr Asp Asp Thr Ala Thr Tyr Tyr Cys Ala
                85                  90                  95
Gly Gly Leu Arg Leu Trp Phe Ala Tyr Trp Gly Gln Gly Thr Leu Val
            100                 105                 110
Thr Val Ser Ala
        115

<210>  63
<211>  113
<212>  PRT
<213>  Artificial sequence

<400>  63
Asp Ile Val Met Thr Gln Ser Pro Ser Ser Leu Ala Met Ser Val Gly

1               5                   10                  15
Gln Lys Val Thr Met Ser Cys Lys Ser Ser Gln Ser Leu Leu Asn Ser
            20                  25                  30
Ser Asn Gln Lys Asn Tyr Leu Ala Trp Tyr Gln Gln Lys Pro Gly Gln
        35                  40                  45
Ser Pro Lys Leu Leu Val Tyr Phe Ala Ser Thr Arg Glu Ser Gly Val
    50                  55                  60
Pro Asp Arg Phe Ile Gly Ser Gly Ser Gly Thr Asp Phe Thr Leu Thr
65                  70                  75                  80
Ile Ser Ser Val Gln Ala Glu Asp Leu Ala Asp Tyr Phe Cys Gln Gln
                85                  90                  95
His Tyr Ser Thr Pro Phe Thr Phe Gly Ser Gly Thr Lys Leu Glu Ile
            100                 105                 110
Lys

<210>  64
<211>  348
<212>  DNA
<213>  Artificial sequence

<400>  64
caagttcagc tgaaggagag cggccccggt ctggtggctc ctagccagtc tttaagcatc      60
acttgtaccg tgagcggctt ctctttaacc agctacggcg tgagctgggt gagacagcct     120
cccggtaagg gtttagagtg gctgggagtg atctggggcg acggcagcac caactaccac     180
agcgctttaa tctctcgtct gtccatcagc aaggacaaca gcaagagcca agttttttta      240
aagctgaact ctttacagac cgacgacacc gccacctact actgcgccgg aggtttaagg     300
ctgtggtttg cctactgggg ccaaggtact ttagtgacag tgagcgcc                  348

<210>  65
<211>  339
<212>  DNA
<213>  Artificial sequence

<400>  65
gacatcgtga tgacccagtc ccccagctct ttagctatga gcgtgggcca gaaggtgacc      60
atgagctgca agtccagcca gtctttactg aacagcagca accagaagaa ctatttagct     120
tggtaccagc agaagcccgg ccagagcccc aagctgctgg tgtacttcgc cagcactcgt     180
gagagcggcg tgcccgatcg tttcatcggc agcggcagcg gcaccgattt cactttaacc     240
atcagcagcg tgcaagctga ggatctggcc gactacttct gccagcagca ctacagcacc     300
cccttcacct tcggcagcgg caccaagctg gagatcaag                            339

<210>  66
<211>  120
<212>  PRT
<213>  Artificial sequence

<400>  66
Gln Val Gln Leu Gln Gln Ser Gly Ala Glu Leu Val Lys Pro Gly Ala
1               5                   10                  15
Ser Val Lys Met Ser Cys Lys Ala Ser Gly Tyr Thr Phe Thr Thr Tyr
            20                  25                  30
Pro Ile Glu Trp Met Lys Gln Asn His Gly Lys Ser Leu Glu Trp Ile
        35                  40                  45
Gly Asn Phe His Pro Tyr Asn Asp Asp Thr Lys Tyr Asn Glu Lys Phe
    50                  55                  60
Lys Gly Lys Ala Thr Leu Thr Val Glu Lys Ser Ser Ser Thr Val Tyr
65                  70                  75                  80
Leu Glu Leu Ser Arg Leu Thr Ser Asp Asp Ser Ala Val Tyr Tyr Cys
                85                  90                  95
Ala Arg Arg Gly Tyr Gly Ser Ser Tyr Ala Met Asp Tyr Trp Gly Gln
            100                 105                 110
Gly Thr Ser Val Thr Val Ser Ser

115                 120

<210>  67
<211>  113
<212>  PRT
<213>  Artificial sequence

<400>  67
Asp Ile Val Met Thr Gln Ser Pro Ser Ser Leu Ser Val Ser Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asp His Ser Tyr Pro Tyr Thr Phe Gly Gly Gly Thr Lys Leu Glu Ile
            100                 105                 110
Lys

<210>  68
<211>  360
<212>  DNA
<213>  Artificial sequence

<400>  68
caagttcagc tgcagcagag cggagccgaa ctggtgaagc ccggtgccag cgtgaagatg      60
agctgcaagg ccagcggcta caccttcacc acctacccca tcgagtggat gaagcagaac     120
cacggcaagt ctttagagtg gatcggcaac ttccacccct acaacgacga caccaagtac     180
aacgagaagt tcaagggcaa ggccacttta accgtggaga agagcagcag caccgtgtat     240
ttagagctga gcagactgac cagcgacgac agcgccgtgt actactgcgc tcgtagaggc     300
tacggcagca gctacgccat ggactactgg ggccaaggta ccagcgtgac cgtgagcagc     360

<210>  69
<211>  339
<212>  DNA
<213>  Artificial sequence

<400>  69
gacatcgtga tgacccagag ccctagctct ttaagcgtga gcgccggcga gaaggtgacc      60
atgagctgca agagcagcca gtctttactg aacagcggca accagaagaa ctatttagct     120
tggtaccaac agaagcccgg ccagcccccc aagctgctga tctacggcgc cagcaccaga     180
gagagcggag tgcccgatag gtttaccggc agcggcagcg gcaccgactt cactttaacc     240
atcagcagcg tgcaagctga ggatctggcc gtgtactact gccagaacga ccacagctac     300
ccctacacct tcggcggcgg caccaagctg gagatcaag                            339

<210>  70
<211>  113
<212>  PRT
<213>  Artificial sequence

<400>  70
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asn Tyr Tyr Tyr Pro Leu Thr Phe Gly Ala Gly Thr Lys Leu Glu Leu
            100                 105                 110
Lys

<210>  71
<211>  113
<212>  PRT
<213>  Artificial sequence

<400>  71
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asp Tyr Ser Leu Pro Phe Thr Phe Gly Ala Gly Thr Lys Leu Glu Leu
            100                 105                 110
Lys

<210>  72
<211>  118
<212>  PRT
<213>  Artificial sequence

<400>  72
Glu Val Lys Leu Val Glu Ser Gly Gly Gly Leu Val Gln Pro Gly Gly
1               5                   10                  15
Ser Leu Lys Leu Ser Cys Ala Ala Ser Gly Phe Thr Phe Ser Arg Tyr
            20                  25                  30
Gly Met Ala Trp Val Arg Gln Ala Pro Gly Lys Gly Pro Glu Trp Val
        35                  40                  45
Ala Phe Ile Ser Asn Ser Ala Tyr Ser Ile Tyr Tyr Ala Asp Thr Val
    50                  55                  60
Thr Gly Arg Phe Thr Ile Ser Arg Glu Asn Ala Lys Asn Thr Leu Tyr
65                  70                  75                  80
Leu Glu Met Ser Ser Leu Arg Ser Glu Asp Thr Ala Met Tyr Tyr Cys
                85                  90                  95
Ala Arg Leu Gly Tyr Gly Asn Ser Phe Ala Tyr Trp Gly Gln Gly Thr
            100                 105                 110
Leu Val Thr Val Ser Ala
        115

<210>  73
<211>  113
<212>  PRT
<213>  Artificial sequence

<400>  73

Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Gly Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asp Tyr Ser Tyr Pro Leu Thr Phe Gly Ala Gly Thr Lys Leu Glu Leu
            100                 105                 110
Lys

<210>  74
<211>  118
<212>  PRT
<213>  Artificial sequence

<400>  74
Glu Val Lys Leu Val Glu Ser Gly Gly Gly Leu Val Gln Pro Gly Gly
1               5                   10                  15
Ser Leu Lys Leu Ser Cys Ala Ala Ser Gly Phe Gly Phe Ser Asp Tyr
            20                  25                  30
Gly Met Ala Trp Val Arg Gln Ala Pro Arg Lys Gly Pro Glu Trp Val
        35                  40                  45
Ala Phe Ile Ser Asn Ser Ala Tyr Ser Ile Tyr Tyr Ala Asp Thr Val
    50                  55                  60
Thr Gly Arg Phe Thr Ile Ser Arg Glu Asn Ala Lys Asn Thr Leu Tyr
65                  70                  75                  80
Leu Glu Met Ser Ser Leu Arg Ser Glu Asp Thr Ala Met Tyr Tyr Cys
                85                  90                  95
Ala Arg Leu Gly Tyr Gly Asn Ser Phe Ala Tyr Trp Gly Gln Gly Thr
            100                 105                 110
Leu Val Thr Val Ser Ala
        115

<210>  75
<211>  113
<212>  PRT
<213>  Artificial sequence

<400>  75
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Pro Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asp Tyr Ser Tyr Pro Leu Thr Phe Gly Ala Gly Thr Lys Leu Glu Leu
            100                 105                 110
Lys

<210>  76
<211>  113
<212>  PRT
<213>  Artificial sequence

<400>  76
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Glu Leu Phe Tyr Pro Leu Thr Phe Gly Ala Gly Thr Lys Leu Glu Leu
            100                 105                 110
Lys

<210>  77
<211>  118
<212>  PRT
<213>  Artificial sequence

<400>  77
Glu Val Lys Leu Val Glu Ser Gly Gly Gly Leu Val Gln Pro Gly Gly
1               5                   10                  15
Ser Leu Lys Leu Ser Cys Ala Ala Ser Gly Phe Thr Phe Ser Asp Tyr
            20                  25                  30
Gly Met Ala Trp Val Arg Gln Ala Pro Arg Lys Gly Pro Glu Trp Val
        35                  40                  45
Ala Phe Ile Ser Asn Leu Ala Tyr Ser Ile Tyr Tyr Ala Asp Thr Val
    50                  55                  60
Thr Gly Arg Phe Thr Ile Ser Arg Glu Asn Ala Lys Asn Thr Leu Tyr
65                  70                  75                  80
Leu Glu Met Ser Ser Leu Arg Ser Glu Asp Thr Ala Met Tyr Tyr Cys
                85                  90                  95
Ala Arg Leu Gly Arg Gly Asn Ser Phe Ala Tyr Trp Gly Gln Gly Thr
            100                 105                 110
Leu Val Thr Val Ser Ala
        115

<210>  78
<211>  113
<212>  PRT
<213>  Artificial sequence

<400>  78
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn

85                  90                  95
Asp Tyr Ser Phe Pro Leu Thr Phe Gly Ala Gly Thr Lys Leu Glu Leu
            100                 105                 110
Lys

<210>  79
<211>  118
<212>  PRT
<213>  Artificial sequence

<400>  79
Glu Val Lys Leu Val Glu Ser Gly Gly Gly Leu Val Gln Pro Gly Gly
1               5                   10                  15
Ser Leu Lys Leu Ser Cys Ala Ala Ser Gly Phe Gly Phe Ser Asp Tyr
            20                  25                  30
Gly Met Ala Trp Val Arg Gln Ala Pro Arg Lys Gly Pro Glu Trp Val
        35                  40                  45
Ala Phe Ile Ser Asn Ser Ala Tyr Ser Ile Tyr Tyr Ala Asp Thr Val
    50                  55                  60
Thr Gly Arg Phe Thr Ile Ser Arg Glu Asn Ala Lys Asn Thr Leu Tyr
65                  70                  75                  80
Leu Glu Met Ser Ser Leu Arg Ser Glu Asp Thr Ala Met Tyr Tyr Cys
                85                  90                  95
Ala Arg Leu Gly Arg Gly Asn Ser Phe Ala Tyr Trp Gly Gln Gly Thr
            100                 105                 110
Leu Val Thr Val Ser Ala
        115

<210>  80
<211>  113
<212>  PRT
<213>  Artificial sequence

<400>  80
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Gly Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asn Tyr Tyr Tyr Pro Leu Thr Phe Gly Ala Gly Thr Lys Leu Glu Leu
            100                 105                 110
Lys

<210>  81
<211>  113
<212>  PRT
<213>  Artificial sequence

<400>  81
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln

35                  40                  45
Pro Pro Lys Leu Leu Ile Tyr Trp Gly Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Glu Leu Phe Tyr Pro Leu Thr Phe Gly Ala Gly Thr Lys Leu Glu Leu
            100                 105                 110
Lys

<210>  82
<211>  118
<212>  PRT
<213>  Artificial sequence

<400>  82
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Gly Phe Ser Asp Tyr
            20                  25                  30
Gly Met Ala Trp Val Arg Gln Ala Pro Gly Lys Gly Leu Glu Trp Val
        35                  40                  45
Ser Phe Ile Ser Asn Ser Ala Tyr Ser Ile Tyr Tyr Ala Asp Thr Val
    50                  55                  60
Thr Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Leu Gly Arg Gly Asn Ser Phe Ala Tyr Trp Gly Gln Gly Thr
            100                 105                 110
Leu Val Thr Val Ser Ser
        115

<210>  83
<211>  354
<212>  DNA
<213>  Artificial sequence

<400>  83
gaggtgcagc tggtggagag cggcggcggc ctggtgcagc ccggcggcag cctgagactg      60
agctgcgccg ccagcggctt cggcttcagc gactacggca tggcctgggt gagacaggcc     120
cccggcaagg gcctggagtg ggtgagcttc atcagcaaca gcgcctacag catctactac     180
gccgacaccg tgaccggcag attcaccatc agcagagaca acgccaagaa cagcctgtac     240
ctgcagatga acagcctgag agccgaggac accgccgtgt actactgcgc cagactgggc     300
agaggcaaca gcttcgccta ctggggccag ggcaccctgg tgaccgtgag cagc           354

<210>  84
<211>  113
<212>  PRT
<213>  Artificial sequence

<400>  84
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Gly Ser Thr Arg Glu Ser Gly Val
    50                  55                  60
Pro Asp Arg Phe Ser Gly Ser Gly Ser Gly Thr Asp Phe Thr Leu Thr

65                  70                  75                  80
Ile Ser Ser Leu Gln Ala Glu Asp Val Ala Val Tyr Tyr Cys Gln Asn
                85                  90                  95
Glu Leu Phe Tyr Pro Leu Thr Phe Gly Gln Gly Thr Lys Leu Glu Ile
            100                 105                 110
Lys

<210>  85
<211>  339
<212>  DNA
<213>  Artificial sequence

<400>  85
gacatcgtga tgacccagag ccccgacagc ctggccgtga gcctgggcga gagagccacc      60
atcaactgca agagcagcca gagcctgctg aacagcggca accagaagaa ctacctgacc     120
tggtaccagc agaagcccgg ccagcccccc aagctgctga tctactgggg cagcaccaga     180
gagagcggcg tgcccgacag attcagcggc agcggcagcg gcaccgactt caccctgacc     240
atcagcagcc tgcaggccga ggacgtggcc gtgtactact gccagaacga gctgttctac     300
cccctgacct tcggccaggg caccaagctg gagatcaag                            339

<210>  86
<211>  113
<212>  PRT
<213>  Artificial sequence

<400>  86
Glu Ile Val Met Thr Gln Ser Pro Pro Thr Leu Ser Leu Ser Pro Gly
1               5                   10                  15
Glu Arg Val Thr Leu Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Ala Pro Arg Leu Leu Ile Tyr Trp Gly Ser Thr Arg Glu Ser Ser Ile
    50                  55                  60
Pro Ala Arg Phe Ser Gly Ser Gly Ser Gly Thr Asp Phe Thr Leu Thr
65                  70                  75                  80
Ile Ser Ser Leu Gln Pro Glu Asp Leu Ala Val Tyr Tyr Cys Gln Asn
                85                  90                  95
Glu Leu Phe Tyr Pro Leu Thr Phe Gly Gln Gly Thr Lys Leu Glu Ile
            100                 105                 110
Lys

<210>  87
<211>  339
<212>  DNA
<213>  Artificial sequence

<400>  87
gagatcgtga tgacccagag cccccccacc ctgagcctga gccccggcga gagagtgacc      60
ctgagctgca agagcagcca gagcctgctg aacagcggca accagaagaa ctacctgacc     120
tggtaccagc agaagcccgg ccaggccccc agactgctga tctactgggg cagcaccaga     180
gagagcagca tccccgccag attcagcggc agcggcagcg gcaccgactt caccctgacc     240
atcagcagcc tgcagcccga ggacctggcc gtgtactact gccagaacga gctgttctac     300
cccctgacct tcggccaggg caccaagctg gagatcaag                            339

<210>  88
<211>  118
<212>  PRT
<213>  Artificial sequence

<400>  88

Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Gly Phe Ser Asp Tyr
            20                  25                  30
Gly Met Ala Trp Val Arg Gln Ala Pro Gly Lys Gly Leu Glu Trp Val
        35                  40                  45
Ser Phe Ile Ser Asn Ser Ala Tyr Ser Ile Tyr Tyr Ala Asp Thr Val
    50                  55                  60
Thr Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Leu Gly Arg Gly Asn Ser Phe Ala Tyr Trp Gly Gln Gly Thr
            100                 105                 110
Leu Val Thr Val Ser Ser
        115

<210>  89
<211>  354
<212>  DNA
<213>  Artificial sequence

<400>  89
gaggtgcagc tgctggagag cggcggcggc ctggtgcagc ccggcggcag cctgagactg      60
agctgcgccg ccagcggctt cggcttcagc gactacggca tggcctgggt gagacaggcc     120
cccggcaagg gcctggagtg ggtgagcttc atcagcaaca gcgcctacag catctactac     180
gccgacaccg tgaccggcag attcaccatc agcagagaca acagcaagaa caccctgtac     240
ctgcagatga acagcctgag agccgaggac accgccgtgt actactgcgc cagactgggc     300
agaggcaaca gcttcgccta ctggggccag ggcaccctgg tgaccgtgag cagc           354

<210>  90
<211>  113
<212>  PRT
<213>  Artificial sequence

<400>  90
Asp Ile Val Met Thr Gln Ser Pro Leu Ser Leu Pro Val Thr Leu Gly
1               5                   10                  15
Gln Pro Ala Ser Ile Ser Cys Lys Ser Ser Gln Ser Leu Leu Asn Ser
            20                  25                  30
Gly Asn Gln Lys Asn Tyr Leu Thr Trp Tyr Gln Gln Arg Pro Gly Gln
        35                  40                  45
Ser Pro Arg Leu Leu Ile Tyr Trp Gly Ser Thr Arg Glu Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Gln Asn
                85                  90                  95
Glu Leu Phe Tyr Pro Leu Thr Phe Gly Gln Gly Thr Lys Leu Glu Ile
            100                 105                 110
Lys

<210>  91
<211>  339
<212>  DNA
<213>  Artificial sequence

<400>  91
gacatcgtga tgacccagag ccccctgagc ctgcccgtga ccctgggcca gcccgccagc      60
atcagctgca agagcagcca gagcctgctg aacagcggca accagaagaa ctacctgacc     120
tggtaccagc agagacccgg ccagagcccc agactgctga tctactgggg cagcaccaga     180
gagagcggcg tgcccgacag attcagcggc agcggcagcg gcaccgactt caccctgaag     240

atcagcagag tggaggccga ggacgtgggc gtgtactact gccagaacga gctgttctac     300
cccctgacct tcggccaggg caccaagctg gagatcaag                            339

<210>  92
<211>  468
<212>  PRT
<213>  Artificial sequence

<400>  92
Met Glu Thr Asp Thr Leu Leu Leu Trp Val Leu Leu Leu Trp Val Pro
1               5                   10                  15
Gly Ser Thr Gly Glu Val Gln Leu Val Glu Ser Gly Gly Gly Leu Val
            20                  25                  30
Gln Pro Gly Gly Ser Leu Arg Leu Ser Cys Ala Ala Ser Gly Phe Gly
        35                  40                  45
Phe Ser Asp Tyr Gly Met Ala Trp Val Arg Gln Ala Pro Gly Lys Gly
    50                  55                  60
Leu Glu Trp Val Ser Phe Ile Ser Asn Ser Ala Tyr Ser Ile Tyr Tyr
65                  70                  75                  80
Ala Asp Thr Val Thr Gly Arg Phe Thr Ile Ser Arg Asp Asn Ala Lys
                85                  90                  95
Asn Ser Leu Tyr Leu Gln Met Asn Ser Leu Arg Ala Glu Asp Thr Ala
            100                 105                 110
Val Tyr Tyr Cys Ala Arg Leu Gly Arg Gly Asn Ser Phe Ala Tyr Trp
        115                 120                 125
Gly Gln Gly Thr Leu Val Thr Val Ser Ser Ala Ser Thr Lys Gly Pro
    130                 135                 140
Ser Val Phe Pro Leu Ala Pro Ser Ser Lys Ser Thr Ser Gly Gly Thr
145                 150                 155                 160
Ala Ala Leu Gly Cys Leu Val Lys Asp Tyr Phe Pro Glu Pro Val Thr
                165                 170                 175
Val Ser Trp Asn Ser Gly Ala Leu Thr Ser Gly Val His Thr Phe Pro
            180                 185                 190
Ala Val Leu Gln Ser Ser Gly Leu Tyr Ser Leu Ser Ser Val Val Thr
        195                 200                 205
Val Pro Ser Ser Ser Leu Gly Thr Gln Thr Tyr Ile Cys Asn Val Asn
    210                 215                 220
His Lys Pro Ser Asn Thr Lys Val Asp Lys Lys Val Glu Pro Lys Ser
225                 230                 235                 240
Cys Asp Lys Thr His Thr Cys Pro Pro Cys Pro Ala Pro Glu Leu Leu
                245                 250                 255
Gly Gly Pro Ser Val Phe Leu Phe Pro Pro Lys Pro Lys Asp Thr Leu
            260                 265                 270
Met Ile Ser Arg Thr Pro Glu Val Thr Cys Val Val Val Asp Val Ser
        275                 280                 285
His Glu Asp Pro Glu Val Lys Phe Asn Trp Tyr Val Asp Gly Val Glu
    290                 295                 300
Val His Asn Ala Lys Thr Lys Pro Arg Glu Glu Gln Tyr Asn Ser Thr
305                 310                 315                 320
Tyr Arg Val Val Ser Val Leu Thr Val Leu His Gln Asp Trp Leu Asn
                325                 330                 335
Gly Lys Glu Tyr Lys Cys Lys Val Ser Asn Lys Ala Leu Pro Ala Pro
            340                 345                 350
Ile Glu Lys Thr Ile Ser Lys Ala Lys Gly Gln Pro Arg Glu Pro Gln
        355                 360                 365
Val Tyr Thr Leu Pro Pro Ser Arg Glu Glu Met Thr Lys Asn Gln Val
    370                 375                 380
Ser Leu Thr Cys Leu Val Lys Gly Phe Tyr Pro Ser Asp Ile Ala Val
385                 390                 395                 400
Glu Trp Glu Ser Asn Gly Gln Pro Glu Asn Asn Tyr Lys Thr Thr Pro
                405                 410                 415
Pro Val Leu Asp Ser Asp Gly Ser Phe Phe Leu Tyr Ser Lys Leu Thr
            420                 425                 430

Val Asp Lys Ser Arg Trp Gln Gln Gly Asn Val Phe Ser Cys Ser Val
        435                 440                 445
Met His Glu Ala Leu His Asn His Tyr Thr Gln Lys Ser Leu Ser Leu
    450                 455                 460
Ser Pro Gly Lys
465

<210>  93
<211>  1422
<212>  DNA
<213>  Artificial sequence

<400>  93
gaattcgccg ccaccatgga gaccgacacc ctgctgctgt gggtgctgct gctgtgggtg      60
cccggatcca ccggcgaggt gcagctggtg gagagcggcg gcggcctggt gcagcccggc     120
ggcagcctga gactgagctg cgccgccagc ggcttcggct tcagcgacta cggcatggcc     180
tgggtgagac aggcccccgg caagggcctg gagtgggtga gcttcatcag caacagcgcc     240
tacagcatct actacgccga caccgtgacc ggcagattca ccatcagcag agacaacgcc     300
aagaacagcc tgtacctgca gatgaacagc ctgagagccg aggacaccgc cgtgtactac     360
tgcgccagac tgggcagagg caacagcttc gcctactggg gccagggcac cctggtgacc     420
gtgagcagcg ctagcaccaa gggcccatcg gtcttccccc tggcaccctc ctccaagagc     480
acctctgggg gcacagcggc cctgggctgc ctggtcaagg actacttccc cgaaccggtg     540
acggtgtcgt ggaactcagg cgccctgacc agcggcgtgc acaccttccc ggccgtccta     600
cagtcctcag gactctactc cctcagcagc gtggtgaccg tgccctccag cagcttgggc     660
acccagacct acatctgcaa cgtgaatcac aagcccagca acaccaaggt ggacaagaag     720
gttgagccca aatcttgtga caaaactcac acatgcccac cgtgcccagc acctgaactc     780
ctggggggac cgtcagtctt cctcttcccc ccaaaaccca aggacaccct catgatctcc     840
cggacccctg aggtcacatg cgtggtggtg gacgtgagcc acgaagaccc tgaggtcaag     900
ttcaactggt acgtggacgg cgtggaggtg cataatgcca agacaaagcc gcgggaggag     960
cagtacaaca gcacgtaccg tgtggtcagc gtcctcaccg tcctgcacca ggactggctg    1020
aatggcaagg agtacaagtg caaggtctcc aacaaagccc tcccagcccc catcgagaaa    1080
accatctcca aagccaaagg gcagccccga gaaccacagg tgtacaccct gcccccatcc    1140
cgggaggaga tgaccaagaa ccaggtcagc ctgacctgcc tggtcaaagg cttctatccc    1200
agcgacatcg ccgtggagtg ggagagcaat gggcagccgg agaacaacta caagaccacg    1260
cctcccgtgc tggactccga cggctccttc ttcctctaca gcaagctcac cgtggacaag    1320
agcaggtggc agcaggggaa cgtcttctca tgctccgtga tgcatgaggc tctgcacaac    1380
cactacacgc agaagagcct ctccctgtct ccgggtaaat ga                        1422

<210>  94
<211>  240
<212>  PRT
<213>  Artificial sequence

<400>  94
Met Glu Thr Asp Thr Leu Leu Leu Trp Val Leu Leu Leu Trp Val Pro
1               5                   10                  15
Gly Ser Thr Gly Asp Ile Val Met Thr Gln Ser Pro Asp Ser Leu Ala
            20                  25                  30
Val Ser Leu Gly Glu Arg Ala Thr Ile Asn Cys Lys Ser Ser Gln Ser
        35                  40                  45
Leu Leu Asn Ser Gly Asn Gln Lys Asn Tyr Leu Thr Trp Tyr Gln Gln
    50                  55                  60
Lys Pro Gly Gln Pro Pro Lys Leu Leu Ile Tyr Trp Gly Ser Thr Arg
65                  70                  75                  80
Glu Ser Gly Val Pro Asp Arg Phe Ser Gly Ser Gly Ser Gly Thr Asp
                85                  90                  95
Phe Thr Leu Thr Ile Ser Ser Leu Gln Ala Glu Asp Val Ala Val Tyr
            100                 105                 110
Tyr Cys Gln Asn Glu Leu Phe Tyr Pro Leu Thr Phe Gly Gln Gly Thr
        115                 120                 125
Lys Leu Glu Ile Lys Arg Thr Val Ala Ala Pro Ser Val Phe Ile Phe
    130                 135                 140
Pro Pro Ser Asp Glu Gln Leu Lys Ser Gly Thr Ala Ser Val Val Cys

145                 150                 155                 160
Leu Leu Asn Asn Phe Tyr Pro Arg Glu Ala Lys Val Gln Trp Lys Val
                165                 170                 175
Asp Asn Ala Leu Gln Ser Gly Asn Ser Gln Glu Ser Val Thr Glu Gln
            180                 185                 190
Asp Ser Lys Asp Ser Thr Tyr Ser Leu Ser Ser Thr Leu Thr Leu Ser
        195                 200                 205
Lys Ala Asp Tyr Glu Lys His Lys Val Tyr Ala Cys Glu Val Thr His
    210                 215                 220
Gln Gly Leu Ser Ser Pro Val Thr Lys Ser Phe Asn Arg Gly Glu Cys
225                 230                 235                 240

<210>  95
<211>  738
<212>  DNA
<213>  Artificial sequence

<400>  95
gaattcgccg ccaccatgga gaccgacacc ctgctgctgt gggtgctgct gctgtgggtg      60
cccggatcca ccggcgacat cgtgatgacc cagagccccg acagcctggc cgtgagcctg     120
ggcgagagag ccaccatcaa ctgcaagagc agccagagcc tgctgaacag cggcaaccag     180
aagaactacc tgacctggta ccagcagaag cccggccagc cccccaagct gctgatctac     240
tggggcagca ccagagagag cggcgtgccc gacagattca gcggcagcgg cagcggcacc      300
gacttcaccc tgaccatcag cagcctgcag gccgaggacg tggccgtgta ctactgccag     360
aacgagctgt tctaccccct gaccttcggc cagggcacca agctggagat caagcgtacg      420
gtggctgcac catctgtctt catcttcccg ccatctgatg agcagttgaa atctggaact     480
gcctctgttg tgtgcctgct gaataacttc tatcccagag aggccaaagt acagtggaag     540
gtggataacg ccctccaatc gggtaactcc caggagagtg tcacagagca ggacagcaag     600
gacagcacct acagcctcag cagcaccctg acgctgagca aagcagacta cgagaaacac     660
aaagtctacg cctgcgaagt cacccatcag ggcctgagct cgcccgtcac aaagagcttc     720
aacaggggag agtgttga                                                   738

<210>  96
<211>  467
<212>  PRT
<213>  Artificial sequence

<400>  96
Met Gly Trp Ser Cys Ile Ile Leu Phe Leu Val Ala Thr Ala Thr Gly
1               5                   10                  15
Val His Ser Glu Val Gln Leu Val Glu Ser Gly Gly Gly Leu Val Gln
            20                  25                  30
Pro Gly Gly Ser Leu Arg Leu Ser Cys Ala Ala Ser Gly Phe Gly Phe
        35                  40                  45
Ser Asp Tyr Gly Met Ala Trp Val Arg Gln Ala Pro Gly Lys Gly Leu
    50                  55                  60
Glu Trp Val Ser Phe Ile Ser Asn Ser Ala Tyr Ser Ile Tyr Tyr Ala
65                  70                  75                  80
Asp Thr Val Thr Gly Arg Phe Thr Ile Ser Arg Asp Asn Ala Lys Asn
                85                  90                  95
Ser Leu Tyr Leu Gln Met Asn Ser Leu Arg Ala Glu Asp Thr Ala Val
            100                 105                 110
Tyr Tyr Cys Ala Arg Leu Gly Arg Gly Asn Ser Phe Ala Tyr Trp Gly
        115                 120                 125
Gln Gly Thr Leu Val Thr Val Ser Ser Ala Ser Thr Lys Gly Pro Ser
    130                 135                 140
Val Phe Pro Leu Ala Pro Ser Ser Lys Ser Thr Ser Gly Gly Thr Ala
145                 150                 155                 160
Ala Leu Gly Cys Leu Val Lys Asp Tyr Phe Pro Glu Pro Val Thr Val
                165                 170                 175
Ser Trp Asn Ser Gly Ala Leu Thr Ser Gly Val His Thr Phe Pro Ala
            180                 185                 190
Val Leu Gln Ser Ser Gly Leu Tyr Ser Leu Ser Ser Val Val Thr Val

195                 200                 205
Pro Ser Ser Ser Leu Gly Thr Gln Thr Tyr Ile Cys Asn Val Asn His
    210                 215                 220
Lys Pro Ser Asn Thr Lys Val Asp Lys Lys Val Glu Pro Lys Ser Cys
225                 230                 235                 240
Asp Lys Thr His Thr Cys Pro Pro Cys Pro Ala Pro Glu Leu Leu Gly
                245                 250                 255
Gly Pro Ser Val Phe Leu Leu Pro Pro Lys Pro Lys Asp Thr Leu Met
            260                 265                 270
Ile Ser Arg Thr Pro Glu Val Thr Cys Val Val Val Asp Val Ser His
        275                 280                 285
Glu Asp Pro Glu Val Lys Phe Asn Trp Tyr Val Asp Gly Val Glu Val
    290                 295                 300
His Asn Ala Lys Thr Lys Pro Pro Glu Glu Gln Tyr Asn Ser Thr Leu
305                 310                 315                 320
Arg Val Val Ser Ile Leu Thr Val Leu His Gln Asp Trp Leu Asn Gly
                325                 330                 335
Lys Glu Tyr Lys Cys Lys Val Ser Asn Lys Ala Leu Pro Ala Pro Ile
            340                 345                 350
Glu Lys Thr Ile Ser Lys Ala Lys Gly Gln Pro Arg Glu Pro Gln Val
        355                 360                 365
Tyr Thr Leu Pro Pro Ser Arg Asp Glu Leu Thr Lys Asn Gln Val Ser
    370                 375                 380
Leu Thr Cys Leu Val Lys Gly Phe Tyr Pro Ser Asp Ile Ala Val Glu
385                 390                 395                 400
Trp Glu Ser Asn Gly Gln Pro Glu Asn Asn Tyr Lys Thr Thr Pro Leu
                405                 410                 415
Val Leu Asp Ser Asp Gly Ser Phe Phe Leu Tyr Ser Lys Leu Thr Val
            420                 425                 430
Asp Lys Ser Arg Trp Gln Gln Gly Asn Val Phe Ser Cys Ser Val Met
        435                 440                 445
His Glu Ala Leu His Asn His Tyr Thr Gln Lys Ser Leu Ser Leu Ser
    450                 455                 460
Pro Gly Lys
465

<210>  97
<211>  1422
<212>  DNA
<213>  Artificial sequence

<400>  97
cctagggcca ccatgggatg gtcctgcatc attctcttcc tggtggccac cgctaccgga      60
gtgcacagcg aggtgcagct ggtggagagc ggcggcggcc tggtgcagcc cggcggcagc     120
ctgagactga gctgcgccgc cagcggcttc ggcttcagcg actacggcat ggcctgggtg     180
agacaggccc ccggcaaggg cctggagtgg gtgagcttca tcagcaacag cgcctacagc     240
atctactacg ccgacaccgt gaccggcaga ttcaccatca gcagagacaa cgccaagaac     300
agcctgtacc tgcagatgaa cagcctgaga gccgaggaca ccgccgtgta ctactgcgcc     360
agactgggca gaggcaacag cttcgcctac tggggccagg gcaccctggt gaccgtgagc     420
agcgcttcga ccaagggccc atcggtcttc cccctggcac cctcctccaa gagcacctct     480
gggggcacag cggccctggg ctgcctggtc aaggactact tccccgaacc ggtgacggtg     540
tcgtggaact caggcgccct gaccagcggc gtgcacacct tcccggctgt cctacagtcc     600
tcaggactct actccctcag cagcgtggtg accgtgccct ccagcagctt gggcacccag     660
acctacatct gcaacgtgaa tcacaagccc agcaacacca aggtggacaa gaaagttgag     720
cccaaatctt gtgacaaaac tcacacatgc ccaccgtgcc cagcacctga actcctgggg     780
ggaccgtcag tcttcctcct gcccccaaaa cccaaggaca ccctcatgat ctcccggacc     840
cctgaggtca catgcgtggt ggtggacgtg agccacgaag accctgaggt caagttcaac     900
tggtacgtgg acggcgtgga ggtgcataat gccaagacaa agccgcccga ggagcagtac     960
aacagcacgc tgcgtgtggt cagcatcctc accgtcctgc accaggactg gctgaatggc    1020
aaggagtaca agtgcaaggt ctccaacaaa gccctcccag cccccatcga gaaaaccatc    1080
tccaaagcca aagggcagcc ccgagaacca caggtgtaca ccctgccccc atcccgggat    1140
gagctgacca agaaccaggt cagcctgacc tgcctggtca aaggcttcta tcccagcgac    1200
atcgccgtgg agtgggagag caatgggcag ccggagaaca actacaagac cacgcctctg    1260

gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaatgagtat ac 1422

<210> 98
<211> 240
<212> PRT
<213> Artificial sequence

<400> 98
Met Glu Ser Gln Thr Gln Val Leu Met Ser Leu Leu Phe Trp Val Ser
1               5                   10                  15
Gly Thr Cys Gly Asp Ile Val Met Thr Gln Ser Pro Asp Ser Leu Ala
            20                  25                  30
Val Ser Leu Gly Glu Arg Ala Thr Ile Asn Cys Lys Ser Ser Gln Ser
        35                  40                  45
Leu Leu Asn Ser Gly Asn Gln Lys Asn Tyr Leu Thr Trp Tyr Gln Gln
    50                  55                  60
Lys Pro Gly Gln Pro Pro Lys Leu Leu Ile Tyr Trp Gly Ser Thr Arg
65                  70                  75                  80
Glu Ser Gly Val Pro Asp Arg Phe Ser Gly Ser Gly Ser Gly Thr Asp
                85                  90                  95
Phe Thr Leu Thr Ile Ser Ser Leu Gln Ala Glu Asp Val Ala Val Tyr
            100                 105                 110
Tyr Cys Gln Asn Glu Leu Phe Tyr Pro Leu Thr Phe Gly Gln Gly Thr
        115                 120                 125
Lys Leu Glu Ile Lys Arg Thr Val Ala Ala Pro Ser Val Phe Ile Phe
    130                 135                 140
Pro Pro Ser Asp Glu Gln Leu Lys Ser Gly Thr Ala Ser Val Val Cys
145                 150                 155                 160
Leu Leu Asn Asn Phe Tyr Pro Arg Glu Ala Lys Val Gln Trp Lys Val
                165                 170                 175
Asp Asn Ala Leu Gln Ser Gly Asn Ser Gln Glu Ser Val Thr Glu Gln
            180                 185                 190
Asp Ser Lys Asp Ser Thr Tyr Ser Leu Ser Ser Thr Leu Thr Leu Ser
        195                 200                 205
Lys Ala Asp Tyr Glu Lys His Lys Val Tyr Ala Cys Glu Val Thr His
    210                 215                 220
Gln Gly Leu Ser Ser Pro Val Thr Lys Ser Phe Asn Arg Gly Glu Cys
225                 230                 235                 240

<210> 99
<211> 743
<212> DNA
<213> Artificial sequence

<400> 99
gatatcgcca ccatggagtc ccagacacag gtgctgatga gcctgctgtt ctgggtgtcc 60
ggcacctgtg gcgacatcgt gatgacccag agccccgaca gcctggccgt gagcctgggc 120
gagagagcca ccatcaactg caagagcagc cagagcctgc tgaacagcgg caaccagaag 180
aactacctga cctggtacca gcagaagccc ggccagcccc ccaagctgct gatctactgg 240
ggcagcacca gagagagcgg cgtgcccgac agattcagcg gcagcggcag cggcaccgac 300
ttcaccctga ccatcagcag cctgcaggcc gaggacgtgg ccgtgtacta ctgccagaac 360
gagctgttct accccctgac cttcggccag ggcaccaagc tggagatcaa gcgtacggtg 420
gctgcaccat ctgtcttcat cttcccgcca tctgatgagc agttgaaatc tggaactgcc 480
tctgttgtgt gcctgctgaa taacttctat cccagagagg ccaaagtaca gtggaaggtg 540
gataacgccc tccaatcggg taactcccag gagagtgtca cagagcagga cagcaaggac 600
agcacctaca gcctcagcag caccctgacg ctgagcaaag cagactacga gaaacacaaa 660
gtctacgcct gcgaagtcac ccatcagggc ctgagctcgc ccgtcacaaa gagcttcaac 720
aggggagagt gttgattaat taa 743

<210> 100
<211> 467

<212>  PRT
<213>  Artificial sequence

<400>  100
Met Gly Trp Ser Cys Ile Ile Leu Phe Leu Val Ala Thr Ala Thr Gly
1               5                   10                  15
Val His Ser Gln Val Gln Leu Gln Gln Pro Gly Ala Glu Leu Val Arg
            20                  25                  30
Pro Gly Ala Ser Val Lys Leu Ser Cys Lys Ala Ser Gly Tyr Thr Phe
        35                  40                  45
Thr Ser Tyr Trp Ile Asn Trp Val Lys Gln Arg Pro Gly Gln Gly Leu
    50                  55                  60
Glu Trp Ile Gly Asn Ile Tyr Pro Ser Asp Ser Tyr Thr Asn Tyr Asn
65                  70                  75                  80
Gln Lys Phe Lys Asp Lys Ala Thr Leu Thr Val Asp Lys Ser Ser Ser
                85                  90                  95
Thr Ala Tyr Met Gln Leu Ser Ser Pro Thr Ser Glu Asp Ser Ala Val
            100                 105                 110
Tyr Tyr Cys Thr Arg Ser Trp Arg Gly Asn Ser Phe Asp Tyr Trp Gly
        115                 120                 125
Gln Gly Thr Thr Leu Thr Val Ser Ser Ala Ser Thr Lys Gly Pro Ser
    130                 135                 140
Val Phe Pro Leu Ala Pro Ser Ser Lys Ser Thr Ser Gly Gly Thr Ala
145                 150                 155                 160
Ala Leu Gly Cys Leu Val Lys Asp Tyr Phe Pro Glu Pro Val Thr Val
                165                 170                 175
Ser Trp Asn Ser Gly Ala Leu Thr Ser Gly Val His Thr Phe Pro Ala
            180                 185                 190
Val Leu Gln Ser Ser Gly Leu Tyr Ser Leu Ser Ser Val Val Thr Val
        195                 200                 205
Pro Ser Ser Ser Leu Gly Thr Gln Thr Tyr Ile Cys Asn Val Asn His
    210                 215                 220
Lys Pro Ser Asn Thr Lys Val Asp Lys Lys Val Glu Pro Lys Ser Cys
225                 230                 235                 240
Asp Lys Thr His Thr Cys Pro Pro Cys Pro Ala Pro Glu Leu Leu Gly
                245                 250                 255
Gly Pro Ser Val Phe Leu Leu Pro Pro Lys Pro Lys Asp Thr Leu Met
            260                 265                 270
Ile Ser Arg Thr Pro Glu Val Thr Cys Val Val Val Asp Val Ser His
        275                 280                 285
Glu Asp Pro Glu Val Lys Phe Asn Trp Tyr Val Asp Gly Val Glu Val
    290                 295                 300
His Asn Ala Lys Thr Lys Pro Pro Glu Glu Gln Tyr Asn Ser Thr Leu
305                 310                 315                 320
Arg Val Val Ser Ile Leu Thr Val Leu His Gln Asp Trp Leu Asn Gly
                325                 330                 335
Lys Glu Tyr Lys Cys Lys Val Ser Asn Lys Ala Leu Pro Ala Pro Ile
            340                 345                 350
Glu Lys Thr Ile Ser Lys Ala Lys Gly Gln Pro Arg Glu Pro Gln Val
        355                 360                 365
Tyr Thr Leu Pro Pro Ser Arg Asp Glu Leu Thr Lys Asn Gln Val Ser
    370                 375                 380
Leu Thr Cys Leu Val Lys Gly Phe Tyr Pro Ser Asp Ile Ala Val Glu
385                 390                 395                 400
Trp Glu Ser Asn Gly Gln Pro Glu Asn Asn Tyr Lys Thr Thr Pro Leu
                405                 410                 415
Val Leu Asp Ser Asp Gly Ser Phe Phe Leu Tyr Ser Lys Leu Thr Val
            420                 425                 430
Asp Lys Ser Arg Trp Gln Gln Gly Asn Val Phe Ser Cys Ser Val Met
        435                 440                 445
His Glu Ala Leu His Asn His Tyr Thr Gln Lys Ser Leu Ser Leu Ser
    450                 455                 460
Pro Gly Lys

465

<210>  101
<211>  8
<212>  PRT
<213>  Artificial sequence

<400>  101
Gly Phe Gly Phe Ser Asp Tyr Gly
1               5

<210>  102
<211>  8
<212>  PRT
<213>  Artificial sequence

<400>  102
Gly Phe Thr Phe Ser Asp Tyr Gly
1               5

<210>  103
<211>  8
<212>  PRT
<213>  Artificial sequence

<400>  103
Gly Phe Thr Phe Ser Arg Tyr Gly
1               5

<210>  104
<211>  8
<212>  PRT
<213>  Artificial sequence

<400>  104
Ile Ser Asn Leu Ala Tyr Ser Ile
1               5

<210>  105
<211>  8
<212>  PRT
<213>  Artificial sequence

<400>  105
Ile Ser Asn Ser Ala Tyr Ser Ile
1               5

<210>  106
<211>  11
<212>  PRT
<213>  Artificial sequence

<400>  106
Ala Arg Leu Gly Arg Gly Asn Ser Phe Ala Tyr
1               5                   10

<210>  107
<211>  11
<212>  PRT
<213>  Artificial sequence

<400>  107
Ala Arg Leu Gly Tyr Gly Asn Ser Phe Ala Tyr

1               5                   10

<210>  108
<211>  17
<212>  PRT
<213>  Artificial sequence

<400>  108
Lys Ser Ser Gln Ser Leu Leu Asn Ser Gly Asn Gln Lys Asn Tyr Leu
1               5                   10                  15
Thr

<210>  109
<211>  7
<212>  PRT
<213>  Artificial sequence

<400>  109
Trp Ala Ser Thr Arg Glu Pro
1               5

<210>  110
<211>  7
<212>  PRT
<213>  Artificial sequence

<400>  110
Trp Ala Ser Thr Arg Glu Ser
1               5

<210>  111
<211>  7
<212>  PRT
<213>  Artificial sequence

<400>  111
Trp Gly Ser Thr Arg Glu Ser
1               5

<210>  112
<211>  9
<212>  PRT
<213>  Artificial sequence

<400>  112
Gln Asn Asp Tyr Ser Phe Pro Leu Thr
1               5

<210>  113
<211>  9
<212>  PRT
<213>  Artificial sequence

<400>  113
Gln Asn Asp Tyr Ser Leu Pro Phe Thr
1               5

<210>  114
<211>  9
<212>  PRT
<213>  Artificial sequence

<400>  114
Gln Asn Asp Tyr Ser Tyr Pro Leu Thr
1               5

<210>  115
<211>  9
<212>  PRT
<213>  Artificial sequence

<400>  115
Gln Asn Glu Leu Phe Tyr Pro Leu Thr
1               5

<210>  116
<211>  9
<212>  PRT
<213>  Artificial sequence

<400>  116
Gln Asn Asn Tyr Tyr Tyr Pro Leu Thr
1               5